# EUROPEAN PATENT SPECIFICATION

(11) **EP 1 148 136 B1**
(45) Date of publication and mention of the grant of the patent: **05.12.2012**
(21) Application number: 01116362.3
(22) Date of filing: 10.09.1999
(51) Int. Cl.: C12N 15/53, C12N 9/02, C12N 15/81, C12N 5/10, C12Q 1/68, C12P 7/44, C12R 1/74, C07K 14/40

(54) **Cytochrome P450 monooxygenase and NADPH cytochrome P450 oxidoreductase genes and proteins related to the omega hydroxylase complex of Candida tropicalis and methods relating thereto**
Cytochrom P450-Monooxygenase und NADPH-Cytochrom P450-Oxidoreduktase Gene und Proteine des Omega-Hydroxylase-Komplexes von Candida tropicalis und damit verbundene Verfahren
Génes de la monooxygénase du cytochrome P450 et de l'oxidoreductase NADPH du cytochrome P450 et protéines associées au complexe de l'omega hydroxylase de Candida tropicalis et procédes associes

(30) Priority: 05.10.1998 US 103099 P; 10.03.1999 US 123555 P
(43) Date of publication of application: 24.10.2001
(62) Divisional of application: 99946862.2
(73) Proprietor: Cognis IP Management GmbH, 40589 Düsseldorf (DE)
(72) Inventor: Wilson, C. Ron, Loveland, OH 45140 (US); Craft, David L., Fort Thomas, KY 41075 (US); Eirich, L. Dudley, Cincinnati, OH 45150 (US); Eshoo, Mark, Fairfax, CA 94930 (US); Madduri, Krishna M., Westfield, IN 46074 (US); Cornett, Cathy A., Crescent Springs, KYX 41017 (US); Brenner, Alfred A., Santa Rosa, CA 95401 (US); Tang, Maria, Fairfield, CA 94533 (US); Loper, John C., Cincinnati, OH 45213 (US); Gleeson, Martin, San Diego, CA 92130 (US)
(74) Representative: Herzog, Martin

(56) References cited:
- WO-A-91/14781
- SEGHEZZI ET AL.: "C. tropicalis CYP52A8 gene" EMBL SEQUENCE DATABASE, 27 June 1992 (1992-06-27), XP002139854 -& SEGHEZZI ET AL.: "Cytochrome P450 52A8" SWISSPROT SEQUENCE DATABASE, 1 April 1993 (1993-04-01), XP002139855 -& DNA CELL BIOL, vol. 11, 1992, pages 767-780, XP000920960
- SANGLARD D ET AL: "CHARACTERIZATION OF THE ALKANE-INDUCIBLE CYTOCHROME P450 (P450ALK) GENE FROM THE YEAST CANDIDA TROPICALIS: IDENTIFICATION OF A NEW P450 GENE FAMILY" GENE, ELSEVIER BIOMEDICAL PRESS. AMSTERDAM, NL, vol. 76, no. 1, 1989, pages 121-136, XP000914689 ISSN: 0378-1119
- SEGHEZZI W ET AL: "CHARACTERIZATION OF A SECOND ALKANE-INDUCIBLE CYTOCHROME P450-ENCODING GENE, CYP52A2, FROM CANDIDA TROPICALIS" GENE, ELSEVIER BIOMEDICAL PRESS. AMSTERDAM, NL, vol. 106, 1991, pages 51-60, XP000914690 ISSN: 0378-1119
- OHKUMA M ET AL: "CYP52 (CYTOCHROME P450ALK) MULTIGENE FAMILY IN CANDIDA MALTOSA: IDENTIFICATION AND CHARACTERIZATION OF EIGHT MEMBERS" DNA AND CELL BIOLOGY, NEW YORK, NY, US, vol. 14, no. 2, 1995, pages 163-173, XP000920907 ISSN: 1044-5498
- NELSON ET AL.: "P450 SUPERFAMILY: UPDATE ON NEW SEQUENCES, GENE MAPPING, ACCESSION NUMBERS AND NOMENCLATURE" PHARMACOGENETICS, vol. 6, no. 1, February 1996 (1996-02), pages 1-42, XP000196724 ISSN: 0960-314X
- SEGHEZZI W ET AL: "IDENTIFICATION AND CHARACTERIZATION OF ADDITIONAL MEMBERS OF THE CYTOCHROME P450 MULTIGENE FAMILY CYP52 OF CANDIDA TROPICALIS", DNA AND CELL BIOLOGY, MARY ANN LIEBERT, NEW YORK, NY, US, vol. 11, no. 10, 1 January 1992 (1992-01-01), pages 767-780, XP000920960, ISSN: 1044-5498

### SEQUENCE LISTING

<110> Henkel Corporation
<120> CYTOCHROME P450 MONOOXYGENASE AND NADPH CYTOCHROME P450 OXIDOREDUCTASE GENES AND PROTEINS RELATED TO THE OMEGA HYDROXYLASE COMPLEX OF CANDIDA TROPICALIS AND METHODS RELATING THERETO
<130> PCT
<140> PCT/US99/20797
   <141> 1999-10-09
<160> 107
<170> PatentIn Ver. 2.1
<210> 1
   <211> 32
   <212> DNA
   <213> Artificial Sequence
<220>
   <223> Description of Artificial Sequence: Primer for CYP52A2A gene
<400> 1
   ccttaattaa atgcacgaag cggagataaa ag 32
<210> 2
   <211> 30
   <212> DNA
   <213> Artificial Sequence
<220>
   <223> Description of Artificial Sequence: Primer for CYP52A2A gene
<400> 2
   ccttaattaa gcataagctt gctcgagtct 30
<210> 3
   <211> 31
   <212> DNA
   <213> Artificial Sequence
<220>
   <223> Description of Artificial Sequence: Primer for CYP52A3A gene
<400> 3
   ccttaattaa acgcaatggg aacatggagt g 31
<210> 4
   <211> 34
   <212> DNA
   <213> Artificial Sequence
<220>
   <223> Description of Artificial Sequence: Primer for CYP52A3A gene
<400> 4
   ccttaattaa tcgcactacg gttattggta tcag 34
<210> 5
   <211> 29
   <212> DNA
   <213> Artificial Sequence
<220>
   <223> Description of Artificial Sequence: Primer for CYP52A5A gene
<400> 5
   ccttaattaa tcaaagtacg ttcaggcgg 29
<210> 6
   <211> 34
   <212> DNA
   <213> Artificial Sequence
<220>
   <223> Description of Artificial Sequence: Primer for CYP52A5A gene
<400> 6
   ccttaattaa ggcagacaac aacttggcaa agtc 34
<210> 7
   <211> 31
   <212> DNA
   <213> Artificial Sequence
<220>
   <223> Description of Artificial Sequence: Primer for CPRB gene
<400> 7
   ccttaattaa gaggtcgttg gttgagtttt c 31
<210> 8
   <211> 29
   <212> DNA
   <213> Artificial Sequence
<220>
   <223> Description of Artificial Sequence: Primer for CPRB gene
<400> 8
   ccttaattaa ttgataatga cgttgcggg 29
<210> 9
   <211> 33
   <212> DNA
   <213> Artificial Sequence
<220>
   <223> Description of Artificial Sequence: Primer for URA3A gene
<400> 9
   aggcgcgccg gagtccaaaa agaccaacct ctg 33
<210> 10
   <211> 34
   <212> DNA
   <213> Artificial Sequence
<220>
   <223> Description of Artificial Sequence: Primer for URA3A gene
<400> 10
   ccttaattaa tacgtggata ccttcaagca agtg 34
<210> 11
   <211> 35
   <212> DNA
   <213> Artificial Sequence
<220>
   <223> Description of Artificial Sequence: Primer for URA3A gene
<400> 11
   ccttaattaa gctcacgagt tttgggattt tcgag 35
<210> 12
   <211> 35
   <212> DNA
   <213> Artificial Sequence
<220>
   <223> Description of Artificial Sequence: Primer for URA3A gene
<400> 12
   gggtttaaac cgcagaggtt ggtctttttg gactc 35
<210> 13
   <211> 10
   <212> DNA
   <213> Artificial Sequence
<220>
   <223> Description of Artificial Sequence: PmeI restriction site
<400> 13
   gggtttaaac 10
<210> 14
   <211> 9
   <212> DNA
   <213> Artificial Sequence
<220>
   <223> Description of Artificial Sequence: AscI restriction site
<400> 14
   aggcgcgcc 9
<210> 15
   <211> 10
   <212> DNA
   <213> Artificial Sequence
<220>
   <223> Description of Artificial Sequence: PacI restriction site
<400> 15
   ccttaattaa 10
<210> 16
   <211> 21
   <212> DNA
   <213> Artificial Sequence
<220>
   <223> Description of Artificial Sequence: Primer for CPR gene
<400> 16
   tcycaaacwg gtacwgcwga a 21
<210> 17
   <211> 21
   <212> DNA
   <213> Artificial Sequence
<220>
   <223> Description of Artificial Sequence: Primer for CPR gene
<400> 17
   ggtttgggta aytcwactta t 21
<210> 18
   <211> 18
   <212> DNA
   <213> Artificial Sequence
<220>
   <223> Description of Artificial Sequence: Primer for CPR gene
<400> 18
   cgttattatc atttcttc 18
<210> 19
   <211> 20
   <212> DNA
   <213> Artificial Sequence
<220>
   <223> Description of Artificial Sequence: Primer for CPR gene
<400> 19
   gcmacaccrg tacctggacc 20
<210> 20
   <211> 18
   <212> DNA
   <213> Artificial Sequence
<220>
   <223> Description of Artificial Sequence: Primer for CPR gene
<400> 20
   atcccaatcg taatcagc 18
<210> 21
   <211> 18
   <212> DNA
   <213> Artificial Sequence
<220>
   <223> Description of Artificial Sequence: Primer for CPR gene
<400> 21
   acttgtcttc gtttagca 18
<210> 22
   <211> 18
   <212> DNA
   <213> Artificial Sequence
<220>
   <223> Description of Artificial Sequence: Primer for CPR gene
<400> 22
   ctacgtctgt ggtgatgc 18
<210> 23
   <211> 17
   <212> DNA
   <213> Artificial Sequence
<220>
   <223> Description of Artificial Sequence: Primer for CYP gene
<220>
   <221> misc_feature
   <222> (3)..(4)
   <223> n=dATP or dCTP or dGTP or dTTP
<220>
   <221> misc_feature
   <222> (9)..(10)
   <223> n=dATP or dCTP or dGTP or dTTP
<220>
   <221> misc feature
   <222> (12)..(13)
   <223> n=dATP or dCTP or dGTP or dTTP
<220>
   <221> misc feature
   <222> (15)..(16)
   <223> n=dATP or dCTP or dGTP or dTTP
<400> 23
   cgngayacna_cngcngg 17
<210> 24
   <211> 17
   <212> DNA
   <213> Artificial Sequence
<220>
   <223> Description of Artificial Sequence: Primer for CYP gene
<220>
   <221> misc_feature
   <222> (9) .. (10)
   <223> n=dATP or dCTP or dGTP or dTTP
<220>
   <221> misc feature
   <222> (12)..(13)
   <223> n=dATP or dCTP or dGTP or dTTP
<220>
   <221> misc_feature
   <222> (15)..(16)
   <223> n=dATP or dCTP or dGTP or dTTP
<400> 24
   agrgayacna cngcngg 17
<210> 25
   <211> 17
   <212> DNA
   <213> Artificial Sequence
<220>
   <223> Description of Artificial Sequence: Primer for CYP gene
<220>
   <221> misc_feature
   <222> (3)..(4)
   <223> n=dATP or dCTP or dGTP or dTTP
<220>
   <221> misc_feature
   <222> (15)..(16)
   <223> n=dATP or dCTP or dGTP or dTTP
<400> 25
   agngcraayt gytgncc 17
<210> 26
   <211> 18
   <212> DNA
   <213> Artificial Sequence
<220>
   <223> Description of Artificial Sequence: Primer for CYP gene
<220>
   <221> misc feature
   <222> (4)..(5)
   <223> n=dATP or dCTP or dGTP or dTTP
<220>
   <221> misc_feature
   <222> (16)..(17)
   <223> n=dATP or dCTP or dGTP or dTTP
<400> 26
   yaangcraay tgytgncc 18
<210> 27
   <211> 29
   <212> DNA
   <213> Artificial Sequence
<220>
   <223> Description of Artificial Sequence: Primer for CYP gene
<400> 27
   attcaacggt ggtccaagaa tctgtttgg 29
<210> 28
   <211> 25
   <212> DNA
   <213> Artificial Sequence
<220>
   <223> Description of Artificial Sequence: Primer for CYP gene
<400> 28
   gagctatgtt gagaccacag tttgc 25
<210> 29
   <211> 26
   <212> DNA
   <213> Artificial Sequence
<220>
   <223> Description of Artificial Sequence: Primer for CYP gene
<400> 29
   cttcagttaa agcaaattgt ttggcc 26
<210> 30
   <211> 25
   <212> DNA
   <213> Artificial Sequence
<220>
   <223> Description of Artificial Sequence: Primer for pTriplEX vector
<400> 30
   ctcgggaagc gcgccattgt gttgg 25
<210> 31
   <211> 29
   <212> DNA
   <213> Artificial Sequence
<220>
   <223> Description of Artificial Sequence: Primer for pTriplEx vector
<400> 31
   taatacgact cactataggg cgaattggc 29
<210> 32
   <211> 21
   <212> DNA -
   <213> Artificial Sequence
<220>
   <223> Description of Artificial Sequence: Primer for CYP gene
<400> 32
   tgrytcaaac catctytctg g 21
<210> 33
   <211> 17
   <212> DNA
   <213> Artificial Sequence
<220>
   <223> Description of Artificial Sequence: Primer for CYP gene
<400> 33
   ggaccggcgt taaaggg 17
<210> 34
   <211> 23
   <212> DNA
   <213> Artificial Sequence
<220>
   <223> Description of Artificial Sequence: Primer for CYP gene
<400> 34
   catagtcgwa tyatgcttag acc 23
<210> 35
   <211> 17
   <212> DNA
   <213> Artificial Sequence
<220>
   <223> Description of Artificial Sequence: Primer for CYP gene
<400> 35 .
   ggaccaccat tgaatgg 17
<210> 36
   <211> 540
   <212> DNA
   <213> Candida tropicalis
<400> 36
   atgattgaac aactcctaga atattggtat gtcgttgtgc cagtgttgta catcatcaaa 60
   caactccttg catacacaaa gactcgcgtc ttgatgaaaa agttgggtgc tgctccagtc 120
   acaaacaagt tgtacgacaa cgctttcggt atcgtcaatg gatggaaggc tctccagttc 180
   aagaaagagg gcagggctca agagtacaac gattacaagt ttgaccactc caagaaccca 240
   agcgtgggca cctacgtcag tattcttttc ggcaccagga tcgtcgtgac caaagatcca 300
   gagaatatca aagctatttt ggcaacccag tttggtgatt tttctttggg caagaggcac 360
   actcttttta agcctttgtt aggtgatggg atcttcacat tggacggcga aggctggaag 420
   cacagcagag ccatgttgag accacagttt gccagagaac aagttgctca tgtgacgtcg 480
   ttggaaccac acttccagtt gttgaagaag catattctta agcacaaggg tgaatacttt 540
<210> 37
   <211> 25
   <212> DNA
   <213> Artificial Sequence
<220>
   <223> Description of Artificial Sequence: Primer for CYP52A1A gene
<400> 37
   ccgatgaagt tttcgacgag taccc 25
<210> 38
   <211> 26
   <212> DNA
   <213> Artificial Sequence
<220>
   <223> Description of Artificial Sequence: Primer for CYP52A1A gene
<400> 38
   aaggctttaa cgtgtccaat ctggtc 26
<210> 39
   <211> 27
   <212> DNA
   <213> Artificial Sequence
<220>
   <223> Description of Artificial Sequence: Primer for CYP52A2A gene
<400> 39
   attatcgcca catacttcac caaatgg 27
<210> 40
   <211> 24
   <212> DNA
   <213> Artificial Sequence
<220>
   <223> Description of Artificial Sequence: Primer for CYP52A2A gene
<400> 40
   cgagatcgtg gatacgctgg agtg 24
<210> 41
   <211> 25
   <212> DNA
   <213> Artificial Sequence
<220>
   <223> Description of Artificial Sequence: Primer for CYP52A3A gene
<400> 41
   gccactcggt aactttgtca gggac 25
<210> 42
   <211> 26
   <212> DNA
   <213> Artificial Sequence
<220>
   <223> Description of Artificial Sequence: Primer for CYP52A3A gene
<400> 42
   cattgaactg agtagccaaa acagcc 26
<210> 43
   <211> 27
   <212> DNA
   <213> Artificial Sequence
<220>
   <223> Description of Artificial Sequence: Primer for CYP52A3A and CYP52A3B genes
<400> 43
   cctacgtttg gtatcgctac tccgttg 27
<210> 44
   <211> 22
   <212> DNA
   <213> Artificial Sequence
<220>
   <223> Description of Artificial Sequence: Primer for CYP52A3A and CYP52A3B genes
<400> 44
   tttccagcca gcaccgtcca ag 22
<210> 45
   <211> 24
   <212> DNA
   <213> Artificial Sequence
<220>
   <223> Description of Artificial Sequence: Primer for CYP52D4A gene
<400> 45
   gcagagccga tctatgttgc gtcc 24
<210> 46
   <211> 25
   <212> DNA
   <213> Artificial Sequence
<220>
   <223> Description of Artificial Sequence: Primer for CYP52D4A gene
<400> 46
   tcattgaatg cttccaggaa cctcg 25
<210> 47
   <211> 20
   <212> DNA
   <213> Artificial Sequence
<220>
   <223> Description of Artificial Sequence: Primer for CYP52A5A and CYP52A5B genes
<400> 47
   aagagggcag ggctcaagag 20
<210> 48
   <211> 21
   <212> DNA
   <213> Artificial Sequence
<220>
   <223> Description of Artificial Sequence: Primer for CYP52A5A and CYP52A5B genes
<400> 48
   tccatgtgaa gatcccatca c 21
<210> 49
   <211> 20
   <212> DNA
   <213> Artificial Sequence
<220>
   <223> Description of Artificial Sequence: Primer for CYP52A8A gene
<400> 49
   cttgaaggcc gtgttgaacg 20
<210> 50
   <211> 21
   <212> DNA
   <213> Artificial Sequence
<220>
   <223> Description of Artificial Sequence: Primer for CYP52A8A gene
<400> 50
   caggatttgt ctgagttgcc g 21
<210> 51
   <211> 28
   <212> DNA
   <213> Artificial Sequence
<220>
   <223> Description of Artificial Sequence: Primer for POX4A and POX4B genes
<400> 51
   ccattgcctt gagatacgcc attggtag 28
<210> 52
   <211> 26
   <212> DNA
   <213> Artificial Sequence
<220>
   <223> Description of Artificial Sequence: Primer for POX4A and POX4B genes
<400> 52
   agccttggtg tcgttctttt caacgg 26
<210> 53
   <211> 26
   <212> DNA
   <213> Artificial Sequence
<220>
   <223> Description of Artificial Sequence: Primer for POX5A gene
<400> 53
   ttgggtttgt ttgtttcctg tgtccg 26
<210> 54
   <211> 27
   <212> DNA
   <213> Artificial Sequence
<220>
   <223> Description of Artificial Sequence: Primer for POX5A gene
<400> 54
   cctttgacct tcaatctggc gtagacg 27
<210> 55
   <211> 26
   <212> DNA
   <213> Artificial Sequence
<220>
   <223> Description of Artificial Sequence: Primer for CPRA gene
<400> 55
   gtttgctgaa tacgctgaag gtgatg 26
<210> 56
   <211> 27
   <212> DNA
   <213> Artificial Sequence
<220>
   <223> Description of Artificial Sequence: Primer for CPRA gene
<400> 56
   tggagctgaa caactctctc gtctcgg 27
<210> 57
   <211> 20
   <212> DNA
   <213> Artificial Sequence
<220>
   <223> Description of Artificial Sequence: Primer for CPRA and CPRB genes
<400> 57 -
   ttcctcaaca cggacagcgg 20
<210> 58
   <211> 24
   <212> DNA
   <213> Artificial Sequence
<220>
   <223> Description of Artificial Sequence: Primer for CPRA and CPRB genes
<400> 58
   agtcaaccag gtgtggaact cgtc 24
<210> 59
   <211> 49
   <212> DNA
   <213> Artificial Sequence
<220>
   <223> Description of Artificial Sequence: Primer for CYP52A5A gene
<400> 59
   ggatcctaat acgactcact atagggagga agagggcagg gctcaagag 49
<210> 60
   <211> 42
   <212> DNA
   <213> Artificial Sequence
<220>
   <223> Description of Artificial Sequence: Primer for CYP52A5A gene
<400> 60
   tccatgtgaa gatcccatca cgagtgtgcc tcttgcccaa ag 42
<210> 61
   <211> 54
   <212> DNA
   <213> Artificial Sequence
<220> -
   <223> Description of Artificial Sequence: Primer for CYP52A1A gene
<400> 61
   ggatcctaat acgactcact atagggaggc cgatgaagtt ttcgacgagt accc 54
<210> 62
   <211> 52
   <212> DNA
   <213> Artificial Sequence
<220>
   <223> Description of Artificial Sequence: Primer for CYP52A1A gene
<400> 62
   aaggctttaa cgtgtccaat ctggtcaaca tagctctgga gtgcttccaa cc 52
<210> 63
   <211> 56
   <212> DNA
   <213> Artificial Sequence
<220>
   <223> Description of Artificial Sequence: Primer for CYP52A2A gene
<400> 63
   ggatcctaat acgactcact atagggagga ttatcgccac atacttcacc aaatgg 56
<210> 64
   <211> 52
   <212> DNA
   <213> Artificial Sequence
<220>
   <223> Description of Artificial Sequence: Primer for CYP52A2A gene
<400> 64
   cgagatcgtg gatacgctgg agtgcgtcgc tcttcttctt caacaattca ag 52
<210> 65
   <211> 49
   <212> DNA
   <213> Artificial Sequence
<220>
   <223> Description of Artificial Sequence: Primer for CYP52A3A gene
<400> 65
   cattgaactg agtagccaaa acagcccatg gtttcaatca atgggaggc 49
<210> 66
   <211> 54
   <212> DNA
   <213> Artificial Sequence
<220>
   <223> Description of Artificial Sequence: Primer for CYP52A3A gene
<400> 66
   ggatcctaat acgactcact atagggaggg ccactcggta actttgtcag ggac 54
<210> 67
   <211> 56
   <212> DNA
   <213> Artificial Sequence
<220>
   <223> Description of Artificial Sequence: Primer for CYP52A3A and CYP52A3B genes
<400> 67
   ggatcctaat acgactcact atagggaggc ctacgtttgg tatcgctact ccgttg 56
<210> 68
   <211> 48
   <212> DNA
   <213> Artificial Sequence
<220>
   <223> Description of Artificial Sequence: Primer for CYP52A3A and CYP52A3B genes
<400> 68
   tttccagcca gcaccgtcca agcaacaagg agtacaagaa atcgtgtc 48
<210> 69
   <211> 53
   <212> DNA
   <213> Artificial Sequence
<220>
   <223> Description of Artificial Sequence: Primer for CYP52D4A gene
<400> 69
   ggatcctaat acgactcact atagggaggg cagagccgat ctatgttgcg tcc 53
<210> 70
   <211> 45
   <212> DNA
   <213> Artificial Sequence
<220>
   <223> Description of Artificial Sequence: Primer for CYP52D4A gene
<400> 70
   tcattgaatg cttccaggaa cctcgccaca tccatcgaga accgg 45
<210> 71
   <211> 49
   <212> DNA
   <213> Artificial Sequence
<220>
   <223> Description of Artificial Sequence: Primer for CYP52A8A gene
<400> 71
   ggatcctaat acgactcact atagggaggc ttgaaggccg tgttgaacg 49
<210> 72
   <211> 46
   <212> DNA
   <213> Artificial Sequence
<220>
   <223> Description of Artificial Sequence: Primer for CYP52A8A gene
<400> 72
   caggatttgt ctgagttgcc gcctgatcaa gataggatcc ttgccg 46
<210> 73
   <211> 56
   <212> DNA
   <213> Artificial Sequence
<220>
   <223> Description of Artificial Sequence: Primer for CPRA gene
<400> 73
   ggatcctaat acgactcact atagggaggg gtttgctgaa tacgctgaag gtgatg 56
<210> 74
   <211> 52
   <212> DNA
   <213> Artificial Sequence
<220>
   <223> Description of Artificial Sequence: Primer for CPRA gene
<400> 74
   tggagctgaa caactctctc gtctcgggtg gtcgaatgga cccttggtca ag 52
<210> 75
   <211> 49
   <212> DNA
   <213> Artificial Sequence
<220>
   <223> Description of Artificial Sequence: Primer for CPRA and CPRB genes
<400> 75
   ggatcctaat acgactcact atagggaggt tcctcaacac ggacagcgg 49
<210> 76
   <211> 49
   <212> DNA-
   <213> Artificial Sequence
<220>
   <223> Description of Artificial Sequence: Primer for CPRA and CPRB genes
<400> 76
   agtcaaccag gtgtggaact cgtcggtggc aacaatgaaa aacaccaag 49
<210> 77
   <211> 57
   <212> DNA
   <213> Artificial Sequence
<220>
   <223> Description of Artificial Sequence: Primer for POX4A and POX4B genes
<400> 77
   ggatcctaat acgactcact atagggaggc cattgccttg agatacgcca ttggtag 57
<210> 78
   <211> 53
   <212> DNA
   <213> Artificial Sequence
<220>
   <223> Description of Artificial Sequence: Primer for POX4A and POX4B genes
<400> 78
   agccttggtg tcgttctttt caacggaagg tggtctcgat ggtgtgttca acc 53
<210> 79
   <211> 55
   <212> DNA
   <213> Artificial Sequence
<220>
   <223> Description of Artificial Sequence: Primer for POX5A gene
<400> 79
   ggatcctaat acgactcact atagggaggt tgggtttgtt tgtttcctgt gtccg 55
<210> 80
   <211> 50
   <212> DNA
   <213> Artificial Sequence
<220>
   <223> Description of Artificial Sequence: Primer for POX5A gene
<400> 80
   cctttgacct tcaatctggc gtagacgcag caccaccgat ccaccacttg 50
<210> 81
   <211> 4206
   <212> DNA
   <213> Candida tropicalis
<400> 81
   catcaagatc atctatgggg ataattacga cagcaacatt gcagaaagag cgttggtcac 60
   aatcgaaaga gcctatggcg ttgccgtcgt tgaggcaaat gacagcacca acaataacga 120
   tggtcccagt gaagagcctt cagaacagtc cattgttgac gcttaaggca cggataatta 180
   cgtggggcaa aggaacgcgg aattagttat ggggggatca aaagcggaag atttgtgttg 240
   cttgtgggtt ttttccttta tttttcatat gatttctttg cgcaagtaac atgtgccaat 300
   ttagtttgtg attagcgtgc cccacaattg gcatcgtgga cgggcgtgtt ttgtcatacc 360
   ccaagtctta actagctcca cagtctcgac ggtgtctcga cgatgtcttc ttccacccct 420
   cccatgaatc attcaaagtt gttgggggat ctccaccaag ggcaccggag ttaatgctta 480
   tgtttctccc actttggttg tgattggggt agtctagtga gttggagatt ttcttttttt 540
   cgcaggtgtc tccgatatcg aaatttgatg aatatagaga gaagccagat cagcacagta 600
   gattgccttt gtagttagag atgttgaaca gcaactagtt gaattacacg ccaccacttg 660
   acagcaagtg cagtgagctg taaacgatgc agccagagtg tcaccaccaa ctgacgttgg 720
   gtggagttgt tgttgttgtt gttggcaggg ccatattgct aaacgaagac aagtagcaca 780
   aaacccaagc ttaagaacaa aaataaaaaa aattcatacg acaattccaa agccattgat 840
   ttacataatc aacagtaaga cagaaaaaac tttcaacatt tcaaagttcc ctttttccta 900
   ttacttcttt tttttcttct ttccttcttt ccttctgttt ttcttacttt atcagtcttt 960
   tacttgtttt tgcaattcct catcctcctc ctactcctcc tcaccatggc tttagacaag 1020
   ttagatttgt atgtcatcat aacattggtg gtcgctgtag ccgcctattt tgctaagaac 1080
   cagttccttg atcagcccca ggacaccggg ttcctcaaca cggacagcgg aagcaactcc 1140
   agagacgtct tgctgacatt gaagaagaat aataaaaaca cgttgttgtt gtttgggtcc 1200
   cagacgggta cggcagaaga ttacgccaac aaattgtcca gagaattgca ctccagattt 1260
   ggcttgaaaa cgatggttgc agatttcgct gattacgatt gggataactt cggagatatc 1320
   accgaagaca tcttggtgtt tttcattgtt gccacctatg gtgagggtga acctaccgat 1380
   aatgccgacg agttccacac ctggttgact gaagaagctg acactttgag taccttgaaa 1440
   tacaccgtgt tcgggttggg taactccacg tacgagttct tcaatgccat tggtagaaag 1500
   tttgacagat tgttgagcga gaaaggtggt gacaggtttg ctgaatacgc tgaaggtgat 1560
   gacggtactg gcaccttgga cgaagatttc atggcctgga aggacaatgt ctttgacgcc 1620
   ttgaagaatg atttgaactt tgaagaaaag gaattgaagt acgaaccaaa cgtgaaattg 1680
   actgagagag acgacttgtc tgctgctgac tcccaagttt ccttgggtga gccaaacaag 1740
   aagtacatca actccgaggg catcgacttg accaagggtc cattcgacca cacccaccca 1800
   tacttggcca gaatcaccga gacgagagag ttgttcagct ccaaggacag acactgtatc 1860
   cacgttgaat ttgacatttc tgaatcgaac ttgaaataca ccaccggtga ccatctagct 1920
   atctggccat ccaactccga cgaaaacatt aagcaatttg ccaagtgttt cggattggaa 1980
   gataaactcg acactgttat tgaattgaag gcgttggact ccacttacac catcccattc 2040
   ccaaccccaa ttacctacgg tgctgtcatt agacaccatt tagaaatctc cggtccagtc 2100
   tcgagacaat tctttttgtc aattgctggg tttgctcctg atgaagaaac aaagaaggct 2160
   tttaccagac ttggtggtga caagcaagaa ttcgccgcca aggtcacccg cagaaagttc 2220
   aacattgccg atgccttgtt atattcctcc aacaacgctc catggtccga tgttcctttt 2280
   gaattcctta ttgaaaacgt tccacacttg actccacgtt actactccat ttcgtcttcg 2340
   tcattgagtg aaaagcaact catcaacgtt actgcagttg ttgaagccga agaagaagct 2400
   gatggcagac cagtcactgg tgttgtcacc aacttgttga agaacgttga aattgtgcaa 2460
   aacaagactg gcgaaaagcc acttgtccac tacgatttga gcggcccaag aggcaagttc 2520
   aacaagttca agttgccagt gcatgtgaga agatccaact ttaagttgcc aaagaactcc 2580
   accaccccag ttatcttgat tggtccaggt actggtgttg ccccattgag aggttttgtc 2640
   agagaaagag ttcaacaagt caagaatggt gtcaatgttg gcaagacttt gttgttttat 2700
   ggttgcagaa actccaacga ggactttttg tacaagcaag aatgggccga gtacgcttct 2760
   gttttgggtg aaaactttga gatgttcaat gccttctcca gacaagaccc atccaagaag 2820
   gtttacgtcc aggataagat tttagaaaac agccaacttg tgcacgagtt gttgactgaa 2880
   ggtgccatta tctacgtctg tggtgatgcc agtagaatgg ctagagacgt gcagaccaca 2940
   atttccaaga ttgttgctaa aagcagagaa attagtgaag acaaggctgc tgaattggtc 3000
   aagtcctgga aggtccaaaa tagataccaa gaagatgttt ggtagactca aacgaatctc 3060
   tctttctccc aacgcattta tgaatcttta ttctcattga agctttacat atgttctaca 3120
   ctttattttt tttttttttt ttattattat attacgaaac ataggtcaac tatatatact 3180
   tgattaaatg ttatagaaac aataactatt atctactcgt ctacttcttt ggcattgaca 3240
   tcaacattac cgttcccatt accgttgccg ttggcaatgc cgggatattt agtacagtat 3300
   ctccaatccg gatttgagct attgtagatc agctgcaagt cattctccac cttcaaccag 3360
   tacttatact tcatctttga cttcaagtcc aagtcataaa tattacaagt tagcaagaac 3420
   ttctggccat ccacgatata gacgttattc acgttattat gcgacgtatg gatgtggtta 3480
   tccttattga acttctcaaa cttcaaaaac aaccccacgt cccgcaacgt cattatcaac 3540
   gacaagttct ggctcacgtc gtcggagctc gtcaagttct caattagatc gttcttgtta 3600
   ttgatcttct ggtactttct caattgctgg aacacattgt cctcgttgtt caaatagatc 3660
   ttgaacaact ttttcaacgg gatcaacttc tcaatctggg ccaagatctc cgccgggatc 3720
   ttcagaaaca agtcctgcaa cccctggtcg atggtctccg ggtacaacaa gtccaagggg 3780
   cagaagtgtc taggcacgtg tttcaactgg ttcaacgaac atgttcgaca gtagttcgag 3840
   ttatagttat cgtacaacca ttttggtttg atttcgaaaa tgacggagct gatgccatca 3900
   ttctcctggt tcctctcata gtacaactgg cacttcttcg agaggctcaa ttcctcgtag 3960
   ttcccgtcca agatattcgg caacaagagc ccgtaccgct cacggagcat caagtcgtgg 4020
   ccctggttgt tcaacttgtt gatgaagtcc gaggtcaaga caatcaactg gatgtcgatg 4080
   atctggtgcg ggaacaagtt cttgcatttt agctcgatga agtcgtacaa ctcacacgtc 4140
   gagatatact cctgttcctc cttcaagagc cggatccgca agagcttgtg cttcaagtag 4200
   tcgttg                                                            4206
<210> 82
   <211> 4145
   <212> DNA
   <213> Candida tropicalis
<400> 82
   tatatgatat atgatatatc ttcctgtgta attattattc gtattcgtta atacttacta 60
   catttttttt tctttattta tgaagaaaag gagagttcgt aagttgagtt gagtagaata 120
   ggctgttgtg catacgggga gcagaggaga gtatccgacg aggaggaact gggtgaaatt 180
   tcatctatgc tgttgcgtcc tgtactgtac tgtaaatctt agatttccta gaggttgttc 240
   tagcaaataa agtgtttcaa gatacaattt tacaggcaag ggtaaaggat caactgatta 300
   gcggaagatt ggtgttgcct gtggggttct tttatttttc atatgatttc tttgcgcgag 360
   taacatgtgc caatctagtt tatgattagc gtacctccac aattggcatc ttggacgggc 420
   gtgttttgtc ttaccccaag ccttatttag ttccacagtc tcgacggtgt ctcgccgatg 480
   tcttctccca cccctcgcag gaatcattcg aagttgttgg gggatctcct ccgcagttta 540
   tgttcatgtc tttcccactt tggttgtgat tggggtagcg tagtgagttg gtgattttct 600
   tttttcgcag gtgtctccga tatcgaagtt tgatgaatat aggagccaga tcagcatggt 660
   atattgcctt tgtagataga gatgttgaac aacaactagc tgaattacac accaccgcta 720
   aacgatgcgc acagggtgtc accgccaact gacgttgggt ggagttgttg ttggcagggc 780
   catattgcta aacgaagaga agtagcacaa aacccaaggt taagaacaat taaaaaaatt 840
   catacgacaa ttccacagcc atttacataa tcaacagcga caaatgagac agaaaaaact 900
   ttcaacattt caaagttccc tttttcctat tacttctttt tttctttcct tcctttcatt 960
   tcctttcctt ctgcttttat tactttacca gtcttttgct tgtttttgca attcctcatc 1020
   ctcctcctca ccatggcttt agacaagtta gatttgtatg tcatcataac attggtggtc 1080
   gctgtggccg cctattttgc taagaaccag ttccttgatc agccccagga caccgggttc 1140
   ctcaacacgg acagcggaag caactccaga gacgtcttgc tgacattgaa gaagaataat 1200
   aaaaacacgt tgttgttgtt tgggtcccag accggtacgg cagaagatta cgccaacaaa 1260
   ttgtcaagag aattgcactc cagatttggc ttgaaaacca tggttgcaga tttcgctgat 1320
   tacgattggg ataacttcgg agatatcacc gaagatatct tggtgttttt catcgttgcc 1380
   acctacggtg agggtgaacc taccgacaat gccgacgagt tccacacctg gttgactgaa 1440
   gaagctgaca ctttgagtac tttgagatat accgtgttcg ggttgggtaa ctccacctac 1500
   gagttcttca atgctattgg tagaaagttt gacagattgt tgagtgagaa aggtggtgac 1560
   agatttgctg aatatgctga aggtgacgac ggcactggca ccttggacga agatttcatg 1620
   gcctggaagg ataatgtctt tgacgccttg aagaatgact tgaactttga agaaaaggaa 1680
   ttgaagtacg aaccaaacgt gaaattgact gagagagatg acttgtctgc tgccgactcc 1740
   caagtttcct tgggtgagcc aaacaagaag tacatcaact ccgagggcat cgacttgacc 1800
   aagggtccat tcgaccacac ccacccatac ttggccagga tcaccgagac cagagagttg 1860
   ttcagctcca aggaaagaca ctgtattcac gttgaatttg acatttctga atcgaacttg 1920
   aaatacacca ccggtgacca tctagccatc tggccatcca actccgacga aaacatcaag 1980
   caatttgcca agtgtttcgg attggaagat aaactcgaca ctgttattga attgaaggca 2040
   ttggactcca cttacaccat tccattccca actccaatta cttacggtgc tgtcattaga 2100
   caccatttag aaatctccgg tccagtctcg agacaattct ttttgtcgat tgctgggttt 2160
   gctcctgatg aagaaacaaa gaagactttc accagacttg gtggtgacaa acaagaattc 2220
   gccaccaagg ttacccgcag aaagttcaac attgccgatg ccttgttata ttcctccaac 2280
   aacactccat ggtccgatgt tccttttgag ttccttattg aaaacatcca acacttgact 2340
   ccacgttact actccatttc ttcttcgtcg ttgagtgaaa aacaactcat caatgttact 2400
   gcagtcgttg aggccgaaga agaagccgat ggcagaccag tcactggtgt tgttaccaac 2460
   ttgttgaaga acattgaaat tgcgcaaaac aagactggcg aaaagccact tgttcactac 2520
   gatttgagcg gcccaagagg caagttcaac aagttcaagt tgccagtgca cgtgagaaga 2580
   tccaacttta agttgccaaa gaactccacc accccagtta tcttgattgg tccaggtact 2640
   ggtgttgccc cattgagagg tttcgttaga gaaagagttc aacaagtcaa gaatggtgtc 2700
   aatgttggca agactttgtt gttttatggt tgcagaaact ccaacgagga ctttttgtac 2760
   aagcaagaat gggccgagta cgcttctgtt ttgggtgaaa actttgagat gttcaatgcc 2820
   ttctctagac aagacccatc caagaaggtt tacgtccagg ataagatttt agaaaacagc 2880
   caacttgtgc acgaattgtt gaccgaaggt gccattatct acgtctgtgg tgacgccagt 2940
   agaatggcca gagacgtcca gaccacgatc tccaagattg ttgccaaaag cagagaaatc 3000
   agtgaagaca aggccgctga attggtcaag tcctggaaag tccaaaatag ataccaagaa 3060
   gatgtttggt agactcaaac gaatctctct ttctcccaac gcatttatga atattctcat 3120
   tgaagtttta catatgttct atatttcatt ttttttttat tatattacga aacataggtc 3180
   aactatatat acttgattaa atgttataga aacaataatt attatctact cgtctacttc 3240
   tttggcattg gcattggcat tggcattggc attgccgttg ccgttggtaa tgccgggata 3300
   tttagtacag tatctccaat ccggatttga gctattgtaa atcagctgca agtcattctc 3360
   caccttcaac cagtacttat acttcatctt tgacttcaag tccaagtcat aaatattaca 3420
   agttagcaag aacttctggc catccacaat atagacgtta ttcacgttat tatgcgacgt 3480
   atggatatgg ttatccttat tgaacttctc aaacttcaaa aacaacccca cgtcccgcaa 3540
   cgtcattatc aacgacaagt tctgactcac gtcgtcggag ctcgtcaagt tctcaattag 3600
   atcgttcttg ttattgatct tctggtactt tctcaactgc tggaacacat tgtcctcgtt 3660
   gttcaaatag atcttgaaca acttcttcaa gggaatcaac ttttcgatct gggccaagat 3720
   ttccgccggg atcttcagaa acaagtcctg caacccctgg tcgatggtct cggggtacaa 3780
   caagtctaag gggcagaagt gtctaggcac gtgtttcaac tggttcaagg aacatgttcg 3840
   acagtagttc gagttatagt tatcgtacaa ccactttggc ttgatttcga aaatgacgga 3900
   gctgatccca tcattctcct ggttcctttc atagtacaac tggcatttct tcgagagact 3960
   caactcctcg tagttcccgt ccaagatatt cggcaacaag agcccgtagc gctcacggag 4020
   catcaagtcg tggccctggt tgttcaactt gttgatgaag tccgatgtca agacaatcaa 4080
   ctggatgtcg atgatctggt gcggaaacaa gttcttgcac tttagctcga tgaagtcgta 4140
   caact 4145
<210> 83
   <211> 679
   <212> PRT
   <213> Candida tropicalis
<400> 83
   Met Ala Leu Asp Lys Leu Asp Leu Tyr Val Ile Ile Thr Leu Val Val
   1 5 10 15
   Ala Val Ala Ala Tyr Phe Ala Lys Asn Gln Phe Leu Asp Gln Pro Gln
   20 25 30
   Asp Thr Gly Phe Leu Asn Thr Asp Ser Gly Ser Asn Ser Arg Asp Val
   35 40 45
   Leu Leu Thr Leu Lys Lys Asn Asn Lys Asn Thr Leu Leu Leu Phe Gly
   50 55 60
   Ser Gln Thr Gly Thr Ala Glu Asp Tyr Ala Asn Lys Leu Ser Arg Glu
   65 70 75 80
   Leu His Ser Arg Phe Gly Leu Lys Thr Met Val Ala Asp Phe Ala Asp
   85 90 95
   Tyr Asp Trp Asp Asn Phe Gly Asp Ile Thr Glu Asp Ile Leu Val Phe
   100 105 110
   Phe Ile Val Ala Thr Tyr Gly Glu Gly Glu Pro Thr Asp Asn Ala Asp
   115 120 125
   Glu Phe His Thr Trp Leu Thr Glu Glu Ala Asp Thr Leu Ser Thr Leu
   130 135 140
   Lys Tyr Thr Val Phe Gly Leu Gly Asn Ser Thr Tyr Glu Phe Phe Asn
   145 150 155 160
   Ala Ile Gly Arg Lys Phe Asp Arg Leu Leu Ser Glu Lys Gly Gly Asp
   165 170 175
   Arg Phe Ala Glu Tyr Ala Glu Gly Asp Asp Gly Thr Gly Thr Leu Asp
   180 185 190
   Glu Asp Phe Met Ala Trp Lys Asp Asn Val Phe Asp Ala Leu Lys Asn
   195 200 205
   Asp Leu Asn Phe Glu Glu Lys Glu Leu Lys Tyr Glu Pro Asn Val Lys
   210 215 220
   Leu Thr Glu Arg Asp Asp Leu Ser Ala Ala Asp Ser Gln Val Ser Leu
   225 230 235 240
   Gly Glu Pro Asn Lys Lys Tyr Ile Asn Ser Glu Gly Ile Asp Leu Thr
   245 250 255
   Lys Gly Pro Phe Asp His Thr His Pro Tyr Leu Ala Arg Ile Thr Glu
   260 265 270
   Thr Arg Glu Leu Phe Ser Ser Lys Asp Arg His Cys Ile His Val Glu
   275 280 285
   Phe Asp Ile Ser Glu Ser Asn Leu Lys Tyr Thr Thr Gly Asp His Leu
   290 295 300
   Ala Ile Trp Pro Ser Asn Ser Asp Glu Asn Ile Lys Gln Phe Ala Lys
   305 310 315 320
   Cys Phe Gly Leu Glu Asp Lys Leu Asp Thr Val Ile Glu Leu Lys Ala
   325 330 335
   Leu Asp Ser Thr Tyr Thr Ile Pro Phe Pro Thr Pro Ile Thr Tyr Gly
   340 345 350
   Ala Val Ile Arg His His Leu Glu Ile Ser Gly Pro Val Ser Arg Gln
   355 360 365
   Phe Phe Leu Ser Ile Ala Gly Phe Ala Pro Asp Glu Glu Thr Lys Lys
   370 375 380
   Ala Phe Thr Arg Leu Gly Gly Asp Lys Gln Glu Phe Ala Ala Lys Val
   385 390 395 400
   Thr Arg Arg Lys Phe Asn Ile Ala Asp Ala Leu Leu Tyr Ser Ser Asn
   405 410 415
   Asn Ala Pro Trp Ser Asp Val Pro Phe Glu Phe Leu Ile Glu Asn Val
   420 425 430
   Pro His Leu Thr Pro Arg Tyr Tyr Ser Ile Ser Ser Ser Ser Leu Ser
   435 440 445
   Glu Lys Gln Leu Ile Asn Val Thr Ala Val Val Glu Ala Glu Glu Glu
   450 455 460
   Ala Asp Gly Arg Pro Val Thr Gly Val Val Thr Asn Leu Leu Lys Asn
   465 470 475 480
   Val Glu Ile Val Gln Asn Lys Thr Gly Glu Lys Pro Leu Val His Tyr
   485 490 495
   Asp Leu Ser Gly Pro Arg Gly Lys Phe Asn Lys Phe Lys Leu Pro Val
   500 505 510
   His Val Arg Arg Ser Asn Phe Lys Leu Pro Lys Asn Ser Thr Thr Pro
   515 520 525
   Val Ile Leu Ile Gly Pro Gly Thr Gly Val Ala Pro Leu Arg Gly Phe
   530 535 540
   Val Arg Glu Arg Val Gln Gln Val Lys Asn Gly Val Asn Val Gly Lys
   545 550 555 560
   Thr Leu Leu Phe Tyr Gly Cys Arg Asn Ser Asn Glu Asp Phe Leu Tyr
   565 570 575
   Lys Gln Glu Trp Ala Glu Tyr Ala Ser Val Leu Gly Glu Asn Phe Glu
   580 585 590
   Met Phe Asn Ala Phe Ser Arg Gln Asp Pro Ser Lys Lys Val Tyr Val
   595 600 605
   Gln Asp Lys Ile Leu Glu Asn Ser Gln Leu Val His Glu Leu Leu Thr
   610 615 620
   Glu Gly Ala Ile Ile Tyr Val Cys Gly Asp Ala Ser Arg Met Ala Arg
   625 630 635 640
   Asp Val Gln Thr Thr Ile Ser Lys Ile Val Ala Lys Ser Arg Glu Ile
   645 650 655
   Ser Glu Asp Lys Ala Ala Glu Leu Val Lys Ser Trp Lys Val Gln Asn
   660 665 670
   Arg Tyr Gln Glu Asp Val Trp
   675
<210> 84
   <211> 679
   <212> PRT
   <213> Candida tropicalis
<400> 84
   Met Ala Leu Asp Lys Leu Asp Leu Tyr Val Ile Ile Thr Leu Val Val
   1 5 10 15
   Ala Val Ala Ala Tyr Phe Ala Lys Asn Gln Phe Leu Asp Gln Pro Gln
   20 25 30
   Asp Thr Gly Phe Leu Asn Thr Asp Ser Gly Ser Asn Ser Arg Asp Val
   35 40 45
   Leu Leu Thr Leu Lys Lys Asn Asn Lys Asn Thr Leu Leu Leu Phe Gly
   50 55 60
   Ser Gln Thr Gly Thr Ala Glu Asp Tyr Ala Asn Lys Leu Ser Arg Glu
   65 70 75 80
   Leu His Ser Arg Phe Gly Leu Lys Thr Met Val Ala Asp Phe Ala Asp
   85 90 95
   Tyr Asp Trp Asp Asn Phe Gly Asp Ile Thr Glu Asp Ile Leu Val Phe
   100 105 110
   Phe Ile Val Ala Thr Tyr Gly Glu Gly Glu Pro Thr Asp Asn Ala Asp
   115 120 125
   Glu Phe His Thr Trp Leu Thr Glu Glu Ala Asp Thr Leu Ser Thr Leu
       130                 135                 140
   Arg Tyr Thr Val Phe Gly Leu Gly Asn Ser Thr Tyr Glu Phe Phe Asn
   145                 150                 155                 160
   Ala Ile Gly Arg Lys Phe Asp Arg Leu Leu Ser Glu Lys Gly Gly Asp
                   165                 170                 175
   Arg Phe Ala Glu Tyr Ala Glu Gly Asp Asp Gly Thr Gly Thr Leu Asp
               180                 185                 190
   Glu Asp Phe Met Ala Trp Lys Asp Asn Val Phe Asp Ala Leu Lys Asn
           195                 200                 205
   Asp Leu Asn Phe Glu Glu Lys Glu Leu Lys Tyr Glu Pro Asn Val Lys
       210                 215                 220
   Leu Thr Glu Arg Asp Asp Leu Ser Ala Ala Asp Ser Gln Val Ser Leu
   225                 230                 235                 240
   Gly Glu Pro Asn Lys Lys Tyr Ile Asn Ser Glu Gly Ile Asp Leu Thr
                   245                 250                 255
   Lys Gly Pro Phe Asp His Thr His Pro Tyr Leu Ala Arg Ile Thr Glu
               260                 265                 270
   Thr Arg Glu Leu Phe Ser Ser Lys Glu Arg His Cys Ile His Val Glu
           275                 280                 285
   Phe Asp Ile Ser Glu Ser Asn Leu Lys Tyr Thr Thr Gly Asp His Leu
       290                 295                 300
   Ala Ile Trp Pro Ser Asn Ser Asp Glu Asn Ile Lys Gln Phe Ala Lys
   305                 310                 315                 320
   Cys Phe Gly Leu Glu Asp Lys Leu Asp Thr Val Ile Glu Leu Lys Ala
                   325                 330                 335
   Leu Asp Ser Thr Tyr Thr Ile Pro Phe Pro Thr Pro Ile Thr Tyr Gly
               340                 345                 350
   Ala Val Ile Arg His His Leu Glu Ile Ser Gly Pro Val Ser Arg Gln
           355                 360                 365
   Phe Phe Leu Ser Ile Ala Gly Phe Ala Pro Asp Glu Glu Thr Lys Lys
       370                 375                 380
   Thr Phe Thr Arg Leu Gly Gly Asp Lys Gln Glu Phe Ala Thr Lys Val
   385 390 395 400
   Thr Arg Arg Lys Phe Asn Ile Ala Asp Ala Leu Leu Tyr Ser Ser Asn
   405 410 415
   Asn Thr Pro Trp Ser Asp Val Pro Phe Glu Phe Leu Ile Glu Asn Ile
   420 425 430
   Gln His Leu Thr Pro Arg Tyr Tyr Ser Ile Ser Ser Ser Ser Leu Ser
   435 440 445
   Glu Lys Gln Leu Ile Asn Val Thr Ala Val Val Glu Ala Glu Glu Glu
   450 455 460
   Ala Asp Gly Arg Pro Val Thr Gly Val Val Thr Asn Leu Leu Lys Asn
   465 470 475 480
   Ile Glu Ile Ala Gln Asn Lys Thr Gly Glu Lys Pro Leu Val His Tyr
   485 490 495
   Asp Leu Ser Gly Pro Arg Gly Lys Phe Asn Lys Phe Lys Leu Pro Val
   500 505 510
   His Val Arg Arg Ser Asn Phe Lys Leu Pro Lys Asn Ser Thr Thr Pro
   515 520 525
   Val Ile Leu Ile Gly Pro Gly Thr Gly Val Ala Pro Leu Arg Gly Phe
   530 535 540
   Val Arg Glu Arg Val Gln Gln Val Lys Asn Gly Val Asn Val Gly Lys
   545 550 555 560
   Thr Leu Leu Phe Tyr Gly Cys Arg Asn Ser Asn Glu Asp Phe Leu Tyr
   565 570 575
   Lys Gln Glu Trp Ala Glu Tyr Ala Ser Val Leu Gly Glu Asn Phe Glu
   580 585 590
   Met Phe Asn Ala Phe Ser Arg Gln Asp Pro Ser Lys Lys Val Tyr Val
   595 600 605
   Gln Asp Lys Ile Leu Glu Asn Ser Gln Leu Val His Glu Leu Leu Thr
   610 615 620
   Glu Gly Ala Ile Ile Tyr Val Cys Gly Asp Ala Ser Arg Met Ala Arg
   625 630 635 640
   Asp Val Gln Thr Thr Ile Ser Lys Ile Val Ala Lys Ser Arg Glu Ile
                   645                 650                 655
   Ser Glu Asp Lys Ala Ala Glu Leu Val Lys Ser Trp Lys Val Gln Asn
               660                 665                 670
   Arg Tyr Gln Glu Asp Val Trp
           675
<210> 85
   <211> 4115
   <212> DNA
   <213> Candida tropicalis
<400> 85
   catatgcgct aatcttcttt ttctttttat cacaggagaa actatcccac ccccacttcg 60
   aaacacaatg acaactcctg cgtaacttgc aaattcttgt ctgactaatt gaaaactccg 120
   gacgagtcag acctccagtc aaacggacag acagacaaac acttggtgcg atgttcatac 180
   ctacagacat gtcaacgggt gttagacgac ggtttcttgc aaagacaggt gttggcatct 240
   cgtacgatgg caactgcagg aggtgtcgac ttctccttta ggcaatagaa aaagactaag 300
   agaacagcgt ttttacaggt tgcattggtt aatgtagtat ttttttagtc ccagcattct 360
   gtgggttgct ctgggtttct agaataggaa atcacaggag aatgcaaatt cagatggaag 420
   aacaaagaga taaaaaacaa aaaaaaactg agttttgcac caatagaatg tttgatgata 480
   tcatccactc gctaaacgaa tcatgtgggt gatcttctct ttagttttgg tctatcataa 540
   aacacatgaa agtgaaatcc aaatacacta cactccgggt attgtccttc gttttacaga 600
   tgtctcattg tcttactttt gaggtcatag gagttgcctg tgagagatca cagagattat 660
   cacactcaca tttatcgtag tttcctatct catgctgtgt gtctctggtt ggttcatgag 720
   tttggattgt tgtacattaa aggaatcgct ggaaagcaaa gctaactaaa ttttctttgt 780
   cacaggtaca ctaacctgta aaacttcact gccacgccag tctttcctga ttgggcaagt 840
   gcacaaacta caacctgcaa aacagcactc cgcttgtcac aggttgtctc ctctcaacca 900
   acaaaaaaat aagattaaac tttctttgct catgcatcaa tcggagttat ctctgaaaga 960
   gttgcctttg tgtaatgtgt gccaaactca aactgcaaaa ctaaccacag aatgatttcc 1020
   ctcacaatta tataaactca cccacatttc cacagaccgt aatttcatgt ctcactttct 1080
   cttttgctct tcttttactt agtcaggttt gataacttcc ttttttatta ccctatctta 1140
   tttatttatt tattcattta taccaaccaa ccaaccatgg ccacacaaga aatcatcgat 1200
   tctgtacttc cgtacttgac caaatggtac actgtgatta ctgcagcagt attagtcttc 1260
   cttatctcca caaacatcaa gaactacgtc aaggcaaaga aattgaaatg tgtcgatcca 1320
   ccatacttga aggatgccgg tctcactggt attctgtctt tgatcgccgc catcaaggcc 1380
   aagaacgacg gtagattggc taactttgcc gatgaagttt tcgacgagta cccaaaccac 1440
   accttctact tgtctgttgc cggtgctttg aagattgtca tgactgttga cccagaaaac 1500
   atcaaggctg tcttggccac ccaattcact gacttctcct tgggtaccag acacgcccac 1560
   tttgctcctt tgttgggtga cggtatcttc accttggacg gagaaggttg gaagcactcc 1620
   agagctatgt tgagaccaca gtttgctaga gaccagattg gacacgttaa agccttggaa 1680
   ccacacatcc aaatcatggc taagcagatc aagttgaacc agggaaagac tttcgatatc 1740
   caagaattgt tctttagatt taccgtcgac accgctactg agttcttgtt tggtgaatcc 1800
   gttcactcct tgtacgatga aaaattgggc atcccaactc caaacgaaat cccaggaaga 1860
   gaaaactttg ccgctgcttt caacgtttcc caacactact tggccaccag aagttactcc 1920
   cagacttttt actttttgac caaccctaag gaattcagag actgtaacgc caaggtccac 1980
   cacttggcca agtactttgt caacaaggcc ttgaacttta ctcctgaaga actcgaagag 2040
   aaatccaagt ccggttacgt tttcttgtac gaattggtta agcaaaccag agatccaaag 2100
   gtcttgcaag atcaattgtt gaacattatg gttgccggaa gagacaccac tgccggtttg 2160
   ttgtcctttg ctttgtttga attggctaga cacccagaga tgtggtccaa gttgagagaa 2220
   gaaatcgaag ttaactttgg tgttggtgaa gactcccgcg ttgaagaaat taccttcgaa 2280
   gccttgaaga gatgtgaata cttgaaggct atccttaacg aaaccttgcg tatgtaccca 2340
   tctgttcctg tcaactttag aaccgccacc agagacacca ctttgccaag aggtggtggt 2400
   gctaacggta ccgacccaat ctacattcct aaaggctcca ctgttgctta cgttgtctac 2460
   aagacccacc gtttggaaga atactacggt aaggacgcta acgacttcag accagaaaga 2520
   tggtttgaac catctactaa gaagttgggc tgggcttatg ttccattcaa cggtggtcca 2580
   agagtctgct tgggtcaaca attcgccttg actgaagctt cttatgtgat cactagattg 2640
   gcccagatgt ttgaaactgt ctcatctgat ccaggtctcg aataccctcc accaaagtgt 2700
   attcacttga ccatgagtca caacgatggt gtctttgtca agatgtaaag tagtcgatgc 2760
   tgggtattcg attacatgtg tataggaaga ttttggtttt ttattcgttc ttttttttaa 2820
   tttttgttaa attagtttag agatttcatt aatacataga tgggtgctat ttccgaaact 2880
   ttacttctat cccctgtatc ccttattatc cctctcagtc acatgattgc tgtaattgtc 2940
   gtgcaggaca caaactccct aacggactta aaccataaac aagctcagaa ccataagccg 3000
   acatcactcc ttcttctctc ttctccaacc aatagcatgg acagacccac cctcctatcc 3060
   gaatcgaaga cccttattga ctccataccc acctggaagc ccctcaagcc acacacgtca 3120
   tccagcccac ccatcaccac atccctctac tcgacaacgt ccaaagacgg cgagttctgg 3180
   tgtgcccgga aatcagccat cccggccaca tacaagcagc cgttgattgc gtgcatactc 3240
   ggcgagccca caatgggagc cacgcattcg gaccatgaag caaagtacat tcacgagatc 3300
   acgggtgttt cagtgtcgca gattgagaag ttcgacgatg gatggaagta cgatctcgtt 3360
   gcggattacg acttcggtgg gttgttatct aaacgaagat tctatgagac gcagcatgtg 3420
   tttcggttcg aggattgtgc gtacgtcatg agtgtgcctt ttgatggacc caaggaggaa 3480
   ggttacgtgg ttgggacgta cagatccatt gaaaggttga gctggggtaa agacggggac 3540
   gtggagtgga ccatggcgac gacgtcggat cctggtgggt ttatcccgca atggataact 3600
   cgattgagca tccctggagc aatcgcaaaa gatgtgccta gtgtattaaa ctacatacag 3660
   aaataaaaac gtgtcttgat tcattggttt ggttcttgtt gggttccgag ccaatatttc 3720
   acatcatctc ctaaattctc caagaatccc aacgtagcgt agtccagcac gccctctgag 3780
   atcttattta atatcgactt ctcaaccacc ggtggaatcc cgttcagacc attgttacct 3840
   gtagtgtgtt tgctcttgtt cttgatgaca atgatgtatt tgtcacgata cctgaaataa 3900
   taaaacatcc agtcattgag cttattactc gtgaacttat gaaagaactc attcaagccg 3960
   ttcccaaaaa acccagaatt gaagatcttg ctcaactggt catgcaagta gtagatcgcc 4020
   atgatctgat actttaccaa gctatcctct ccaagttctc ccacgtacgg caagtacggc 4080
   aacgagctct ggaagctttg ttgtttgggg tcata                            4115
<210> 86
   <211> 3948
   <212> DNA
   <213> Candida tropicalis
<400> 86 .
   gacctgtgac gcttccggtg tcttgccacc agtctccaag ttgaccgacg cccaagtcat 60
   gtaccacttt atttccggtt acacttccaa gatggctggt actgaagaag gtgtcacgga 120
   accacaagct actttctccg cttgtttcgg tcaaccattc ttggtgttgc acccaatgaa 180
   gtacgctcaa caattgtctg acaagatctc gcaacacaag gctaacgcct ggttgttgaa 240
   caccggttgg gttggttctt ctgctgctag aggtggtaag agatgctcat tgaagtacac 300
   cagagccatt ttggacgcta tccactctgg tgaattgtcc aaggttgaat acgaaacttt 360
   cccagtcttc aacttgaatg tcccaacctc ctgtccaggt gtcccaagtg aaatcttgaa 420
   cccaaccaag gcctggaccg gaaggtgttg actccttcaa caaggaaatc aagtctttgg 480
   ctggtaagtt tgctgaaaac ttcaagacct atgctgacca agctaccgct gaagtgagag 540
   ctgcaggtcc agaagcttaa agatatttat tcattattta gtttgcctat ttatttctca 600
   ttacccatca tcattcaaca ctatatataa agttacttcg gatatcattg taatcgtgcg 660
   tgtcgcaatt ggatgatttg gaactgcgct tgaaacggat tcatgcacga agcggagata 720
   aaagattacg taatttatct cctgagacaa ttttagccgt gttcacacgc ccttctttgt 780
   tctgagcgaa ggataaataa ttagacttcc acagctcatt ctaatttccg tcacgcgaat 840
   attgaagggg ggtacatgtg gccgctgaat gtgggggcag taaacgcagt ctctcctctc 900
   ccaggaatag tgcaacggag gaaggataac ggatagaaag cggaatgcga ggaaaatttt 960
   gaacgcgcaa gaaaagcaat atccgggcta ccaggttttg agccagggaa cacactccta 1020
   tttctgctca atgactgaac atagaaaaaa caccaagacg caatgaaacg cacatggaca 1080
   tttagacctc cccacatgtg atagtttgtc ttaacagaaa agtataataa gaacccatgc 1140
   cgtccctttt ctttcgccgc ttcaactttt ttttttttat cttacacaca tcacgaccat 1200
   gactgtacac gatattatcg ccacatactt caccaaatgg tacgtgatag taccactcgc 1260
   tttgattgct tatagagtcc tcgactactt ctatggcaga tacttgatgt acaagcttgg 1320
   tgctaaacca tttttccaga aacagacaga cggctgtttc ggattcaaag ctccgcttga 1380
   attgttgaag aagaagagcg acggtaccct catagacttc acactccagc gtatccacga 1440
   tctcgatcgt cccgatatcc caactttcac attcccggtc ttttccatca accttgtcaa 1500
   tacccttgag ccggagaaca tcaaggccat cttggccact cagttcaacg atttctcctt 1560
   gggtaccaga cactcgcact ttgctccttt gttgggtgat ggtatcttta cgttggatgg 1620
   cgccggctgg aagcacagca gatctatgtt gagaccacag tttgccagag aacagatttc 1680
   ccacgtcaag ttgttggagc cacacgttca ggtgttcttc aaacacgtca gaaaggcaca 1740
   gggcaagact tttgacatcc aggaattgtt tttcagattg accgtcgact ccgccaccga 1800
   gtttttgttt ggtgaatccg ttgagtcctt gagagatgaa tctatcggca tgtccatcaa 1860
   tgcgcttgac tttgacggca aggctggctt tgctgatgct tttaactatt cgcagaatta 1920
   tttggcttcg agagcggtta tgcaacaatt gtactgggtg ttgaacggga aaaagtttaa 1980
   ggagtgcaac gctaaagtgc acaagtttgc tgactactac gtcaacaagg ctttggactt 2040
   gacgcctgaa caattggaaa agcaggatgg ttatgtgttt ttgtacgaat tggtcaagca 2100
   aaccagagac aagcaagtgt tgagagacca attgttgaac atcatggttg ctggtagaga 2160
   caccaccgcc ggtttgttgt cgtttgtttt ctttgaattg gccagaaacc cagaagttac 2220
   caacaagttg agagaagaaa ttgaggacaa gtttggactc ggtgagaatg ctagtgttga 2280
   agacatttcc tttgagtcgt tgaagtcctg tgaatacttg aaggctgttc tcaacgaaac 2340
   cttgagattg tacccatccg tgccacagaa tttcagagtt gccaccaaga acactaccct 2400
   cccaagaggt ggtggtaagg acgggttgtc tcctgttttg gtgagaaagg gtcagaccgt 2460
   tatttacggt gtctacgcag cccacagaaa cccagctgtt tacggtaagg acgctcttga 2520
   gtttagacca gagagatggt ttgagccaga gacaaagaag cttggctggg ccttcctccc 2580
   attcaacggt ggtccaagaa tctgtttggg acagcagttt gccttgacag aagcttcgta 2640
   tgtcactgtc aggttgctcc aggagtttgc acacttgtct atggacccag acaccgaata 2700
   tccacctaag aaaatgtcgc atttgaccat gtcgcttttc gacggtgcca atattgagat 2760
   gtattagagg gtcatgtgtt attttgattg tttagtttgt aattactgat taggttaatt 2820
   catggattgt tatttattga taggggtttg cgcgtgttgc attcacttgg gatcgttcca 2880
   ggttgatgtt tccttccatc ctgtcgagtc aaaaggagtt ttgttttgta actccggacg 2940
   atgttttaaa tagaaggtcg atctccatgt gattgttttg actgttactg tgattatgta 3000
   atctgcggac gttatacaag catgtgattg tggttttgca gccttttgca cgacaaatga 3060
   tcgtcagacg attacgtaat ctttgttaga ggggtaaaaa aaaacaaaat ggcagccaga 3120
   atttcaaaca ttctgcaaac aatgcaaaaa atgggaaact ccaacagaca aaaaaaaaaa 3180
   ctccgcagca ctccgaaccc acagaacaat ggggcgccag aattattgac tattgtgact 3240
   tttttacgct aacgctcatt gcagtgtagt gcgtcttaca cggggtattg ctttctacaa 3300
   tgcaagggca cagttgaagg tttgcaccta acgttgcccc gtgtcaactc aatttgacga 3360
   gtaacttcct aagctcgaat tatgcagctc gtgcgtcaac ctatgtgcag gaaagaaaaa 3420
   atccaaaaaa atcgaaaatg cgactttcga ttttgaataa accaaaaaga aaaatgtcgc 3480
   acttttttct cgctctcgct ctctcgaccc aaatcacaac aaatcctcgc gcgcagtatt 3540
   tcgacgaaac cacaacaaat aaaaaaaaca aattctacac cacttctttt tcttcaccag 3600
   tcaacaaaaa acaacaaatt atacaccatt tcaacgattt ttgctcttat aaatgctata 3660
   taatggttta attcaactca ggtatgttta ttttactgtt ttcagctcaa gtatgttcaa 3720
   atactaacta cttttgatgt ttgtcgcttt tctagaatca aaacaacgcc cacaacacgc 3780
   cgagcttgtc gaatagacgg tttgtttact cattagatgg tcccagatta cttttcaagc 3840
   caaagtctct cgagttttgt ttgctgtttc cccaattcct aactatgaag ggtttttata 3900
   aggtccaaag accccaaggc atagtttttt tggttccttc ttgtcgtg          3948
<210> 87
   <211> 3755
   <212> DNA
   <213> Candida tropicalis
<400> 87
   gctcaacaat tgtctgacaa gatctcgcaa cacaaggcta acgcctggtt gttgaacact 60
   ggttgggttg gttcttctgc tgctagaggt ggtaagagat gttcattgaa gtacaccaga 120
   gccattttgg acgctatcca ctctggtgaa ttgtccaagg ttgaatacga gactttccca 180
   gtcttcaact tgaatgtccc aacctcctgc ccaggtgtcc caagtgaaat cttgaaccca 240
   accaaggcct ggaccgaagg tgttgactcc ttcaacaagg aaatcaagtc tttggctggt 300
   aagtttgctg aaaacttcaa gacctatgct gaccaagcta ccgctgaagt tagagctgca 360
   ggtccagaag cttaaagata tttattcact atttagtttg cctatttatt tctcatcacc 420
   catcatcatt caacaatata tataaagtta tttcggaact catatatcat tgtaatcgtg 480
   cgtgttgcaa ttgggtaatt tgaaactgta gttggaacgg attcatgcac gatgcggaga 540
   taacacgaga ttatctccta agacaatttt ggcctcattc acacgccctt cttctgagct 600
   aaggataaat aattagactt cacaagttca ttaaaatatc cgtcacgcga aaactgcaac 660
   aataaggaag gggggggtag acgtagccga tgaatgtggg gtgccagtaa acgcagtctc 720
   tctctccccc cccccccccc ccccctcagg aatagtacaa cgggggaagg ataacggata 780
   gcaagtggaa tgcgaggaaa attttgaatg cgcaaggaaa gcaatatccg ggctatcagg 840
   ttttgagcca ggggacacac tcctcttctg cacaaaaact taacgtagac aaaaaaaaaa 900
   aactccacca agacacaatg aatcgcacat ggacatttag acctccccac atgtgaaagc 960
   ttctctggcg aaagcaaaaa aagtataata aggacccatg ccttccctct tcctgggccg 1020
   tttcaacttt ttctttttct ttgtctatca acacacacac acctcacgac catgactgca 1080
   caggatatta tcgccacata catcaccaaa tggtacgtga tagtaccact cgctttgatt 1140
   gcttataggg tcctcgacta cttttacggc agatacttga tgtacaagct tggtgctaaa 1200
   ccgtttttcc agaaacaaac agacggttat ttcggattca aagctccact tgaattgtta 1260
   aaaaagaaga gtgacggtac cctcatagac ttcactctcg agcgtatcca agcgctcaat 1320
   cgtccagata tcccaacttt tacattccca atcttttcca tcaaccttat cagcaccctt 1380
   gagccggaga acatcaaggc tatcttggcc acccagttca acgatttctc cttgggcacc 1440
   agacactcgc actttgctcc tttgttgggc gatggtatct ttaccttgga cggtgccggc 1500
   tggaagcaca gcagatctat gttgagacca cagtttgcca gagaacagat ttcccacgtc 1560
   aagttgttgg agccacacat gcaggtgttc ttcaagcacg tcagaaaggc acagggcaag 1620
   acttttgaca tccaagaatt gtttttcaga ttgaccgtcg actccgccac tgagtttttg 1680
   tttggtgaat ccgttgagtc cttgagagat gaatctattg ggatgtccat caatgcactt 1740
   gactttgacg gcaaggctgg ctttgctgat gcttttaact actcgcagaa ctatttggct 1800
   tcgagagcgg ttatgcaaca attgtactgg gtgttgaacg ggaaaaagtt taaggagtgc 1860
   aacgctaaag tgcacaagtt tgctgactat tacgtcagca aggctttgga cttgacacct 1920
   gaacaattgg aaaagcagga tggttatgtg ttcttgtacg agttggtcaa gcaaaccaga 1980
   gacaggcaag tgttgagaga ccagttgttg aacatcatgg ttgccggtag agacaccacc 2040
   gccggtttgt tgtcgtttgt tttctttgaa ttggccagaa acccagaggt gaccaacaag 2100
   ttgagagaag aaatcgagga caagtttggt cttggtgaga atgctcgtgt tgaagacatt 2160
   tcctttgagt cgttgaagtc atgtgaatac ttgaaggctg ttctcaacga aactttgaga 2220
   ttgtacccat ccgtgccaca gaatttcaga gttgccacca aaaacactac ccttccaagg 2280
   ggaggtggta aggacgggtt atctcctgtt ttggtcagaa agggtcaaac cgttatgtac 2340
   ggtgtctacg ctgcccacag aaacccagct gtctacggta aggacgccct tgagtttaga 2400
   ccagagaggt ggtttgagcc agagacaaag aagcttggct gggccttcct tccattcaac 2460
   ggtggtccaa gaatttgctt gggacagcag tttgccttga cagaagcttc gtatgtcact 2520
   gtcagattgc tccaagagtt tggacacttg tctatggacc ccaacaccga atatccacct 2580
   aggaaaatgt cgcatttgac catgtccctt ttcgacggtg ccaacattga gatgtattag 2640
   aggatcatgt gttatttttg attggtttag tctgtttgta gctattgatt aggttaattc 2700
   acggattgtt atttattgat agggggtgcg tgtgtgtgtg tgtgttgcat tcacatggga 2760
   tcgttccagg ttgttgtttc cttccatcct gttgagtcaa aaggagtttt gttttgtaac 2820
   tccggacgat gtcttagata gaaggtcgat ctccatgtga ttgtttgact gctactctga 2880
   ttatgtaatc tgtaaagcct agacgttatg caagcatgtg attgtggttt ttgcaacctg 2940
   tttgcacgac aaatgatcga cagtcgatta cgtaatccat attatttaga ggggtaataa 3000
   aaaataaatg gcagccagaa tttcaaacat tttgcaaaca atgcaaaaga tgagaaactc 3060
   caacagaaaa aataaaaaaa ctccgcagca ctccgaacca acaaaacaat ggggggcgcc 3120
   agaattattg actattgtga ctttttttta ttttttccgt taactttcat tgcagtgaag 3180
   tgtgttacac ggggtggtga tggtgttggt ttctacaatg caagggcaca gttgaaggtt 3240
   tccacataac gttgcaccat atcaactcaa tttatcctca ttcatgtgat aaaagaagag 3300
   ccaaaaggta attggcagac cccccaaggg gaacacggag tagaaagcaa tggaaacacg 3360
   cccatgacag tgccatttag cccacaacac atctagtatt cttttttttt tttgtgcgca 3420
   ggtgcacacc tggactttag ttattgcccc ataaagttaa caatctcacc tttggctctc 3480
   ccagtgtctc cgcctccaga tgctcgtttt acaccctcga gctaacgaca acacaacacc 3540
   catgagggga atgggcaaag ttaaacactt ttggtttcaa tgattcctat ttgctactct 3600
   cttgttttgt gttttgattt gcaccatgtg aaataaacga caattatata taccttttcg 3660
   tctgtcctcc aatgtctctt tttgctgcca ttttgctttt tgctttttgc ttttgcactc 3720
   tctcccactc ccacaatcag tgcagcaaca cacaa 3755
<210> 88
   <211> 3900
   <212> DNA
   <213> Candida tropicalis
<400> 88
   gacatcataa tgacccggtt atttcgccct caggttgctt atttgagccg taaagtgcag 60
   tagaaacttt gccttgggtt caaactctag tataatggtg ataactggtt gcactcttgc 120
   cataggcatg aaaataggcc gttatagtac tatatttaat aagcgtagga gtataggatg 180
   catatgaccg gtttttctat atttttaaga taatctctag taaattttgt attctcagta 240
   ggatttcatc aaatttcgca accaattctg gcgaaaaaat gattctttta cgtcaaaagc 300
   tgaatagtgc agtttaaagc acctaaaatc acatatacag cctctagata cgacagagaa 360
   gctctttatg atctgaagaa gcattagaat agctactatg agccactatt ggtgtatata 420
   ttagggattg gtgcaattaa gtacgtacta ataaacagaa gaaaatactt aaccaatttc 480
   tggtgtatac ttagtggtga gggacctttt ctgaacattc gggtcaaact tttttttgga 540
   gtgcgacatc gatttttcgt ttgtgtaata atagtgaacc tttgtgtaat aaatcttcat 600
   gcaagacttg cataattcga gcttgggagt tcacgccaat ttgacctcgt tcatgtgata 660
   aaagaaaagc caaaaggtaa ttagcagacg caatgggaac atggagtgga aagcaatgga 720
   agcacgccca ggacggagta atttagtcca cactacatct gggggttttt tttttgtgcg 780
   caagtacaca cctggacttt agtttttgcc ccataaagtt aacaatctaa cctttggctc 840
   tccaactctc tccgccccca aatattcgtt tttacaccct caagctagcg acagcacaac 900
   acccattaga ggaatggggc aaagttaaac acttttggct tcaatgattc ctattcgcta 960
   ctacattctt ctcttgtttt gtgctttgaa ttgcaccatg tgaaataaac gacaattata 1020
   tatacctttt catccctcct cctatatctc tttttgctac attttgtttt ttacgtttct 1080
   tgcttttgca ctctcccact cccacaaaga aaaaaaaact acactatgtc gtcttctcca 1140
   tcgtttgccc aagaggttct cgctaccact agtccttaca tcgagtactt tcttgacaac 1200
   tacaccagat ggtactactt catacctttg gtgcttcttt cgttgaactt tataagtttg 1260
   ctccacacaa ggtacttgga acgcaggttc cacgccaagc cactcggtaa ctttgtcagg 1320
   gaccctacgt ttggtatcgc tactccgttg cttttgatct acttgaagtc gaaaggtacg 1380
   gtcatgaagt ttgcttgggg cctctggaac aacaagtaca tcgtcagaga cccaaagtac 1440
   aagacaactg ggctcaggat tgttggcctc ccattgattg aaaccatgga cccagagaac 1500
   atcaaggctg ttttggctac tcagttcaat gatttctctt tgggaaccag acacgatttc 1560
   ttgtactcct tgttgggtga cggtattttc accttggacg gtgctggctg gaaacatagt 1620
   agaactatgt tgagaccaca gtttgctaga gaacaggttt ctcacgtcaa gttgttggag 1680
   ccacacgttc aggtgttctt caagcacgtt agaaagcacc gcggtcaaac gttcgacatc 1740
   caagaattgt tcttcaggtt gaccgtcgac tccgccaccg agttcttgtt tggtgagtct 1800
   gctgaatcct tgagggacga atctattgga ttgaccccaa ccaccaagga tttcgatggc 1860
   agaagagatt tcgctgacgc tttcaactat tcgcagactt accaggccta cagatttttg 1920
   ttgcaacaaa tgtactggat cttgaatggc tcggaattca gaaagtcgat tgctgtcgtg 1980
   cacaagtttg ctgaccacta tgtgcaaaag gctttggagt tgaccgacga tgacttgcag 2040
   aaacaagacg gctatgtgtt cttgtacgag ttggctaagc aaaccagaga cccaaaggtc 2100
   ttgagagacc agttattgaa cattttggtt gccggtagag acacgaccgc cggtttgttg 2160
   tcatttgttt tctacgagtt gtcaagaaac cctgaggtgt ttgctaagtt gagagaggag 2220
   gtggaaaaca gatttggact cggtgaagaa gctcgtgttg aagagatctc gtttgagtcc 2280
   ttgaagtctt gtgagtactt gaaggctgtc atcaatgaaa ccttgagatt gtacccatcg 2340
   gttccacaca actttagagt tgctaccaga aacactaccc tcccaagagg tggtggtgaa 2400
   gatggatact cgccaattgt cgtcaagaag ggtcaagttg tcatgtacac tgttattgct 2460
   acccacagag acccaagtat ctacggtgcc gacgctgacg tcttcagacc agaaagatgg 2520
   tttgaaccag aaactagaaa gttgggctgg gcatacgttc cattcaatgg tggtccaaga 2580
   atctgtttgg gtcaacagtt tgccttgacc gaagcttcat acgtcactgt cagattgctc 2640
   caggagtttg cacacttgtc tatggaccca gacaccgaat atccaccaaa attgcagaac 2700
   accttgacct tgtcgctctt tgatggtgct gatgttagaa tgtactaagg ttgcttttcc 2760
   ttgctaattt tcttctgtat agcttgtgta tttaaattga atcggcaatt gatttttctg 2820
   ataccaataa ccgtagtgcg atttgaccaa aaccgttcaa actttttgtt ctctcgttga 2880
   cgtgctcgct catcagcact gtttgaagac gaaagagaaa attttttgta aacaacactg 2940
   tccaaattta cccaacgtga accattatgc aaatgagcgg ccctttcaac tggtcgctgg 3000
   aagcattcgg ggatatctac aacgccctta agtttgaaac agacattgat ttagacacca 3060
   tagatttcag cggcatcaag aatgaccttg cccacatttt gacgacccca acaccactgg 3120
   aagaatcacg ccagaaacta ggcgatggat ccaagcctgt gaccttgccc aatggagacg 3180
   aagtggagtt gaaccaagcg ttcctagaag ttaccacatt attgtcgaat gagtttgact 3240
   tggaccaatt gaacgcggca gagttgttat actacgctgg cgacatatcc tacaagaagg 3300
   gcacatcaat cgcagacagt gccagattgt cttattattt gagagcaaac tacatcttga 3360
   acatacttgg gtatttgatt tcgaagcagc gattggattt gatagtcacg gacaacgacg 3420
   cgttgtttga tagtattttg aaaagttttg aaaagatcta caagttgata agcgtgttga 3480
   acgatatgat tgacaagcaa aaggtgacaa gcgacatcaa cagtctagca ttcatcaatt 3540
   gcatcaacta ctcgagaggt caactattct ccgcacacga acttttggga ctggttttgt 3600
   ttggattggt cgacatctat ttcaaccagt ttggcacatt agacaactac aagaaggtat 3660
   tggcattgat actgaagaac atcagcgatg aagacatctt gatcatacac ttcctcccat 3720
   cgacactaca attgtttaag ctggtgttgg acaagaaaga cgacgctgca gttgaacagt 3780
   tctacaagta catcacttca acagtgtcac gagactacaa ctccaacatc ggctccacag 3840
   ccaaagatga tatcgatttg tccaaaacca aactcagtgg ctttgaggtg ttgacgagtt 3900
<210> 89
   <211> 3668
   <212> DNA
   <213> Candida tropicalis
<400> 89
   cctgcagaat tcgcggccgc gtcgacagag tagcagttat gcaagcatgt gattgtggtt 60
   tttgcaacct gtttgcacga caaatgatcg acagtcgatt acgtaatcca tattatttag 120
   aggggtaata aaaaataaat ggcagccaga atttcaaaca ttttgcaaac aatgcaaaag 180
   atgagaaact ccaacagaaa aaataaaaaa actccgcagc actccgaacc aacaaaacaa 240
   tggggggcgc cagaattatt gactattgtg actttttttt attttttccg ttaactttca 300
   ttgcagtgaa gtgtgttaca cggggtggtg atggtgttgg tttctacaat gcaagggcac 360
   agttgaaggt ttccacataa cgttgcacca tatcaactca atttatcctc attcatgtga 420
   taaaagaaga gccaaaaggt aattggcaga ccccccaagg ggaacacgga gtagaaagca 480
   atggaaacac gcccatgaca gtgccattta gcccacaaca catctagtat tctttttttt 540
   ttttgtgcgc aggtgcacac ctggacttta gttattgccc cataaagtta acaatctcac 600
   ctttggctct cccagtgtct ccgcctccag atgctcgttt tacaccctcg agctaacgac 660
   aacacaacac ccatgagggg aatgggcaaa gttaaacact tttggtttca atgattccta 720
   tttgctactc tcttgttttg tgttttgatt tgcaccatgt gaaataaacg acaattatat 780
   ataccttttc gtctgtcctc caatgtctct ttttgctgcc attttgcttt ttgctttttg 840
   cttttgcact ctctcccact cccacaatca gtgcagcaac acacaaagaa gaaaaataaa 900
   aaaacctaca ctatgtcgtc ttctccatcg tttgctcagg aggttctcgc taccactagt 960
   ccttacatcg agtactttct tgacaactac accagatggt actacttcat ccctttggtg 1020
   cttctttcgt tgaacttcat cagcttgctc cacacaaagt acttggaacg caggttccac 1080
   gccaagccgc tcggtaacgt cgtgttggat cctacgtttg gtatcgctac tccgttgatc 1140
   ttgatctact taaagtcgaa aggtacagtc atgaagtttg cctggagctt ctggaacaac 1200
   aagtacattg tcaaagaccc aaagtacaag accactggcc ttagaattgt cggcctccca 1260
   ttgattgaaa ccatagaccc agagaacatc aaagctgtgt tggctactca gttcaacgat 1320
   ttctccttgg gaactagaca cgatttcttg tactccttgt tgggcgatgg tatttttacc 1380
   ttggacggtg ctggctggaa acacagtaga actatgttga gaccacagtt tgctagagaa 1440
   caggtttccc acgtcaagtt gttggaacca cacgttcagg tgttcttcaa gcacgttaga 1500
   aaacaccgcg gtcagacttt tgacatccaa gaattgttct tcagattgac cgtcgactcc 1560
   gccaccgagt tcttgtttgg tgagtctgct gaatccttga gagacgactc tgttggtttg 1620
   accccaacca ccaaggattt cgaaggcaga ggagatttcg ctgacgcttt caactactcg 1680
   cagacttacc aggcctacag atttttgttg caacaaatgt actggatttt gaatggcgcg 1740
   gaattcagaa agtcgattgc catcgtgcac aagtttgctg accactatgt gcaaaaggct 1800
   ttggagttga ccgacgatga cttgcagaaa caagacggct atgtgttctt gtacgagttg 1860
   gctaagcaaa ctagagaccc aaaggtcttg agagaccagt tgttgaacat tttggttgcc 1920
   ggtagagaca cgaccgccgg tttgttgtcg tttgtgttct acgagttgtc gagaaaccct 1980
   gaagtgtttg ccaagttgag agaggaggtg gaaaacagat ttggactcgg cgaagaggct 2040
   cgtgttgaag agatctcttt tgagtccttg aagtcctgtg agtacttgaa ggctgtcatc 2100
   aatgaagcct tgagattgta cccatctgtt ccacacaact tcagagttgc caccagaaac 2160
   actacccttc caagaggcgg tggtaaagac ggatgctcgc caattgttgt caagaagggt 2220
   caagttgtca tgtacactgt cattggtacc cacagagacc caagtatcta cggtgccgac 2280
   gccgacgtct tcagaccaga aagatggttc gagccagaaa ctagaaagtt gggctgggca 2340
   tatgttccat tcaatggtgg tccaagaatc tgtttgggtc agcagtttgc cttgactgaa 2400
   gcttcatacg tcactgtcag attgctccaa gagtttggaa acttgtccct ggatccaaac 2460
   gctgagtacc caccaaaatt gcagaacacc ttgaccttgt cactctttga tggtgctgac 2520
   gttagaatgt tctaaggttg cttatccttg ctagtgttat ttatagtttg tgtatttaaa 2580
   ttgaatcggc gattgatttt tctggtacta ataactgtag tgggttttga ccaaaaccgt 2640
   tcaaactttt tttttttttt tcttccccct accttcgttg ctcgctcatc agcactgttt 2700
   gaaaacgaaa aaagaaaatt ttttgtaaac aacattgccc aaacttaccc aacgtgaacc 2760
   attataacca aatgagcggc gctttcaact ggtcactgga ggcattcggg gatatctaca 2820
   acacccttaa gtttgaggaa gacattgatt tagacaccat agatttcagc ggcatcaaga 2880
   atgaccttgt ccacattttg acaaccccaa caccactgga agaatcgcgc cagaaactag 2940
   gcgatggatc caagcctgtg gccttgccca atggagacga agtggagttg aaccaagcgt 3000
   tcctagaagt taccacatta ttgtcgaacg agtttgactt ggaccaattg aacgcggccg 3060
   agttgttata ctacgccggc gacatatcct acaagaaggg cacatcaatt gccgacagtg 3120
   ccagattgtc ttactatttg agagcaaact acatcttgaa catacttggg tactttattt 3180
   cgaagcagcg attggatgtg atagtcaccg acaacaacgc gttgtttgat aatattttga 3240
   aaagttttga aaagatctac aagttgataa gcgcgttgaa cgatatgatt gacaagcaaa 3300
   aggtgacaag cgacatcaac agtctagcat ttatcaactg catcaactac tcgaggggtc 3360
   aactattctc cgcacacgaa cttttgggac tggttttgtt tggattggtt gacaactatt 3420
   tcaaccagtt tggctcatta gacaactaca agaaagtatt ggcattgata ctgaagaaca 3480
   tcagtgatga agatatcttg atcgtacgct tcctcccatc gacactacaa ttgtttaagc 3540
   tggtgttgga taagaaagac gacgccactg ttgaccagtt ctacaagtac atcacctcaa 3600
   cagtgtcgca agactacaac tccaacatcg gagccacagc caaagatgat atcgatttgt 3660
   ccaaagcc                                                          3668
<210> 90
   <211> 3826
   <212> DNA
   <213> Candida tropicalis
<400> 90
   tggagtcgcc agacttgctc acttttgact cccttcgaaa ctcaaagtac gttcaggcgg 60
   tgctcaacga aacgctccgt atctacccgg gggtaccacg aaacatgaag acagctacgt 120
   gcaacacgac gttgccacgc ggaggaggca aagacggcaa ggaacctatc ttggtgcaga 180
   agggacagtc cgttgggttg attactattg ccacgcagac ggacccagag tattttgggg 240
   ccgacgctgg tgagtttaag ccggagagat ggtttgattc aagcatgaag aacttggggt 300
   gtaaatactt gccgttcaat gctgggccac ggacttgctt ggggcagcag tacactttga 360
   ttgaagcgag ctacttgcta gtccggttgg cccagaccta ccgggcaata gatttgcagc 420
   caggatcggc gtacccacca agaaagaagt cgttgatcaa catgagtgct gccgacgggg 480
   tgtttgtaaa gctttataag gatgtaacgg tagatggata gttgtgtagg aggagcggag 540
   ataaattaga tttgattttg tgtaaggttt tggatgtcaa cctactccgc acttcatgca 600
   gtgtgtgtga cacaagggtg tactacgtgt gcgtgtgcgc caagagacag cccaaggggg 660
   tggtagtgtg tgttggcgga agtgcatgtg acacaacgcg tgggttctgg ccaatggtgg 720
   actaagtgca ggtaagcagc gacctgaaac attcctcaac gcttaagaca ctggtggtag 780
   agatgcggac caggctattc ttgtcgtgct acccggcgca tggaaaatca actgcgggaa 840
   gaataaattt atccgtagaa tccacagagc ggataaattt gcccacctcc atcatcaacc 900
   acgccgccac taactacatc actcccctat tttctctctc tctctttgtc ttactccgct 960
   cccgtttcct tagccacaga tacacaccca ctgcaaacag cagcaacaat tataaagata 1020
   cgccaggccc accttctttc tttttcttca cttttttgac tgcaactttc tacaatccac 1080
   cacagccacc accacagccg ctatgattga acaactccta gaatattggt atgtcgttgt 1140
   gccagtgttg tacatcatca aacaactcct tgcatacaca aagactcgcg tcttgatgaa 1200
   aaagttgggt gctgctccag tcacaaacaa gttgtacgac aacgctttcg gtatcgtcaa 1260
   tggatggaag gctctccagt tcaagaaaga gggcagggct caagagtaca acgattacaa 1320
   gtttgaccac tccaagaacc caagcgtggg cacctacgtc agtattcttt tcggcaccag 1380
   gatcgtcgtg accaaagatc cagagaatat caaagctatt ttggcaaccc agtttggtga 1440
   tttttctttg ggcaagaggc acactctttt taagcctttg ttaggtgatg ggatcttcac 1500
   attggacggc gaaggctgga agcacagcag agccatgttg agaccacagt ttgccagaga 1560
   acaagttgct catgtgacgt cgttggaacc acacttccag ttgttgaaga agcatattct 1620
   taagcacaag ggtgaatact ttgatatcca ggaattgttc tttagattta ccgttgattc 1680
   ggccacggag ttcttatttg gtgagtccgt gcactcctta aaggacgaat ctattggtat 1740
   caaccaagac gatatagatt ttgctggtag aaaggacttt gctgagtcgt tcaacaaagc 1800
   ccaggaatac ttggctatta gaaccttggt gcagacgttc tactggttgg tcaacaacaa 1860
   ggagtttaga gactgtacca agctggtgca caagttcacc aactactatg ttcagaaagc 1920
   tttggatgct agcccagaag agcttgaaaa gcaaagtggg tatgtgttct tgtacgagct 1980
   tgtcaagcag acaagagacc ccaatgtgtt gcgtgaccag tctttgaaca tcttgttggc 2040
   cggaagagac accactgctg ggttgttgtc gtttgctgtc tttgagttgg ccagacaccc 2100
   agagatctgg gccaagttga gagaggaaat tgaacaacag tttggtcttg gagaagactc 2160
   tcgtgttgaa gagattacct ttgagagctt gaagagatgt gagtacttga aagcgttcct 2220
   taatgaaacc ttgcgtattt acccaagtgt cccaagaaac ttcagaatcg ccaccaagaa 2280
   cacgacattg ccaaggggcg gtggttcaga cggtacctcg ccaatcttga tccaaaaggg 2340
   agaagctgtg tcgtatggta tcaactctac tcatttggac cctgtctatt acggccctga 2400
   tgctgctgag ttcagaccag agagatggtt tgagccatca accaaaaagc tcggctgggc 2460
   ttacttgcca ttcaacggtg gtccaagaat ctgtttgggt cagcagtttg ccttgacgga 2520
   agctggctat gtgttggtta gattggtgca agagttctcc cacgttaggc tggacccaga 2580
   cgaggtgtac ccgccaaaga ggttgaccaa cttgaccatg tgtttgcagg atggtgctat 2640
   tgtcaagttt gactagcggc gtggtgaatg cgtttgattt tgtagtttct gtttgcagta 2700
   atgagataac tattcagata aggcgagtgg atgtacgttt tgtaagagtt tccttacaac 2760
   cttggtgggg tgtgtgaggt tgaggttgca tcttggggag attacacctt ttgcagctct 2820
   ccgtatacac ttgtactctt tgtaacctct atcaatcatg tggggggggg ggttcattgt 2880
   ttggccatgg tggtgcatgt taaatccgcc aactacccaa tctcacatga aactcaagca 2940
   cactaaaaaa aaaaaagatg ttgggggaaa actttggttt cccttcttag taattaaaca 3000
   ctctcactct cactctcact ctctccactc agacaaacca accacctggg ctgcagacaa 3060
   ccagaaaaaa aaagaacaaa atccagatag aaaaacaaag ggctggacaa ccataaataa 3120
   acaatctagg gtctactcca tcttccactg tttcttcttc ttcagactta gctaacaaac 3180
   aactcacttc accatggatt acgcaggcat cacgcgtggc tccatcagag gcgaggcctt 3240
   gaagaaactc gcagaattga ccatccagaa ccagccatcc agcttgaaag aaatcaacac 3300
   cggcatccag aaggacgact ttgccaagtt gttgtctgcc accccgaaaa tccccaccaa 3360
   gcacaagttg aacggcaacc acgaattgtc tgaggtcgcc attgccaaaa aggagtacga 3420
   ggtgttgatt gccttgagcg acgccacaaa agacccaatc aaagtgacct cccagatcaa 3480
   gatcttgatt gacaagttca aggtgtactt gtttgagttg cctgaccaga agttctccta 3540
   ctccatcgtg tccaactccg tcaacatcgc cccctggacc ttgctcgggg agaagttgac 3600
   cacgggcttg atcaacttgg ccttccagaa caacaagcag cacttggacg aggtcattga 3660
   catcttcaac gagttcatcg acaagttctt tggcaacacg gagccgcaat tgaccaactt 3720
   cttgaccttg tgcggtgtgt tggacgggtt gattgaccat gccaacttct tgagcgtgtc 3780
   ctcgcggacc ttcaagatct tcttgaactt ggactcgtat gtggac 3826
<210> 91
   <211> 3910
   <212> DNA
   <213> Candida tropicalis
<400> 91
   ttacaatcat ggagctcgct aggaacccag atgtctggga gaagctccgc gaagaggtca 60
   acacgaactt tggcatggag tcgccagact tgctcacttt tgactctctt agaagctcaa 120
   agtacgttca ggcggtgctc aacgaaacgc ttcgtatcta cccgggggtg ccacgaaaca 180
   tgaagacagc tacgtgcaac acgacgttgc cgcgtggagg aggcaaagac ggtaaggaac 240
   ctattttggt gcagaagggc cagtccgttg ggttgattac tattgccacg cagacggacc 300
   cagagtattt tggggcagat gctggtgagt tcaaaccgga gagatggttt gattcaagca 360
   tgaagaactt ggggtgtaag tacttgccgt tcaatgctgg gccccggact tgtttggggc 420
   agcagtacac tttgattgaa gcgagctatt tgctagtcag gttggcgcag acctaccggg 480
   taatcgattt gctgccaggg tcggcgtacc caccaagaaa gaagtcgttg atcaatatga 540
   gtgctgccga tggggtggtt gtaaagtttc acaaggatct agatggatat gtaaggtgtg 600
   taggaggagc ggagataaat tagatttgat tttgtgtaag gtttagcacg tcaagctact 660
   ccgcactttg tgtgtaggga gcacatactc cgtctgcgcc tgtgccaaga gacggcccag 720
   gggtagtgtg tggtggtgga agtgcatgtg acacaatacc ctggttctgg ccaattgggg 780
   atttagtgta ggtaagctgc gacctgaaac actcctcaac gcttgagaca ctggtgggta 840
   gagatgcggg ccaggaggct attcttgtcg tgctacccgt gcacggaaaa tcgattgagg 900
   gaagaacaaa tttatccgtg aaatccacag agcggataaa tttgtcacat tgctgcgttg 960
   cccacccaca gcattctctt ttctctctct ttgtcttact ccgctcctgt ttccttatcc 1020
   agaaatacac accaactcat ataaagatac gctagcccag ctgtctttct ttttcttcac 1080
   tttttttggt gtgttgcttt tttggctgct actttctaca accaccacca ccaccaccac 1140
   catgattgaa caaatcctag aatattggta tattgttgtg cctgtgttgt acatcatcaa 1200
   acaactcatt gcctacagca agactcgcgt cttgatgaaa cagttgggtg ctgctccaat 1260
   cacaaaccag ttgtacgaca acgttttcgg tatcgtcaac ggatggaagg ctctccagtt 1320
   caagaaagag ggcagagctc aagagtacaa cgatcacaag tttgacagct ccaagaaccc 1380
   aagcgtcggc acctatgtca gtattctttt tggcaccaag attgtcgtga ccaaggatcc 1440
   agagaatatc aaagctattt tggcaaccca gtttggcgat ttttctttgg gcaagagaca 1500
   cgctcttttt aaacctttgt taggtgatgg gatcttcacc ttggacggcg aaggctggaa 1560
   gcatagcaga tccatgttaa gaccacagtt tgccagagaa caagttgctc atgtgacgtc 1620
   gttggaacca cacttccagt tgttgaagaa gcatatcctt aaacacaagg gtgagtactt 1680
   tgatatccag gaattgttct ttagatttac tgtcgactcg gccacggagt tcttatttgg 1740
   tgagtccgtg cactccttaa aggacgaaac tatcggtatc aaccaagacg atatagattt 1800
   tgctggtaga aaggactttg ctgagtcgtt caacaaagcc caggagtatt tgtctattag 1860
   aattttggtg cagaccttct actggttgat caacaacaag gagtttagag actgtaccaa 1920
   gctggtgcac aagtttacca actactatgt tcagaaagct ttggatgcta ccccagagga 1980
   acttgaaaag caaggcgggt atgtgttctt gtatgagctt gtcaagcaga cgagagaccc 2040
   caaggtgttg cgtgaccagt ctttgaacat cttgttggca ggaagagaca ccactgctgg 2100
   gttgttgtcc tttgctgtgt ttgagttggc cagaaaccca cacatctggg ccaagttgag 2160
   agaggaaatt gaacagcagt ttggtcttgg agaagactct cgtgttgaag agattacctt 2220
   tgagagcttg aagagatgtg agtacttgaa agcgttcctt aacgaaacct tgcgtgttta 2280
   cccaagtgtc ccaagaaact tcagaatcgc caccaagaat acaacattgc caaggggtgg 2340
   tggtccagac ggtacccagc caatcttgat ccaaaaggga gaaggtgtgt cgtatggtat 2400
   caactctacc cacttagatc ctgtctatta tggccctgat gctgctgagt tcagaccaga 2460
   gagatggttt gagccatcaa ccagaaagct cggctgggct tacttgccat tcaacggtgg 2520
   gccacgaatc tgtttgggtc agcagtttgc cttgaccgaa gctggttacg ttttggtcag 2580
   attggtgcaa gagttctccc acattaggct ggacccagat gaagtgtatc caccaaagag 2640
   gttgaccaac ttgaccatgt gtttgcagga tggtgctatt gtcaagtttg actagtacgt 2700
   atgagtgcgt ttgattttgt agtttctgtt tgcagtaatg agataactat tcagataagg 2760
   cgggtggatg tacgttttgt aagagtttcc ttacaaccct ggtgggtgtg tgaggttgca 2820
   tcttagggag agatagcacc ttttgcagct ctccgtatac agttttactc tttgtaacct 2880
   atgccaatca tgtggggatt cattgtttgc ccatggtggt gcatgcaaaa tccccccaac 2940
   tacccaatct cacatgaaac tcaagcacac tagaaaaaaa agatgttgcg tgggttcttt 3000
   tgatgttggg gaaaactttc gtttcctttc tcagtaatta aacgttctca ctcagacaaa 3060
   ccacctgggc tgcagacaac cagaaaaaac aaaatccaga tagaagaaga aagggctgga 3120
   caaccataaa taaacaacct agggtccact ccatctttca cttcttcttc ttcagactta 3180
   tctaacaaac gactcacttc accatggatt acgcaggtat cacgcgtggg tccatcagag 3240
   gcgaagcctt gaagaaactc gccgagttga ccatccagaa ccagccatcc agcttgaaag 3300
   aaatcaacac cggcatccag aaggacgact ttgccaagtt gttgtcttcc accccgaaaa 3360
   tccacaccaa gcacaagttg aatggcaacc acgaattgtc cgaagtcgcc attgccaaaa 3420
   aggagtacga ggtgttgatt gccttgagcg acgccacgaa agaaccaatc aaagtcacct 3480
   cccagatcaa gatcttgatt gacaagttca aggtgtactt gtttgagttg cccgaccaga 3540
   agttctccta ctccatcgtg tccaactccg ttaacattgc cccctggacc ttgctcggtg 3600
   agaagttgac cacgggcttg atcaacttgg cgttccagaa caacaagcag cacttggacg 3660
   aagtcatcga catcttcaac gagttcatcg acaagttctt tggcaacaca gagccgcaat 3720
   tgaccaactt cttgaccttg tccggtgtgt tggacgggtt gattgaccat gccaacttct 3780
   tgagcgtgtc ctccaggacc ttcaagatct tcttgaactt ggactcgttt gtggacaact 3840
   cggacttctt gaacgacgtg gagaactact ccgacttttt gtacgacgag ccgaacgagt 3900
   accagaactt 3910
<210> 92
   <211> 3150
   <212> DNA
   <213> Candida tropicalis
<400> 92
   gaattctttg gatctaattc cagctgatct tgctaatcct tatcaacgta gttgtgatca 60
   ttgtttgtct gaattataca caccagtgga agaatatggt ctaatttgca cgtcccactg 120
   gcattgtgtg tttgtggggg ggggggggtg cacacatttt tagtgccatt ctttgttgat 180
   tacccctccc ccctatcatt cattcccaca ggattagttt tttcctcact ggaattcgct 240
   gtccacctgt caaccccccc cccccccccc cccactgccc taccctgccc tgccctgcac 300
   gtcctgtgtt ttgtgctgtg tctttcccac gctataaaag ccctggcgtc cggccaaggt 360
   ttttccaccc agccaaaaaa acagtctaaa aaatttggtt gatccttttt ggttgcaagg 420
   ttttccacca ccacttccac cacctcaact attcgaacaa aagatgctcg atcagatctt 480
   acattactgg tacattgtct tgccattgtt ggccattatc aaccagatcg tggctcatgt 540
   caggaccaat tatttgatga agaaattggg tgctaagcca ttcacacacg tccaacgtga 600
   cgggtggttg ggcttcaaat tcggccgtga attcctcaaa gcaaaaagtg ctgggagact 660
   ggttgattta atcatctccc gtttccacga taatgaggac actttctcca gctatgcttt 720
   tggcaaccat gtggtgttca ccagggaccc cgagaatatc aaggcgcttt tggcaaccca 780
   gtttggtgat ttttcattgg gcagcagggt caagttcttc aaaccattat tggggtacgg 840
   tatcttcaca ttggacgccg aaggctggaa gcacagcaga gccatgttga gaccacagtt 900
   tgccagagaa caagttgctc atgtgacgtc gttggaacca cacttccagt tgttgaagaa 960
   gcatatcctt aaacacaagg gtgagtactt tgatatccag gaattgttct ttagatttac 1020
   tgtcgactcg gccacggagt tcttatttgg tgagtccgtg cactccttaa aggacgagga 1080
   aattggctac gacacgaaag acatgtctga agaaagacgc agatttgccg acgcgttcaa 1140
   caagtcgcaa gtctacgtgg ccaccagagt tgctttacag aacttgtact ggttggtcaa 1200
   caacaaagag ttcaaggagt gcaatgacat tgtccacaag tttaccaact actatgttca 1260
   gaaagccttg gatgctaccc cagaggaact tgaaaagcaa ggcgggtatg tgttcttgta 1320
   tgagcttgtc aagcagacga gagaccccaa ggtgttgcgt gaccagtctt tgaacatctt 1380
   gttggcagga agagacacca ctgctgggtt gttgtccttt gctgtgtttg agttggccag 1440
   aaacccacac atctgggcca agttgagaga ggaaattgaa cagcagtttg gtcttggaga 1500
   agactctcgt gttgaagaga ttacctttga gagcttgaag agatgtgagt acttgaaggc 1560
   cgtgttgaac gaaactttga gattacaccc aagtgtccca agaaacgcaa gatttgcgat 1620
   taaagacacg actttaccaa gaggcggtgg ccccaacggc aaggatccta tcttgatcag 1680
   gaaggatgag gtggtgcagt actccatctc ggcaactcag acaaatcctg cttattatgg 1740
   cgccgatgct gctgatttta gaccggaaag atggtttgaa ccatcaacta gaaacttggg 1800
   atgggctttc ttgccattca acggtggtcc aagaatctgt ttgggacaac agtttgcttt 1860
   gactgaagcc ggttacgttt tggttagact tgttcaggag tttccaaact tgtcacaaga 1920
   ccccgaaacc aagtacccac cacctagatt ggcacacttg acgatgtgct tgtttgacgg 1980
   tgcacacgtc aagatgtcat aggtttcccc atacaagtag ttcagtaatt atacactgtt 2040
   tttactttct cttcatacca aatggacaaa agttttaagc atgcctaaca acgtgaccgg 2100
   acaattgtgt cgcactagta tgtaacaatt gtaaaaatag tgtacactaa tttgtggtgg 2160
   ccggagataa attacagttt ggttttgtgt aaactcgcgg atatctctgg cagtttctct 2220
   tctccgcagc agctttgcca cgggtttgct ctggggccaa caaattcaaa agggggagaa 2280
   acttaacacc ccttatctct ccactctagg ttgtagctct tgtggggatg caattgtcgt 2340
   acgtttttta tgttttgtct agactttgat gattacgttg gatttcttat gtctgaggcg 2400
   tgcttgaaag aagtgtcaaa atgtgacagg cgacgctatt cgacatgaac gcgaaagggt 2460
   tatttgcatc aatacgaggg gctgactcta gtctaggatg gcagtcctag gttgcaaaca 2520
   tgttgcacca tatccctcct ggagttggtc gacctcgcct acgccaccct cagcgatcgg 2580
   cactttccgt tgttcaatat ttctccttcc cattgttcca ggggttatca acaacgttgc 2640
   cggcctcctc cccaaattac aagaaaaata aattgtcgca cggcaccgat ctgtcaaaga 2700
   tacagataaa ccttaaatct gcaaaaacaa gacccctccc catagcctag aagcaccagc 2760
   aagatgatgg agcaactcct ccagtactgg tacatcgcac tctctgtatg gttcatcctt 2820
   cgctacttgg cttcccacgc acgagccgtc tacttgcgcc acaagctcgg cgcggcgcca 2880
   ttcacgcaca cccagtacga cggctggtat gggttcaagt ttgggcggga gtttctcaag 2940
   gcgaagaaga tcgggcggca gacggacttg gtgcatgcgc ggttccgtgg cggcatggac 3000
   accttctcga gctacacttt cggcatccat atcatcctta cccgggaccc ggagaacatc 3060
   aaggcggtct tggcgacgca gttcgatgac ttctcgctcg gtggcaggat caggttcttg 3120
   aagccgttgt tggggtatgg gatattcacg 3150
<210> 93
   <211> 3579
   <212> DNA
   <213> Candida tropicalis
<400> 93
   aaaaccgata caagaagaag acagtcaaca agaacgttaa tgtcaaccag gcgccaagaa 60
   gacggtttgg cggacttgga agaatgtggc atttgcccat gatgtttatg ttctggagag 120
   gtttttcaag gaatcgtcat cctccgccac cacaagaacc accagttaac gagatccata 180
   ttcacaaccc accgcaaggt gacaatgctc aacaacaaca gcaacaacaa caacccccac 240
   aagaacagtg gaataatgcc agtcaacaaa gagtggtgac agacgaggga gaaaacgcaa 300
   gcaacagtgg ttctgatgca agatcagcta caccgcttca tcaggaaaag caggagctcc 360
   caccaccata tgcccatcac gagcaacacc agcaggttag tgtatagtag tctgtagtta 420
   agtcaatgca atgtaccaat aagactatcc cttcttacaa ccaagttttc tgccgcgcct 480
   gtctggcaac agatgctggc cgacacactt tcaactgagt ttggtctaga attcttgcac 540
   atgcacgaca aggaaactct tacaaagaca acacttgtgc tctgatgcca cttgatcttg 600
   ctaagcctta tcaacgtaat tgagatcatt gtttgtctga attatacaca ccagtggaag 660
   aatctggtct aatctgcacg cctcatgggc attgtgtgtt ttgggggggg gggggggggt 720
   gcacacattt ttagtgcgaa tgtttgtttg ctggttcccc ctcccccctc ccccctatca 780
   tgcccacagg attagttttt tcctcactgg aattcgctgt ccacctgtca accccctcac 840
   tgccctgccc tgccctgcac gccctgtgtt ttgtgctgtg gcactcccac gctataaaag 900
   ccctggcgta cggccaaggt ttttcctcac agccaaaaaa aaatttggct gatccttttg 960
   ggctgcaagg tttttcacca ccaccaccac caccacctca actattcaaa caaaggatgc 1020
   tcgaccagat cttccattac tggtacattg tcttgccatt gttggtcatt atcaagcaga 1080
   tcgtggctca tgccaggacc aattatttga tgaagaagtt gggcgctaag ccattcacac 1140
   atgtccaact agacgggtgg tttggcttca aatttggccg tgaattcctc aaagctaaaa 1200
   gtgctgggag gcaggttgat ttaatcatct cccgtttcca cgataatgag gacactttct 1260
   ccagctatgc ttttggcaac catgtggtgt tcaccaggga ccccgagaat atcaaggcgc 1320
   ttttggcaac ccagtttggt gatttttcat tgggaagcag ggtcaaattc ttcaaaccat 1380
   tgttggggta cggtatcttc accttggacg gcgaaggctg gaagcacagc agagccatgt 1440
   tgagaccaca gtttgccaga gagcaagttg ctcatgtgac gtcgttggaa ccacatttcc 1500
   agttgttgaa gaagcatatt cttaagcaca agggtgaata ctttgatatc caggaattgt 1560
   tctttagatt taccgttgat tcagcgacgg agttcttatt tggtgagtcc gtgcactcct 1620
   taagggacga ggaaattggc tacgatacga aggacatggc tgaagaaaga cgcaaatttg 1680
   ccgacgcgtt caacaagtcg caagtctatt tgtccaccag agttgcttta cagacattgt 1740
   actggttggt caacaacaaa gagttcaagg agtgcaacga cattgtccac aagttcacca 1800
   actactatgt tcagaaagcc ttggatgcta ccccagagga acttgaaaaa caaggcgggt 1860
   atgtgttctt gtacgagctt gccaagcaga cgaaagaccc caatgtgttg cgtgaccagt 1920
   ctttgaacat cttgttggct ggaagggaca ccactgctgg gttgttgtcc tttgctgtgt 1980
   ttgagttggc caggaaccca cacatctggg ccaagttgag agaggaaatt gaatcacact 2040
   ttgggctggg tgaggactct cgtgttgaag agattacctt tgagagcttg aagagatgtg 2100
   agtacttgaa agccgtgttg aacgaaacgt tgagattaca cccaagtgtc ccaagaaacg 2160
   caagatttgc gattaaagac acgactttac caagaggcgg tggccccaac ggcaaggatc 2220
   ctatcttgat cagaaagaat gaggtggtgc aatactccat ctcggcaact cagacaaatc 2280
   ctgcttatta tggcgccgat gctgctgatt ttagaccgga aagatggttt gagccatcaa 2340
   ctagaaactt gggatgggct tacttgccat tcaacggtgg tccaagaatc tgcttgggac 2400
   aacagtttgc tttgaccgaa gccggttacg ttttggttag acttgttcag gaattcccta 2460
   gcttgtcaca ggaccccgaa actgagtacc caccacctag attggcacac ttgacgatgt 2520
   gcttgtttga cggggcatac gtcaagatgc aataggtttt ggtttgactt tgtttccata 2580
   tgcaagtagt tcagtaatta cacactaatt tgtggtggcc ggcgataaat taccgtttgg 2640
   ttttgtgtaa aaattcggac atctctggtg gtttcccttc tccgcagcag ctttgccacg 2700
   ggtttgctct gcggccaaca aattcgaaag gggggggggg gggggagaaa gttaacaccc 2760
   cctgttccca ccgtaggctg tagctcttgt ggggggatgt aattgtcgta cgttttcatg 2820
   tttggcccag actttgatga ttacgtaggc tttcttatgt ctaaggcgtg cttgacacaa 2880
   gtgtcaaaag gtgacaggcg acgttattcg acatgaacgc aaaagggtaa tttgcatcga 2940
   tacgaggggt tgcctctggt ctaagaagga ccccccaggt tgcaaacatg ttgcactgca 3000
   tcccactcag agttggtcga ccacgcctac gcttaccctc agcgatcggc actttccgtt 3060
   gctcaatatt tctctccccc ctgcttcccc ccattgttcc agggattatc aacaacgttg 3120
   ccggtctcct ctcccccccc tccccccagt tatgtacaag aaaattaaat tgtcgcacgg 3180
   caccgatacg tcaaagatac agagaaacct taatccctcc catagcctag aagcatcaaa 3240
   aagatgattg agcaactcct ccagtactgg tacattgcac tccctgtatg gttcattctc 3300
   cgctacgtgg cttcccacgc acgaaccatc tacttgcgcc acaagctcgg cgcggcgccg 3360
   ttcacgcaca cccagtacga cggatggtat gggttcaagt ttgggcggga gtttctcaag 3420
   gcgaagaaga ttggaaggca gacggacttg gtgcatgcgc ggttccgtgg agggggcatg 3480
   gatactttct cgagctatac tttcggcatc catatcattc ttactcggga cccggagaac 3540
   atcaaggcgg tcttggcgac gcagttcgat gacttttcg 3579
<210> 94
   <211> 3348
   <212> DNA
   <213> Candida tropicalis
<400> 94
   gatgtggtgc ttgatttctc gagacacatc cttgtgaggt gccatgaatc tgtacctgtc 60
   tgtaagcaca gggaactgct tcaacacctt attgcatatt ctgtctattg caagcgtgtg 120
   ctgcaacgat atctgccaag gtatatagca gaacgtgctg atggttcctc cggtcatatt 180
   ctgttggtag ttctgcaggt aaatttggat gtcaggtagt ggagggaggt ttgtatcggt 240
   tgtgttttct tcttcctctc tctctgattc aacctccacg tctccttcgg gttctgtgtc 300
   tgtgtctgag tcgtactgtt ggattaagtc catcgcatgt gtgaaaaaaa gtagcgctta 360
   tttagacaac cagttcgttg ggcgggtatc agaaatagtc tgttgtgcac gaccatgagt 420
   atgcaacttg acgagacgtc gttaggaatc cacagaatga tagcaggaag cttactacgt 480
   gagagattct gcttagagga tgttctcttc ttgttgattc cattaggtgg gtatcatctc 540
   cggtggtgac aacttgacac aagcagttcc gagaaccacc cacaacaatc accattccag 600
   ctatcacttc tacatgtcaa cctacgatgt atctcatcac catctagttt cttggcaatc 660
   gtttatttgt tatgggtcaa catccaatac aactccacca atgaagaaga aaaacggaaa 720
   gcagaatacc agaatgacag tgtgagttcc tgaccattgc taatctatgg ctatatctag 780
   tttgctatcg tgggatgtga tctgtgtcgt cttcatttgc gtttgtgttt atttcgggta 840
   tgaatattgt tatactaaat acttgatgca caaacatggc gctcgagaaa tcgagaatgt 900
   gatcaacgat gggttctttg ggttccgctt acctttgcta ctcatgcgag ccagcaatga 960
   gggccgactt atcgagttca gtgtcaagag attcgagtcg gcgccacatc cacagaacaa 1020
   gacattggtc aaccgggcat tgagcgttcc tgtgatactc accaaggacc cagtgaatat 1080
   caaagcgatg ctatcgaccc agtttgatga cttttccctt gggttgagac tacaccagtt 1140
   tgcgccgttg ttggggaaag gcatctttac tttggacggc ccagagtgga agcagagccg 1200
   atctatgttg cgtccgcaat ttgccaaaga tcgggtttct catatcctgg atctagaacc 1260
   gcattttgtg ttgcttcgga agcacattga tggccacaat ggagactact tcgacatcca 1320
   ggagctctac ttccggttct cgatggatgt ggcgacgggg tttttgtttg gcgagtctgt 1380
   ggggtcgttg aaagacgaag atgcgaggtt cctggaagca ttcaatgagt cgcagaagta 1440
   tttggcaact agggcaacgt tgcacgagtt gtactttctt tgtgacgggt ttaggtttcg 1500
   ccagtacaac aaggttgtgc gaaagttctg cagccagtgt gtccacaagg cgttagatgt 1560
   tgcaccggaa gacaccagcg agtacgtgtt tctccgcgag ttggtcaaac acactcgaga 1620
   tcccgttgtt ttacaagacc aagcgttgaa cgtcttgctt gctggacgcg acaccaccgc 1680
   gtcgttatta tcgtttgcaa catttgagct agcccggaat gaccacatgt ggaggaagct 1740
   acgagaggag gttatcctga cgatgggacc gtccagtgat gaaataaccg tggccgggtt 1800
   gaagagttgc cgttacctca aagcaatcct aaacgaaact cttcgactat acccaagtgt 1860
   gcctaggaac gcgagatttg ctacgaggaa tacgacgctt cctcgtggcg gaggtccaga 1920
   tggatcgttt ccgattttga taagaaaggg ccagccagtg gggtatttca tttgtgctac 1980
   acacttgaat gagaaggtat atgggaatga tagccatgtg tttcgaccgg agagatgggc 2040
   tgcgttagag ggcaagagtt tgggctggtc gtatcttcca ttcaacggcg gcccgagaag 2100
   ctgccttggt cagcagtttg caatccttga agcttcgtat gttttggctc gattgacaca 2160
   gtgctacacg acgatacagc ttagaactac cgagtaccca ccaaagaaac tcgttcatct 2220
   cacgatgagt cttctcaacg gggtgtacat ccgaactaga acttgattat gtgtttatgg 2280
   ttaatcgggg caaagcactg caagtcattg atgtttgtgg aagcccagca ttggtgttcc 2340
   ggagcatcaa taaccaatgt cttgaagggt ttgattttct tgaccttctt cttcctgagc 2400
   ttctttccgt caaacttgta cagaatggcc atcatttcag gaacaaccac gtacgacggc 2460
   cggtaccgca tctggagtat ctcgccgtcg ttcaagtagc acgaaaacag caacgacgtc 2520
   accatctgct tcccaatctt gacacccaca gatacccctg cggcttcatg gatcaaaaac 2580
   gtcggcaacc ccgcgtatat gtccatgtaa ttctccatgg ccacctccat caacacactg 2640
   atggagcgac tgacggtgcc accactgccc tcggttgagt caaggcagta tgatgccggg 2700
   atccagtact ccaatgggaa cctctgcacg gtgtcgctgc agtttttgag gcgtatttcg 2760
   atccatgatc gttctttggt gctgtagtat aacgagctct tggtgtcctt gaaatggaac 2820
   aggttggatg tgttgttgag tttgtctgcg tgcttggttt gcaagtcttc gatcgagcgt 2880
   agtgagtaga cagttggcgg gggtggtggc tcgggcttta ttctgtgttt gtgtttcctt 2940
   cttagtcttg gaatgacgct gttatcgacg gttcgtagta taagtagcgc caatatgaga 3000
   atgtatatcc gcatcaccca agactcttca gcctgttaca acgactgagg ctgttggccg 3060
   tgtgaccaat tggtttcttt ggtgacctag attggtcccg cagggaaagc aagggctgct 3120
   aggggggcat accaaacaag gtcgtgtaat cagtatctat ggtgctacca tgtgtgtggt 3180
   tggggggaaa ttcccgcatt tttgtgtaac gaaagttcta gaaagttctc gtgggttctg 3240
   agaatctgct ggaaccatcc acccgcattt ccgttgccaa agtgggaaga gcaatcaacc 3300
   caccctgctt tgcccaatca gccattcccc tgggaatata aattcaac 3348
<210> 95
   <211> 523
   <212> PRT
   <213> Candida tropicalis
<400> 95
   Met Ala Thr Gln Glu Ile Ile Asp Ser Val Leu Pro Tyr Leu Thr Lys
   1 5 10 15
   Trp Tyr Thr Val Ile Thr Ala Ala Val Leu Val Phe Leu Ile Ser Thr
   20 25 30
   Asn Ile Lys Asn Tyr Val Lys Ala Lys Lys Leu Lys Cys Val Asp Pro
   35 40 45
   Pro Tyr Leu Lys Asp Ala Gly Leu Thr Gly Ile Leu Ser Leu Ile Ala
   50 55 60
   Ala Ile Lys Ala Lys Asn Asp Gly Arg Leu Ala Asn Phe Ala Asp Glu
   65 70 75 80
   Val Phe Asp Glu Tyr Pro Asn His Thr Phe Tyr Leu Ser Val Ala Gly
   85 90 95
   Ala Leu Lys Ile Val Met Thr Val Asp Pro Glu Asn Ile Lys Ala Val
   100 105 110
   Leu Ala Thr Gln Phe Thr Asp Phe Ser Leu Gly Thr Arg His Ala His
   115 120 125
   Phe Ala Pro Leu Leu Gly Asp Gly Ile Phe Thr Leu Asp Gly Glu Gly
   130 135 140
   Trp Lys His Ser Arg Ala Met Leu Arg Pro Gln Phe Ala Arg Asp Gln
   145 150 155 160
   Ile Gly His Val Lys Ala Leu Glu Pro His Ile Gln Ile Met Ala Lys
   165 170 175
   Gln Ile Lys Leu Asn Gln Gly Lys Thr Phe Asp Ile Gln Glu Leu Phe
   180 185 190
   Phe Arg Phe Thr Val Asp Thr Ala Thr Glu Phe Leu Phe Gly Glu Ser
   195 200 205
   Val His Ser Leu Tyr Asp Glu Lys Leu Gly Ile Pro Thr Pro Asn Glu
   210 215 220
   Ile Pro Gly Arg Glu Asn Phe Ala Ala Ala Phe Asn Val Ser Gln His
   225 230 235 240
   Tyr Leu Ala Thr Arg Ser Tyr Ser Gln Thr Phe Tyr Phe Leu Thr Asn
   245 250 255
   Pro Lys Glu Phe Arg Asp Cys Asn Ala Lys Val His His Leu Ala Lys
               260                 265                 270
   Tyr Phe Val Asn Lys Ala Leu Asn Phe Thr Pro Glu Glu Leu Glu Glu
           275                 280                 285
   Lys Ser Lys Ser Gly Tyr Val Phe Leu Tyr Glu Leu Val Lys Gln Thr
       290                 295                 300
   Arg Asp Pro Lys Val Leu Gln Asp Gln Leu Leu Asn Ile Met Val Ala
   305                 310                 315                 320
   Gly Arg Asp Thr Thr Ala Gly Leu Leu Ser Phe Ala Leu Phe Glu Leu
                   325                 330                 335
   Ala Arg His Pro Glu Met Trp Ser Lys Leu Arg Glu Glu Ile Glu Val
               340                 345                 350
   Asn Phe Gly Val Gly Glu Asp Ser Arg Val Glu Glu Ile Thr Phe Glu
           355                 360                 365
   Ala Leu Lys Arg Cys Glu Tyr Leu Lys Ala Ile Leu Asn Glu Thr Leu
       370                 375                 380
   Arg Met Tyr Pro Ser Val Pro Val Asn Phe Arg Thr Ala Thr Arg Asp
   385                 390                 395                 400
   Thr Thr Leu Pro Arg Gly Gly Gly Ala Asn Gly Thr Asp Pro Ile Tyr
                   405                 410                 415
   Ile Pro Lys Gly Ser Thr Val Ala Tyr Val Val Tyr Lys Thr His Arg
               420                 425                 430
   Leu Glu Glu Tyr Tyr Gly Lys Asp Ala Asn Asp Phe Arg Pro Glu Arg
           435                 440                 445
   Trp Phe Glu Pro Ser Thr Lys Lys Leu Gly Trp Ala Tyr Val Pro Phe
       450                 455                 460
   Asn Gly Gly Pro Arg Val Cys Leu Gly Gln Gln Phe Ala Leu Thr Glu
   465                 470                 475                 480
   Ala Ser Tyr Val Ile Thr Arg Leu Ala Gln Met Phe Glu Thr Val Ser
                   485                 490                 495
   Ser Asp Pro Gly Leu Glu Tyr Pro Pro Pro Lys Cys Ile His Leu Thr
               500                 505                 510
   Met Ser His Asn Asp Gly Val Phe Val Lys Met
   515 520
<210> 96
   <211> 522
   <212> PRT .
   <213> Candida tropicalis
<400> 96
   Met Thr Val His Asp Ile Ile Ala Thr Tyr Phe Thr Lys Trp Tyr Val
   1 5 10 15
   Ile Val Pro Leu Ala Leu Ile Ala Tyr Arg Val Leu Asp Tyr Phe Tyr
   20 25 30
   Gly Arg Tyr Leu Met Tyr Lys Leu Gly Ala Lys Pro Phe Phe Gln Lys
   35 40 45
   Gln Thr Asp Gly Cys Phe Gly Phe Lys Ala Pro Leu Glu Leu Leu Lys
   50 55 60
   Lys Lys Ser Asp Gly Thr Leu Ile Asp Phe Thr Leu Gln Arg Ile His
   65 70 75 80
   Asp Leu Asp Arg Pro Asp Ile Pro Thr Phe Thr Phe Pro Val Phe Ser
   85 90 95
   Ile Asn Leu Val Asn Thr Leu Glu Pro Glu Asn Ile Lys Ala Ile Leu
   100 105 110
   Ala Thr Gln Phe Asn Asp Phe Ser Leu Gly Thr Arg His Ser His Phe
   115 120 125
   Ala Pro Leu Leu Gly Asp Gly Ile Phe Thr Leu Asp Gly Ala Gly Trp
   130 135 140
   Lys His Ser Arg Ser Met Leu Arg Pro Gln Phe Ala Arg Glu Gln Ile
   145 150 155 160
   Ser His Val Lys Leu Leu Glu Pro His Val Gln Val Phe Phe Lys His
   165 170 175
   Val Arg Lys Ala Gln Gly Lys Thr Phe Asp Ile Gln Glu Leu Phe Phe
   180 185 190
   Arg Leu Thr Val Asp Ser Ala Thr Glu Phe Leu Phe Gly Glu Ser Val
   195 200 205
   Glu Ser Leu Arg Asp Glu Ser Ile Gly Met Ser Ile Asn Ala Leu Asp
       210                 215                 220
   Phe Asp Gly Lys Ala Gly Phe Ala Asp Ala Phe Asn Tyr Ser Gln Asn
   225                 230                 235                 240
   Tyr Leu Ala Ser Arg Ala Val Met Gln Gln Leu Tyr Trp Val Leu Asn
                   245                 250                 255
   Gly Lys Lys Phe Lys Glu Cys Asn Ala Lys Val His Lys Phe Ala Asp
               260                 265                 270
   Tyr Tyr Val Asn Lys Ala Leu Asp Leu Thr Pro Glu Gln Leu Glu Lys
           275                 280                 285
   Gln Asp Gly Tyr Val Phe Leu Tyr Glu Leu Val Lys Gln Thr Arg Asp
       290                 295                 300
   Lys Gln Val Leu Arg Asp Gln Leu Leu Asn Ile Met Val Ala Gly Arg
   305                 310                 315                 320
   Asp Thr Thr Ala Gly Leu Leu Ser Phe Val Phe Phe Glu Leu Ala Arg
                   325                 330                 335
   Asn Pro Glu Val Thr Asn Lys Leu Arg Glu Glu Ile Glu Asp Lys Phe
               340                 345                 350
   Gly Leu Gly Glu Asn Ala Ser Val Glu Asp Ile Ser Phe Glu Ser Leu
           355                 360                 365
   Lys Ser Cys Glu Tyr Leu Lys Ala Val Leu Asn Glu Thr Leu Arg Leu
       370                 375                 380
   Tyr Pro Ser Val Pro Gln Asn Phe Arg Val Ala Thr Lys Asn Thr Thr
   385                 390                 395                 400
   Leu Pro Arg Gly Gly Gly Lys Asp Gly Leu Ser Pro Val Leu Val Arg
                   405                 410                 415
   Lys Gly Gln Thr Val Ile Tyr Gly Val Tyr Ala Ala His Arg Asn Pro
               420                 425                 430
   Ala Val Tyr Gly Lys Asp Ala Leu Glu Phe Arg Pro Glu Arg Trp Phe
           435                 440                 445
   Glu Pro Glu Thr Lys Lys Leu Gly Trp Ala Phe Leu Pro Phe Asn Gly
       450                 455                 460
   Gly Pro Arg Ile Cys Leu Gly Gln Gln Phe Ala Leu Thr Glu Ala Ser
   465 470 475 480
   Tyr Val Thr Val Arg Leu Leu Gln Glu Phe Ala His Leu Ser Met Asp
   485 490 495
   Pro Asp Thr Glu Tyr Pro Pro Lys Lys Met Ser His Leu Thr Met Ser
   500 505 510
   Leu Phe Asp Gly Ala Asn Ile Glu Met Tyr
   515 520
<210> 97
   <211> 522
   <212> PRT
   <213> Candida tropicalis
<400> 97
   Met Thr Ala Gln Asp Ile Ile Ala Thr Tyr Ile Thr Lys Trp Tyr Val
   1 5 10 15
   Ile Val Pro Leu Ala Leu Ile Ala Tyr Arg Val Leu Asp Tyr Phe Tyr
   20 25 30
   Gly Arg Tyr Leu Met Tyr Lys Leu Gly Ala Lys Pro Phe Phe Gln Lys
   35 40 45
   Gln Thr Asp Gly Tyr Phe Gly Phe Lys Ala Pro Leu Glu Leu Leu Lys
   50 55 60
   Lys Lys Ser Asp Gly Thr Leu Ile Asp Phe Thr Leu Glu Arg Ile Gln
   65 70 75 80
   Ala Leu Asn Arg Pro Asp Ile Pro Thr Phe Thr Phe Pro Ile Phe Ser
   85 90 95
   Ile Asn Leu Ile Ser Thr Leu Glu Pro Glu Asn Ile Lys Ala Ile Leu
   100 105 110
   Ala Thr Gln Phe Asn Asp Phe Ser Leu Gly Thr Arg His Ser His Phe
   115 120 125
   Ala Pro Leu Leu Gly Asp Gly Ile Phe Thr Leu Asp Gly Ala Gly Trp
   130 135 140
   Lys His Ser Arg Ser Met Leu Arg Pro Gln Phe Ala Arg Glu Gln Ile
   145 150 155 160
   Ser His Val Lys Leu Leu Glu Pro His Met Gln Val Phe Phe Lys His
   165 170 175
   Val Arg Lys Ala Gln Gly Lys Thr Phe Asp Ile Gln Glu Leu Phe Phe
   180 185 190
   Arg Leu Thr Val Asp Ser Ala Thr Glu Phe Leu Phe Gly Glu Ser Val
   195 200 205
   Glu Ser Leu Arg Asp Glu Ser Ile Gly Met Ser Ile Asn Ala Leu Asp
   210 215 220
   Phe Asp Gly Lys Ala Gly Phe Ala Asp Ala Phe Asn Tyr Ser Gln Asn
   225 230 235 240
   Tyr Leu Ala Ser Arg Ala Val Met Gln Gln Leu Tyr Trp Val Leu Asn
   245 250 255
   Gly Lys Lys Phe Lys Glu Cys Asn Ala Lys Val His Lys Phe Ala Asp
   260 265 270
   Tyr Tyr Val Ser Lys Ala Leu Asp Leu Thr Pro Glu Gln Leu Glu Lys
   275 280 285
   Gln Asp Gly Tyr Val Phe Leu Tyr Glu Leu Val Lys Gln Thr Arg Asp
   290 295 300
   Arg Gln Val Leu Arg Asp Gln Leu Leu Asn Ile Met Val Ala Gly Arg
   305 310 315 320
   Asp Thr Thr Ala Gly Leu Leu Ser Phe Val Phe Phe Glu Leu Ala Arg
   325 330 335
   Asn Pro Glu Val Thr Asn Lys Leu Arg Glu Glu Ile Glu Asp Lys Phe
   340 345 350
   Gly Leu Gly Glu Asn Ala Arg Val Glu Asp Ile Ser Phe Glu Ser Leu
   355 360 365
   Lys Ser Cys Glu Tyr Leu Lys Ala Val Leu Asn Glu Thr Leu Arg Leu
   370 375 380
   Tyr Pro Ser Val Pro Gln Asn Phe Arg Val Ala Thr Lys Asn Thr Thr
   385 390 395 400
   Leu Pro Arg Gly Gly Gly Lys Asp Gly Leu Ser Pro Val Leu Val Arg
   405 410 415
   Lys Gly Gln Thr Val Met Tyr Gly Val Tyr Ala Ala His Arg Asn Pro
   420 425 430
   Ala Val Tyr Gly Lys Asp Ala Leu Glu Phe Arg Pro Glu Arg Trp Phe
   435 440 445
   Glu Pro Glu Thr Lys Lys Leu Gly Trp Ala Phe Leu Pro Phe Asn Gly
   450 455 460
   Gly Pro Arg Ile Cys Leu Gly Gln Gln Phe Ala Leu Thr Glu Ala Ser
   465 470 475 480
   Tyr Val Thr Val Arg Leu Leu Gln Glu Phe Gly His Leu Ser Met Asp
   485 490 495
   Pro Asn Thr Glu Tyr Pro Pro Arg Lys Met Ser His Leu Thr Met Ser
   500 505 510
   Leu Phe Asp Gly Ala Asn Ile Glu Met Tyr
   515 520
<210> 98
   <211> 540
   <212> PRT
   <213> Candida tropicalis
<400> 98
   Met Ser Ser Ser Pro Ser Phe Ala Gln Glu Val Leu Ala Thr Thr Ser
   1 5 10 15
   Pro Tyr Ile Glu Tyr Phe Leu Asp Asn Tyr Thr Arg Trp Tyr Tyr Phe
   20 25 30
   Ile Pro Leu Val Leu Leu Ser Leu Asn Phe Ile Ser Leu Leu His Thr
   35 40 45
   Arg Tyr Leu Glu Arg Arg Phe His Ala Lys Pro Leu Gly Asn Phe Val
   50 55 60
   Arg Asp Pro Thr Phe Gly Ile Ala Thr Pro Leu Leu Leu Ile Tyr Leu
   65 70 75 80
   Lys Ser Lys Gly Thr Val Met Lys Phe Ala Trp Gly Leu Trp Asn Asn
   85 90 95
   Lys Tyr Ile Val Arg Asp Pro Lys Tyr Lys Thr Thr Gly Leu Arg Ile
   100 105 110
   Val Gly Leu Pro Leu Ile Glu Thr Met Asp Pro Glu Asn Ile Lys Ala
   115 120 125
   Val Leu Ala Thr Gln Phe Asn Asp Phe Ser Leu Gly Thr Arg His Asp
   130 135 140
   Phe Leu Tyr Ser Leu Leu Gly Asp Gly Ile Phe Thr Leu Asp Gly Ala
   145 150 155 160
   Gly Trp Lys His Ser Arg Thr Met Leu Arg Pro Gln Phe Ala Arg Glu
   165 170 175
   Gln Val Ser His Val Lys Leu Leu Glu Pro His Val Gln Val Phe Phe
   180 185 190
   Lys His Val Arg Lys His Arg Gly Gln Thr Phe Asp Ile Gln Glu Leu
   195 200 205
   Phe Phe Arg Leu Thr Val Asp Ser Ala Thr Glu Phe Leu Phe Gly Glu
   210 215 220
   Ser Ala Glu Ser Leu Arg Asp Glu Ser Ile Gly Leu Thr Pro Thr Thr
   225 230 235 240
   Lys Asp Phe Asp Gly Arg Arg Asp Phe Ala Asp Ala Phe Asn Tyr Ser
   245 250 255
   Gln Thr Tyr Gln Ala Tyr Arg Phe Leu Leu Gln Gln Met Tyr Trp Ile
   260 265 270
   Leu Asn Gly Ser Glu Phe Arg Lys Ser Ile Ala Val Val His Lys Phe
   275 280 285
   Ala Asp His Tyr Val Gln Lys Ala Leu Glu Leu Thr Asp Asp Asp Leu
   290 295 300
   Gln Lys Gln Asp Gly Tyr Val Phe Leu Tyr Glu Leu Ala Lys Gln Thr
   305 310 315 320
   Arg Asp Pro Lys Val Leu Arg Asp Gln Leu Leu Asn Ile Leu Val Ala
   325 330 335
   Gly Arg Asp Thr Thr Ala Gly Leu Leu Ser Phe Val Phe Tyr Glu Leu
   340 345 350
   Ser Arg Asn Pro Glu Val Phe Ala Lys Leu Arg Glu Glu Val Glu Asn
   355 360 365
   Arg Phe Gly Leu Gly Glu Glu Ala Arg Val Glu Glu Ile Ser Phe Glu
   370 375 380
   Ser Leu Lys Ser Cys Glu Tyr Leu Lys Ala Val Ile Asn Glu Thr Leu
   385 390 395 400
   Arg Leu Tyr Pro Ser Val Pro His Asn Phe Arg Val Ala Thr Arg Asn
   405 410 415
   Thr Thr Leu Pro Arg Gly Gly Gly Glu Asp Gly Tyr Ser Pro Ile Val
   420 425 430
   Val Lys Lys Gly Gln Val Val Met Tyr Thr Val Ile Ala Thr His Arg
   435 440 445
   Asp Pro Ser Ile Tyr Gly Ala Asp Ala Asp Val Phe Arg Pro Glu Arg
   450 455 460
   Trp Phe Glu Pro Glu Thr Arg Lys Leu Gly Trp Ala Tyr Val Pro Phe
   465 470 475 480
   Asn Gly Gly Pro Arg Ile Cys Leu Gly Gln Gln Phe Ala Leu Thr Glu
   485 490 495
   Ala Ser Tyr Val Thr Val Arg Leu Leu Gln Glu Phe Ala His Leu Ser
   500 505 510
   Met Asp Pro Asp Thr Glu Tyr Pro Pro Lys Leu Gln Asn Thr Leu Thr
   515 520 525
   Leu Ser Leu Phe Asp Gly Ala Asp Val Arg Met Tyr
   530 535 540
<210> 99
   <211> 540
   <212> PRT
   <213> Candida tropicalis
<400> 99
   Met Ser Ser Ser Pro Ser Phe Ala Gln Glu Val Leu Ala Thr Thr Ser
   1 5 10 15
   Pro Tyr Ile Glu Tyr Phe Leu Asp Asn Tyr Thr Arg Trp Tyr Tyr Phe
   20 25 30
   Ile Pro Leu Val Leu Leu Ser Leu Asn Phe Ile Ser Leu Leu His Thr
           35                  40                  45
   Lys Tyr Leu Glu Arg Arg Phe His Ala Lys Pro Leu Gly Asn Val Val
       50                  55                  60
   Leu Asp Pro Thr Phe Gly Ile Ala Thr Pro Leu Ile Leu Ile Tyr Leu
   65                  70                  75                  80
   Lys Ser Lys Gly Thr Val Met Lys Phe Ala Trp Ser Phe Trp Asn Asn
                   85                  90                  95
   Lys Tyr Ile Val Lys Asp Pro Lys Tyr Lys Thr Thr Gly Leu Arg Ile
               100                 105                 110
   Val Gly Leu Pro Leu Ile Glu Thr Ile Asp Pro Glu Asn Ile Lys Ala
           115                 120                 125
   Val Leu Ala Thr Gln Phe Asn Asp Phe Ser Leu Gly Thr Arg His Asp
       130                 135                 140
   Phe Leu Tyr Ser Leu Leu Gly Asp Gly Ile Phe Thr Leu Asp Gly Ala
   145                 150                 155                 160
   Gly Trp Lys His Ser Arg Thr Met Leu Arg Pro Gln Phe Ala Arg Glu
                   165                 170                 175
   Gln Val Ser His Val Lys Leu Leu Glu Pro His Val Gln Val Phe Phe
               180                 185                 190
   Lys His Val Arg Lys His Arg Gly Gln Thr Phe Asp Ile Gln Glu Leu
           195                 200                 205
   Phe Phe Arg Leu Thr Val Asp Ser Ala Thr Glu Phe Leu Phe Gly Glu
       210                 215                 220
   Ser Ala Glu Ser Leu Arg Asp Asp Ser Val Gly Leu Thr Pro Thr Thr
   225                 230                 235                 240
   Lys Asp Phe Glu Gly Arg Gly Asp Phe Ala Asp Ala Phe Asn Tyr Ser
                   245                 250                 255
   Gln Thr Tyr Gln Ala Tyr Arg Phe Leu Leu Gln Gln Met Tyr Trp Ile
               260                 265                 270
   Leu Asn Gly Ala Glu Phe Arg Lys Ser Ile Ala Ile Val His Lys Phe
           275                 280                 285
   Ala Asp His Tyr Val Gln Lys Ala Leu Glu Leu Thr Asp Asp Asp Leu
       290                 295                 300
   Gln Lys Gln Asp Gly Tyr Val Phe Leu Tyr Glu Leu Ala Lys Gln Thr
   305                 310                 315                 320
   Arg Asp Pro Lys Val Leu Arg Asp Gln Leu Leu Asn Ile Leu Val Ala
                   325                 330                 335
   Gly Arg Asp Thr Thr Ala Gly Leu Leu Ser Phe Val Phe Tyr Glu Leu
               340                 345                 350
   Ser Arg Asn Pro Glu Val Phe Ala Lys Leu Arg Glu Glu Val Glu Asn
           355                 360                 365
   Arg Phe Gly Leu Gly Glu Glu Ala Arg Val Glu Glu Ile Ser Phe Glu
       370                 375                 380
   Ser Leu Lys Ser Cys Glu Tyr Leu Lys Ala Val Ile Asn Glu Ala Leu
   385                 390                 395                 400
   Arg Leu Tyr Pro Ser Val Pro His Asn Phe Arg Val Ala Thr Arg Asn
                   405                 410                 415
   Thr Thr Leu Pro Arg Gly Gly Gly Lys Asp Gly Cys Ser Pro Ile Val
               420                 425                 430
   Val Lys Lys Gly Gln Val Val Met Tyr Thr Val Ile Gly Thr His Arg
           435                 440                 445
   Asp Pro Ser Ile Tyr Gly Ala Asp Ala Asp Val Phe Arg Pro Glu Arg
       450                 455                 460
   Trp Phe Glu Pro Glu Thr Arg Lys Leu Gly Trp Ala Tyr Val Pro Phe
   465                 470                 475                 480
   Asn Gly Gly Pro Arg Ile Cys Leu Gly Gln Gln Phe Ala Leu Thr Glu
                   485                 490                 495
   Ala Ser Tyr Val Thr Val Arg Leu Leu Gln Glu Phe Gly Asn Leu Ser
               500                 505                 510
   Leu Asp Pro Asn Ala Glu Tyr Pro Pro Lys Leu Gln Asn Thr Leu Thr
           515                 520                 525
   Leu Ser Leu Phe Asp Gly Ala Asp Val Arg Met Phe
       530                 535                 540
<210> 100
   <211> 517
   <212> PRT
   <213> Candida tropicalis
<400> 100
   Met Ile Glu Gln Leu Leu Glu Tyr Trp Tyr Val Val Val Pro Val Leu
   1               5                   10                  15
   Tyr Ile Ile Lys Gln Leu Leu Ala Tyr Thr Lys Thr Arg Val Leu Met
               20                  25                  30
   Lys Lys Leu Gly Ala Ala Pro Val Thr Asn Lys Leu Tyr Asp Asn Ala
           35                  40                  45
   Phe Gly Ile Val Asn Gly Trp Lys Ala Leu Gln Phe Lys Lys Glu Gly
       50                  55                  60
   Arg Ala Gln Glu Tyr Asn Asp Tyr Lys Phe Asp His Ser Lys Asn Pro
   65                  70                  75                  80
   Ser Val Gly Thr Tyr Val Ser Ile Leu Phe Gly Thr Arg Ile Val Val
                   85                  90                  95
   Thr Lys Asp Pro Glu Asn Ile Lys Ala Ile Leu Ala Thr Gln Phe Gly
               100                 105                 110
   Asp Phe Ser Leu Gly Lys Arg His Thr Leu Phe Lys Pro Leu Leu Gly
           115                 120                 125
   Asp Gly Ile Phe Thr Leu Asp Gly Glu Gly Trp Lys His Ser Arg Ala
       130                 135                 140
   Met Leu Arg Pro Gln Phe Ala Arg Glu Gln Val Ala His Val Thr Ser
   145                 150                 155                 160
   Leu Glu Pro His Phe Gln Leu Leu Lys Lys His Ile Leu Lys His Lys
                   165                 170                 175
   Gly Glu Tyr Phe Asp Ile Gln Glu Leu Phe Phe Arg Phe Thr Val Asp
               180                 185                 190
   Ser Ala Thr Glu Phe Leu Phe Gly Glu Ser Val His Ser Leu Lys Asp
           195                 200                 205
   Glu Ser Ile Gly Ile Asn Gln Asp Asp Ile Asp Phe Ala Gly Arg Lys
   210 215 220
   Asp Phe Ala Glu Ser Phe Asn Lys Ala Gln Glu Tyr Leu Ala Ile Arg
   225 230 235 240
   Thr Leu Val Gln Thr Phe Tyr Trp Leu Val Asn Asn Lys Glu Phe Arg
   245 250 255
   Asp Cys Thr Lys Leu Val His Lys Phe Thr Asn Tyr Tyr Val Gln Lys
   260 265 270
   Ala Leu Asp Ala Ser Pro Glu Glu Leu Glu Lys Gln Ser Gly Tyr Val
   275 280 285
   Phe Leu Tyr Glu Leu Val Lys Gln Thr Arg Asp Pro Asn Val Leu Arg
   290 295 300
   Asp Gln Ser Leu Asn Ile Leu Leu Ala Gly Arg Asp Thr Thr Ala Gly
   305 310 315 320
   Leu Leu Ser Phe Ala Val Phe Glu Leu Ala Arg His Pro Glu Ile Trp
   325 330 335
   Ala Lys Leu Arg Glu Glu Ile Glu Gln Gln Phe Gly Leu Gly Glu Asp
   340 345 350
   Ser Arg Val Glu Glu Ile Thr Phe Glu Ser Leu Lys Arg Cys Glu Tyr
   355 360 365
   Leu Lys Ala Phe Leu Asn Glu Thr Leu Arg Ile Tyr Pro Ser Val Pro
   370 375 380
   Arg Asn Phe Arg Ile Ala Thr Lys Asn Thr Thr Leu Pro Arg Gly Gly
   385 390 395 400
   Gly Ser Asp Gly Thr Ser Pro Ile Leu Ile Gln Lys Gly Glu Ala Val
   405 410 415
   Ser Tyr Gly Ile Asn Ser Thr His Leu Asp Pro Val Tyr Tyr Gly Pro
   420 425 430
   Asp Ala Ala Glu Phe Arg Pro Glu Arg Trp Phe Glu Pro Ser Thr Lys
   435 440 445
   Lys Leu Gly Trp Ala Tyr Leu Pro Phe Asn Gly Gly Pro Arg Ile Cys
   450 455 460
   Leu Gly Gln Gln Phe Ala Leu Thr Glu Ala Gly Tyr Val Leu Val Arg
   465 470 475 480
   Leu Val Gln Glu Phe Ser His Val Arg Leu Asp Pro Asp Glu Val Tyr
   485 490 495
   Pro Pro Lys Arg Leu Thr Asn Leu Thr Met Cys Leu Gln Asp Gly Ala
   500 505 510
   Ile Val Lys Phe Asp
   515
<210> 101
   <211> 517
   <212> PRT
   <213> Candida tropicalis
<400> 101
   Met Ile Glu Gln Ile Leu Glu Tyr Trp Tyr Ile Val Val Pro Val Leu
   1 5 10 15
   Tyr Ile Ile Lys Gln Leu Ile Ala Tyr Ser Lys Thr Arg Val Leu Met
   20 25 30
   Lys Gln Leu Gly Ala Ala Pro Ile Thr Asn Gln Leu Tyr Asp Asn Val
   35 40 45
   Phe Gly Ile Val Asn Gly Trp Lys Ala Leu Gln Phe Lys Lys Glu Gly
   50 55 60
   Arg Ala Gln Glu Tyr Asn Asp His Lys Phe Asp Ser Ser Lys Asn Pro
   65 70 75 80
   Ser Val Gly Thr Tyr Val Ser Ile Leu Phe Gly Thr Lys Ile Val Val
   85 90 95
   Thr Lys Asp Pro Glu Asn Ile Lys Ala Ile Leu Ala Thr Gln Phe Gly
   100 105 110
   Asp Phe Ser Leu Gly Lys Arg His Ala Leu Phe Lys Pro Leu Leu Gly
   115 120 125
   Asp Gly Ile Phe Thr Leu Asp Gly Glu Gly Trp Lys His Ser Arg Ser
   130 135 140
   Met Leu Arg Pro Gln Phe Ala Arg Glu Gln Val Ala His Val Thr Ser
   145 150 155 160
   Leu Glu Pro His Phe Gln Leu Leu Lys Lys His Ile Leu Lys His Lys
                   165                 170                 175
   Gly Glu Tyr Phe Asp Ile Gln Glu Leu Phe Phe Arg Phe Thr Val Asp
               180                 185                 190
   Ser Ala Thr Glu Phe Leu Phe Gly Glu Ser Val His Ser Leu Lys Asp
           195                 200                 205
   Glu Thr Ile Gly Ile Asn Gln Asp Asp Ile Asp Phe Ala Gly Arg Lys
       210                 215                 220
   Asp Phe Ala Glu Ser Phe Asn Lys Ala Gln Glu Tyr Leu Ser Ile Arg
   225                 230                 235                 240
   Ile Leu Val Gln Thr Phe Tyr Trp Leu Ile Asn Asn Lys Glu Phe Arg
                   245                 250                 255
   Asp Cys Thr Lys Leu Val His Lys Phe Thr Asn Tyr Tyr Val Gln Lys
               260                 265                 270
   Ala Leu Asp Ala Thr Pro Glu Glu Leu Glu Lys Gln Gly Gly Tyr Val
           275                 280                 285
   Phe Leu Tyr Glu Leu Val Lys Gln Thr Arg Asp Pro Lys Val Leu Arg
       290                 295                 300
   Asp Gln Ser Leu Asn Ile Leu Leu Ala Gly Arg Asp Thr Thr Ala Gly
   305                 310                 315                 320
   Leu Leu Ser Phe Ala Val Phe Glu Leu Ala Arg Asn Pro His Ile Trp
                   325                 330                 335
   Ala Lys Leu Arg Glu Glu Ile Glu Gln Gln Phe Gly Leu Gly Glu Asp
               340                 345                 350
   Ser Arg Val Glu Glu Ile Thr Phe Glu Ser Leu Lys Arg Cys Glu Tyr
           355                 360                 365
   Leu Lys Ala Phe Leu Asn Glu Thr Leu Arg Val Tyr Pro Ser Val Pro
       370                 375                 380
   Arg Asn Phe Arg Ile Ala Thr Lys Asn Thr Thr Leu Pro Arg Gly Gly
   385                 390                 395                 400
   Gly Pro Asp Gly Thr Gln Pro Ile Leu Ile Gln Lys Gly Glu Gly Val
                   405                 410                 415
   Ser Tyr Gly Ile Asn Ser Thr His Leu Asp Pro Val Tyr Tyr Gly Pro
   420 425 430
   Asp Ala Ala Glu Phe Arg Pro Glu Arg Trp Phe Glu Pro Ser Thr Arg
   435 440 445
   Lys Leu Gly Trp Ala Tyr Leu Pro Phe Asn Gly Gly Pro Arg Ile Cys
   450 455 460
   Leu Gly Gln Gln Phe Ala Leu Thr Glu Ala Gly Tyr Val Leu Val Arg
   465 470 475 480
   Leu Val Gln Glu Phe Ser His Ile Arg Leu Asp Pro Asp Glu Val Tyr
   485 490 495
   Pro Pro Lys Arg Leu Thr Asn Leu Thr Met Cys Leu Gln Asp Gly Ala
   500 505 510
   Ile Val Lys Phe Asp
   515
<210> 102
   <211> 512
   <212> PRT
   <213> Candida tropicalis
<400> 102
   Met Leu Asp Gln Ile Leu His Tyr Trp Tyr Ile Val Leu Pro Leu Leu
   1 5 10 15
   Ala Ile Ile Asn Gln Ile Val Ala His Val Arg Thr Asn Tyr Leu Met
   20 25 30
   Lys Lys Leu Gly Ala Lys Pro Phe Thr His Val Gln Arg Asp Gly Trp
   35 40 45
   Leu Gly Phe Lys Phe Gly Arg Glu Phe Leu Lys Ala Lys Ser Ala Gly
   50 55 60
   Arg Leu Val Asp Leu Ile Ile Ser Arg Phe His Asp Asn Glu Asp Thr
   65 70 75 80
   Phe Ser Ser Tyr Ala Phe Gly Asn His Val Val Phe Thr Arg Asp Pro
   85 90 95
   Glu Asn Ile Lys Ala Leu Leu Ala Thr Gln Phe Gly Asp Phe Ser Leu
   100 105 110
   Gly Ser Arg Val Lys Phe Phe Lys Pro Leu Leu Gly Tyr Gly Ile Phe
           115                 120                 125
   Thr Leu Asp Ala Glu Gly Trp Lys His Ser Arg Ala Met Leu Arg Pro
       130                 135                 140
   Gln Phe Ala Arg Glu Gln Val Ala His Val Thr Ser Leu Glu Pro His
   145                 150                 155                 160
   Phe Gln Leu Leu Lys Lys His Ile Leu Lys His Lys Gly Glu Tyr Phe
                   165                 170                 175
   Asp Ile Gln Glu Leu Phe Phe Arg Phe Thr Val Asp Ser Ala Thr Glu
               180                 185                 190
   Phe Leu Phe Gly Glu Ser Val His Ser Leu Lys Asp Glu Glu Ile Gly
           195                 200                 205
   Tyr Asp Thr Lys Asp Met Ser Glu Glu Arg Arg Arg Phe Ala Asp Ala
       210                 215                 220
   Phe Asn Lys Ser Gln Val Tyr Val Ala Thr Arg Val Ala Leu Gln Asn
   225                 230                 235                 240
   Leu Tyr Trp Leu Val Asn Asn Lys Glu Phe Lys Glu Cys Asn Asp Ile
                   245                 250                 255
   Val His Lys Phe Thr Asn Tyr Tyr Val Gln Lys Ala Leu Asp Ala Thr
               260                 265                 270
   Pro Glu Glu Leu Glu Lys Gln Gly Gly Tyr Val Phe Leu Tyr Glu Leu
           275                 280                 285
   Val Lys Gln Thr Arg Asp Pro Lys Val Leu Arg Asp Gln Ser Leu Asn
       290                 295                 300
   Ile Leu Leu Ala Gly Arg Asp Thr Thr Ala Gly Leu Leu Ser Phe Ala
   305                 310                 315                 320
   Val Phe Glu Leu Ala Arg Asn Pro His Ile Trp Ala Lys Leu Arg Glu
                   325                 330                 335
   Glu Ile Glu Gln Gln Phe Gly Leu Gly Glu Asp Ser Arg Val Glu Glu
               340                 345                 350
   Ile Thr Phe Glu Ser Leu Lys Arg Cys Glu Tyr Leu Lys Ala Val Leu
           355                 360                 365
   Asn Glu Thr Leu Arg Leu His Pro Ser Val Pro Arg Asn Ala Arg Phe
   370 375 380
   Ala Ile Lys Asp Thr Thr Leu Pro Arg Gly Gly Gly Pro Asn Gly Lys
   385 390 395 400
   Asp Pro Ile Leu Ile Arg Lys Asp Glu Val Val Gln Tyr Ser Ile Ser
   405 410 415
   Ala Thr Gln Thr Asn Pro Ala Tyr Tyr Gly Ala Asp Ala Ala Asp Phe
   420 425 430
   Arg Pro Glu Arg Trp Phe Glu Pro Ser Thr Arg Asn Leu Gly Trp Ala
   435 440 445
   Phe Leu Pro Phe Asn Gly Gly Pro Arg Ile Cys Leu Gly Gln Gln Phe
   450 455 460
   Ala Leu Thr Glu Ala Gly Tyr Val Leu Val Arg Leu Val Gln Glu Phe
   465 470 475 480
   Pro Asn Leu Ser Gln Asp Pro Glu Thr Lys Tyr Pro Pro Pro Arg Leu
   485 490 495
   Ala His Leu Thr Met Cys Leu Phe Asp Gly Ala His Val Lys Met Ser
   500 505 510
<210> 103
   <211> 512
   <212> PRT
   <213> Candida tropicalis
<400> 103
   Met Leu Asp Gln Ile Phe His Tyr Trp Tyr Ile Val Leu Pro Leu Leu
   1 5 10 15
   Val Ile Ile Lys Gln Ile Val Ala His Ala Arg Thr Asn Tyr Leu Met
   20 25 30
   Lys Lys Leu Gly Ala Lys Pro Phe Thr His Val Gln Leu Asp Gly Trp
   35 40 45
   Phe Gly Phe Lys Phe Gly Arg Glu Phe Leu Lys Ala Lys Ser Ala Gly
   50 55 60
   Arg Gln Val Asp Leu Ile Ile Ser Arg Phe His Asp Asn Glu Asp Thr
   65 70 75 80
   Phe Ser Ser Tyr Ala Phe Gly Asn His Val Val Phe Thr Arg Asp Pro
   85 90 95
   Glu Asn Ile Lys Ala Leu Leu Ala Thr Gln Phe Gly Asp Phe Ser Leu
   100 105 110
   Gly Ser Arg Val Lys Phe Phe Lys Pro Leu Leu Gly Tyr Gly Ile Phe
   115 120 125
   Thr Leu Asp Gly Glu Gly Trp Lys His Ser Arg Ala Met Leu Arg Pro
   130 135 140
   Gln Phe Ala Arg Glu Gln Val Ala His Val Thr Ser Leu Glu Pro His
   145 150 155 160
   Phe Gln Leu Leu Lys Lys His Ile Leu Lys His Lys Gly Glu Tyr Phe
   165 170 175
   Asp Ile Gln Glu Leu Phe Phe Arg Phe Thr Val Asp Ser Ala Thr Glu
   180 185 190
   Phe Leu Phe Gly Glu Ser Val His Ser Leu Arg Asp Glu Glu Ile Gly
   195 200 205
   Tyr Asp Thr Lys Asp Met Ala Glu Glu Arg Arg Lys Phe Ala Asp Ala
   210 215 220
   Phe Asn Lys Ser Gln Val Tyr Leu Ser Thr Arg Val Ala Leu Gln Thr
   225 230 235 240
   Leu Tyr Trp Leu Val Asn Asn Lys Glu Phe Lys Glu Cys Asn Asp Ile
   245 250 255
   Val His Lys Phe Thr Asn Tyr Tyr Val Gln Lys Ala Leu Asp Ala Thr
   260 265 270
   Pro Glu Glu Leu Glu Lys Gln Gly Gly Tyr Val Phe Leu Tyr Glu Leu
   275 280 285
   Ala Lys Gln Thr Lys Asp Pro Asn Val Leu Arg Asp Gln Ser Leu Asn
   290 295 300
   Ile Leu Leu Ala Gly Arg Asp Thr Thr Ala Gly Leu Leu Ser Phe Ala
   305 310 315 320
   Val Phe Glu Leu Ala Arg Asn Pro His Ile Trp Ala Lys Leu Arg Glu
   325 330 335
   Glu Ile Glu Ser His Phe Gly Leu Gly Glu Asp Ser Arg Val Glu Glu
   340 345 350
   Ile Thr Phe Glu Ser Leu Lys Arg Cys Glu Tyr Leu Lys Ala Val Leu
   355 360 365
   Asn Glu Thr Leu Arg Leu His Pro Ser Val Pro Arg Asn Ala Arg Phe
   370 375 380
   Ala Ile Lys Asp Thr Thr Leu Pro Arg Gly Gly Gly Pro Asn Gly Lys
   385 390 395 400
   Asp Pro Ile Leu Ile Arg Lys Asn Glu Val Val Gln Tyr Ser Ile Ser
   405 410 415
   Ala Thr Gln Thr Asn Pro Ala Tyr Tyr Gly Ala Asp Ala Ala Asp Phe
   420 425 430
   Arg Pro Glu Arg Trp Phe Glu Pro Ser Thr Arg Asn Leu Gly Trp Ala
   435 440 445
   Tyr Leu Pro Phe Asn Gly Gly Pro Arg Ile Cys Leu Gly Gln Gln Phe
   450 455 460
   Ala Leu Thr Glu Ala Gly Tyr Val Leu Val Arg Leu Val Gln Glu Phe
   465 470 475 480
   Pro Ser Leu Ser Gln Asp Pro Glu Thr Glu Tyr Pro Pro Pro Arg Leu
   485 490 495
   Ala His Leu Thr Met Cys Leu Phe Asp Gly Ala Tyr Val Lys Met Gln
   500 505 510
<210> 104
   <211> 499
   <212> PRT
   <213> Candida tropicalis
<400> 104
   Met Ala Ile Ser Ser Leu Leu Ser Trp Asp Val Ile Cys Val Val Phe
   1 5 10 15
   Ile Cys Val Cys Val Tyr Phe Gly Tyr Glu Tyr Cys Tyr Thr Lys Tyr
   20 25 30
   Leu Met His Lys His Gly Ala Arg Glu Ile Glu Asn Val Ile Asn Asp
   35 40 45
   Gly Phe Phe Gly Phe Arg Leu Pro Leu Leu Leu Met Arg Ala Ser Asn
   50 55 60
   Glu Gly Arg Leu Ile Glu Phe Ser Val Lys Arg Phe Glu Ser Ala Pro
   65 70 75 80
   His Pro Gln Asn Lys Thr Leu Val Asn Arg Ala Leu Ser Val Pro Val
   85 90 95
   Ile Leu Thr Lys Asp Pro Val Asn Ile Lys Ala Met Leu Ser Thr Gln
   100 105 110
   Phe Asp Asp Phe Ser Leu Gly Leu Arg Leu His Gln Phe Ala Pro Leu
   115 120 125
   Leu Gly Lys Gly Ile Phe Thr Leu Asp Gly Pro Glu Trp Lys Gln Ser
   130 135 140
   Arg Ser Met Leu Arg Pro Gln Phe Ala Lys Asp Arg Val Ser His Ile
   145 150 155 160
   Leu Asp Leu Glu Pro His Phe Val Leu Leu Arg Lys His Ile Asp Gly
   165 170 175
   His Asn Gly Asp Tyr Phe Asp Ile Gln Glu Leu Tyr Phe Arg Phe Ser
   180 185 190
   Met Asp Val Ala Thr Gly Phe Leu Phe Gly Glu Ser Val Gly Ser Leu
   195 200 205
   Lys Asp Glu Asp Ala Arg Phe Leu Glu Ala Phe Asn Glu Ser Gln Lys
   210 215 220
   Tyr Leu Ala Thr Arg Ala Thr Leu His Glu Leu Tyr Phe Leu Cys Asp
   225 230 235 240
   Gly Phe Arg Phe Arg Gln Tyr Asn Lys Val Val Arg Lys Phe Cys Ser
   245 250 255
   Gln Cys Val His Lys Ala Leu Asp Val Ala Pro Glu Asp Thr Ser Glu
   260 265 270
   Tyr Val Phe Leu Arg Glu Leu Val Lys His Thr Arg Asp Pro Val Val
   275 280 285
   Leu Gln Asp Gln Ala Leu Asn Val Leu Leu Ala Gly Arg Asp Thr Thr
   290 295 300
   Ala Ser Leu Leu Ser Phe Ala Thr Phe Glu Leu Ala Arg Asn Asp His
   305 310 315 320
   Met Trp Arg Lys Leu Arg Glu Glu Val Ile Leu Thr Met Gly Pro Ser
   325 330 335
   Ser Asp Glu Ile Thr Val Ala Gly Leu Lys Ser Cys Arg Tyr Leu Lys
   340 345 350
   Ala Ile Leu Asn Glu Thr Leu Arg Leu Tyr Pro Ser Val Pro Arg Asn
   355 360 365
   Ala Arg Phe Ala Thr Arg Asn Thr Thr Leu Pro Arg Gly Gly Gly Pro
   370 375 380
   Asp Gly Ser Phe Pro Ile Leu Ile Arg Lys Gly Gln Pro Val Gly Tyr
   385 390 395 400
   Phe Ile Cys Ala Thr His Leu Asn Glu Lys Val Tyr Gly Asn Asp Ser
   405 410 415
   His Val Phe Arg Pro Glu Arg Trp Ala Ala Leu Glu Gly Lys Ser Leu
   420 425 430
   Gly Trp Ser Tyr Leu Pro Phe Asn Gly Gly Pro Arg Ser Cys Leu Gly
   435 440 445
   Gln Gln Phe Ala Ile Leu Glu Ala Ser Tyr Val Leu Ala Arg Leu Thr
   450 455 460
   Gln Cys Tyr Thr Thr Ile Gln Leu Arg Thr Thr Glu Tyr Pro Pro Lys
   465 470 475 480
   Lys Leu Val His Leu Thr Met Ser Leu Leu Asn Gly Val Tyr Ile Arg
   485 490 495
   Thr Arg Thr
<210> 105
   <211> 1712
   <212> DNA
   <213> Candida tropicalis
<400> 105
   ggtaccgagc tcacgagttt tgggattttc gagtttggat tgtttccttt gttgattgaa 60
   ttgacgaaac cagaggtttt caagacagat aagattgggt ttatcaaaac gcagtttgaa 120
   atattccagt tggtttccaa gatatcttga agaagattga cgatttgaaa tttgaagaag 180
   tggagaagat ctggtttgga ttgttggaga atttcaagaa tctcaagatt tactctaacg 240
   acgggtacaa cgagaattgt attgaattga tcaagaacat gatcttggtg ttacagaaca 300
   tcaagttctt ggaccagact gagaatgcca cagatataca aggcgtcatg tgataaaatg 360
   gatgagattt atcccacaat tgaagaaaga gtttatggaa agtggtcaac cagaagctaa 420
   acaggaagaa gcaaacgaag aggtgaaaca agaagaagaa ggtaaataag tattttgtat 480
   tatataacaa acaaagtaag gaatacagat ttatacaata aattgccata ctagtcacgt 540
   gagatatctc atccattccc caactcccaa gaaaaaaaaa aagtgaaaaa aaaaatcaaa 600
   cccaaagatc aacctcccca tcatcatcgt catcaaaccc ccagctcaat tcgcaatggt 660
   tagcacaaaa acatacacag aaagggcatc agcacacccc tccaaggttg cccaacgttt 720
   attccgctta atggagtcca aaaagaccaa cctctgcgcc tcgatcgacg tgaccacaac 780
   cgccgagttc ctttcgctca tcgacaagct cggtccccac atctgtctcg tgaagacgca 840
   catcgatatc atctcagact tcagctacga gggcacgatt gagccgttgc ttgtgcttgc 900
   agagcgccac gggttcttga tattcgagga caggaagttt gctgatatcg gaaacaccgt 960
   gatgttgcag tacacctcgg gggtataccg gatcgcggcg tggagtgaca tcacgaacgc 1020
   gcacggagtg actgggaagg gcgtcgttga agggttgaaa cgcggtgcgg aggggtagaa 1080
   aaaggaaagg ggcgtgttga tgttggcgga gttgtcgagt aaaggctcgt tggcgcatgg 1140
   tgaatatacc cgtgagacga tcgagattgc gaagagtgat cgggagttcg tgattgggtt 1200
   catcgcgcag cgggacatgg ggggtagaga agaagggttt gattggatca tcatgacgcc 1260
   tggtgtgggg ttggatgata aaggcgatgc gttgggccag cagtatagga ctgttgatga 1320
   ggtggttctg actggtaccg atgtgattat tgtcgggaga gggttgtttg gaaaaggaag 1380
   agaccctgag gtggagggaa agagatacag ggatgctgga tggaaggcat acttgaagag 1440
   aactggtcag ttagaataaa tattgtaata aataggtcta tatacataca ctaagcttct 1500
   aggacgtcat tgtagtcttc gaagttgtct gctagtttag ttctcatgat ttcgaaaacc 1560
   aataacgcaa tggatgtagc agggatggtg gttagtgcgt tcctgacaaa cccagagtac 1620
   gccgcctcaa accacgtcac attcgccctt tgcttcatcc gcatcacttg cttgaaggta 1680
   tccacgtacg agttgtaata caccttgaag aa                               1712
<210> 106
   <211> 267
   <212> PRT
   <213> Candida tropicalis
<400> 106
   Met Val Ser Thr Lys Thr Tyr Thr Glu Arg Ala Ser Ala His Pro Ser
   1               5                   10                  15
   Lys Val Ala Gln Arg Leu Phe Arg Leu Met Glu Ser Lys Lys Thr Asn
                   20                  25                  30
   Leu Cys Ala Ser Ile Asp Val Thr Thr Thr Ala Glu Phe Leu Ser Leu
           35                  40                  45
   Ile Asp Lys Leu Gly Pro His Ile Cys Leu Val Lys Thr His Ile Asp
       50                  55                  60
   Ile Ile Ser Asp Phe Ser Tyr Glu Gly Thr Ile Glu Pro Leu Leu Val
   65                  70                  75                  80
   Leu Ala Glu Arg His Gly Phe Leu Ile Phe Glu Asp Arg Lys Phe Ala
                   85                  90                  95
   Asp Ile Gly Asn Thr Val Met Leu Gln Tyr Thr Ser Gly Val Tyr Arg
               100                 105                 110
   Ile Ala Ala Trp Ser Asp Ile Thr Asn Ala His Gly Val Thr Gly Lys
           115                 120                 125
   Gly Val Val Glu Gly Leu Lys Arg Gly Ala Glu Gly Val Glu Lys Glu
       130                 135                 140
   Arg Gly Val Leu Met Leu Ala Glu Leu Ser Ser Lys Gly Ser Leu Ala
   145                 150                 155                 160
   His Gly Glu Tyr Thr Arg Glu Thr Ile Glu Ile Ala Lys Ser Asp Arg
                   165                 170                 175
   Glu Phe Val Ile Gly Phe Ile Ala Gln Arg Asp Met Gly Gly Arg Glu
               180                 185                 190
   Glu Gly Phe Asp Trp Ile Ile Met Thr Pro Gly Val Gly Leu Asp Asp
           195                 200                 205
   Lys Gly Asp Ala Leu Gly Gln Gln Tyr Arg Thr Val Asp Glu Val Val
       210                 215                 220
   Leu Thr Gly Thr Asp Val Ile Ile Val Gly Arg Gly Leu Phe Gly Lys
   225                 230                 235                 240
   Gly Arg Asp Pro Glu Val Glu Gly Lys Arg Tyr Arg Asp Ala Gly Trp
                   245                 250                 255
   Lys Ala Tyr Leu Lys Arg Thr Gly Gln Leu Glu
               260                 265
<210> 107
   <211> 473
   <212> DNA
   <213> Artificial Sequence
<220>
   <223> Description of Artificial Sequence: Probe for CYP52A1 gene
<400> 107
   gtcaaagcaa attgttggcc caagcagact cttggaccac cgttgaatgg aacataagcc 60
   cagcccaact tcttagtaga tggttcaaac catctttctg gtctgaagtc gttagcgtcc 120
   ttaccgtagt attcttccaa acggtgggtc ttgtagacaa cgtaagcaac agtggagcct 180
   ttaggaatgt agattgggtc ggtaccgtta gcaccaccac ctcttggcaa agtggtgtct 240
   ctggtggcgg ttctaaagtt gacaggaaca gatgggtaca tacgcaaggt ttcgttaagg 300
   atagccttca agtattcaca tctcttcaag gcttcgaaag taatttcttc aacgcgggag 360
   tcttcaccaa caccaaagtt aacttcgatt tcttctctca acttggacca catctctggg 420
   tgtctagcca attcaaacaa agcaaaggac aacaaacccg cggtggtgtc tct       473

## Description

### CROSS REFERENCE TO RELATED APPLICATIONS

This application claims priority to U.S. Provisional Application Serial No. 60/103,099 filed October 5, 1998, and U.S. Provisional Application Serial No. 60/083,798 filed May 1,1998.

### BACKGROUND

### 1. Field of the Invention

The present invention relates to a novel gene which encodes enzymes of the ω-hydroxylase complex in yeast *Candida tropicalis* strains. In particular, the invention relates to a novel gene encoding the cytochrome P450 monoxygenase of the ω-hydroxylase complex in yeast *Candida tropicalls.*

### 2. Description of the Related Art

Aliphatic dioic acids are versatile chemical intermediates useful as raw materials for the preparation of perfumes, polymers, adhesives and macrolid antibiotics. While several chemical routes to the synthesis of long-chain alpha, ω-dicarboxylic acids are available, the synthesis is not easy and most methods result in mixtures containing shorter chain lengths. As a result, extensive purification steps are necessary. While it is known that long-chain dioic acids can also be produced by microbial transformation of alkanes, fatty acids or esters thereof, chemical synthesis has remained the most commercially viable route, due to limitations with the current biological approaches.

- Several strains of yeast are known to excrete alpha, ω-dicarboxylic acids as a byproduct when cultured on alkanes or fatty acids as the carbon source. In particular, yeast belonging to the Genus *Candida,* such as *C*. *albicans, C. cloacae, C. guillermondii, C. intermedia, C. lipolytica, C. malloso, C. parapsilosis* and *C*. *zeylenoides* are known to produce such dicarboxylic acids (Agr. Biol. Chem. 35: 2033-2042 (1971)). Also, various strains of *C. tropicalis* are known to produce dicarboxylic acids ranging in chain lengths from C₁₁ through C₁₈ (Okino et al., BM Lawrence, BD Mookherjee and BJ Willis (eds), in Flavors and Fragrances: A World Perspective. Proceedings of the 10th International Conference of Essential Oils, Flavors and Fragrances, Elsevier Science Publishers BV Amsterdam (1988)), and are the basis of several patents as reviewed by Bühler and Schindler, in Aliphatic Hydrocarbons in Biotechnology, H. J. Rehm and G. Reed (eds), Vol. 169, Verlag Chemie, Weinheim (1984).

Studies of the biochemical processes by which yeasts metabolize alkanes and fatty acids have revealed three types of oxidation reactions: α-oxidation of alkanes to alcohols, ω-oxidation of fatty acids to alpha, ω-dicarboxylic acids and the degradative β-oxidation of fatty acids to CO₂ and water. The first two types of oxidations are catalyzed by microsomal enzymes while the last type takes place in the peroxisomes. In *C*. *tropicalis,* the first step in the ω-oxidation pathway is catalyzed by a membrane-bound enzyme complex (ω-hydroxylase complex) including a cytochrome P450 monooxygenase and a NADPH cytochrome reductase. This hydroxylase complex is responsible for the primary oxidation of the terminal methyl group in alkanes and fatty acids (Gilewicz et al., Can. J. Microbiol. 25:201 (1979)). The genes which encode the cytochrome P450 and NADPH reductase components of the complex have previously been identified as P450ALK and P450RED respectively, and have also been cloned and sequenced (Sanglard et al., Gene 76:121-136 (1989)). P450ALK has also been designated P450ALK1. More recently, ALK genes have been designated by the symbol *CYP* and RED genes have been designated by the symbol *CPR.* See, e.g., Nelson, Pharmacogenetics 6(1):1-42 (1996). See also Ohkuma et al., DNA and Cell Biology 14:163-173 (1995), Seghezzi et al., DNA and Cell Biology, 11:767-780 (1992) and Kargel et al., Yeast 12:333-348 (1996). For example, P450ALK is also designated *CYP52* according to the nomenclature of Nelson, *supra.* Fatty acids are ultimately formed from alkanes after two additional oxidation steps, catalyzed by alcohol oxidase (Kemp et al., Appl. Microbiol. and Biotechnol. 28: 370-374 (1988)) and aldehyde dehydrogenase. The fatty acids can be further oxidized through the same or similar pathway to the corresponding dicarboxylic acid. The ω-oxidation of fatty acids proceeds via the ω-hydroxy fatty acid and its aldehyde derivative, to the corresponding dicarboxylic acid without the requirement for CoA activation. However, both fatty acids and dicarboxylic acids can be degraded, after activation to the corresponding acyl-CoA ester through the β-oxidation pathway in the peroxisomes, leading to chain shortening. In mammalian systems, both fatty acid and dicarboxylic acid products of ω-oxidation are activated to their CoA-esters at equal rates and are substrates for both mitochondrial and peroxisomal β-oxidation (J. Biochem., 102:225-234 (1987)). In yeast, β-oxidation takes place solely in the peroxisomes (Agr.Biol. Chem. 49:1821-1828 (1985)).

The production of dicarboxylic acids by fermentation of unsaturated C₁₄-C₁₆ monocarboxylic acids using a strain of the species *C*. *tropicalis* is disclosed in U.S. Patent 4,474,882. The unsaturated dicarboxylic acids correspond to the starting materials in the number and position of the double bonds. Similar processes in which other special microorganisms are used are described in U.S. Patents 3,975,234 and 4,339,536, in British Patent Specification 1,405,026 and in German Patent Publications 21 64 626, 28 53 847, 29 37 292, 29 51 177, and 2140133.

Cytochromes P450 (P450s) are terminal monooxidases of a multicomponent enzyme system as described above. They comprise a superfamily of proteins which exist widely in nature having been isolated from a variety of organisms as described e.g., in Nelson, *supra.* These organisms include various mammals, fish, invertebrates, plants, mollusk, crustaceans, lower eukaryotes and bacteria (Nelson, *supra*). First discovered in rodent liver microsomes as a carbon-monoxide binding pigment as described, e.g., in Garfinkel, Arch. Biochem. Biophys. 77:493-509 (1958), P450s were later named based on their absorption at 450 nm in a reduced-CO coupled difference spectrum as described, e.g., in Omura et al., J. Biol. Chem. 239:2370-2378 (1964).

P450s catalyze the metabolism of a variety of endogenous and exogenous compounds (Nelson, *supra*). Endogenous compounds include steroids, prostanoids, eicosanoids, fat-soluble vitamins, fatty acids, mammalian alkaloids, leukotrines, biogenic amines and phytolexins (Nelson, *supra*). P450 metabolism involves such reactions as epoxidation, hydroxylation, deakylation, N-hydroxylation, sulfoxidation, desulfuration and reductive dehalogenation. These reactions generally make the compound more water soluble, which is conducive for excretion, and more electrophilic. These electrophilic products can have detrimental effects if they react with DNA or other cellular constituents. However, they can react through conjugation with low molecular weight hydrophilic substances resulting in glucoronidation, sulfation, acetylation, amino acid conjugation or glutathione conjugation typically leading to inactivation and elimination as described, e.g., in Klaassen et al., Toxicology, 3rd ed, Macmillan, New York, 1986.

P450s are heme thiolate proteins consisting of a heme moiety bound to a single polypeptide chain of 45,000 to 55,000 Da. The iron of the heme prosthetic group is located at the center of a protoporphyrin ring. Four ligands of the heme iron can be attributed to the porphyrin ring. The fifth ligand is a thiolate anion from a cysteinyl residue of the polypeptide. The sixth ligand is probably a hydroxyl group from an amino acid residue, or a moiety with a similar field strength such as a water molecule as described, e.g., in Goeptar et al., Critical Reviews in Toxicology 25(1):25-65 (1995).

Monooxygenation reactions catalyzed by cytochromes P450 in a eukaryotic membrane-bound system require the transfer of electrons from NADPH to P450 via NADPH-cytochrome P450 reductase (*CPR*) as described, e.g., in Taniguchi et al., Arch. Biochem. Biophys. 232:585 (1984). *CPR* is a flavoprotein of approximately 78,000 Da containing 1 mol of flavin adenine dinucleotide (FAD) and 1 mol of flavin mononucleotide (FMN) per mole of enzyme as described, e.g., in Potter et al., J. Biol. Chem. 258:6906 (1983), incorporated herein by reference. The FAD moiety of *CPR* is the site of electron entry into the enzyme, whereas FMN is the electron-donating site to P450 as described. e.g., in Vermilion et al., J. Biol. Chem. 253:8812 (1978). The overall reaction is as follows:

H⁺ + RH + NADPH + O₂ → ROH + NADP⁺ + H₂O

Binding of a substrate to the catalytic site of P450 apparently results in a conformational change initiating electron transfer from *CPR* to P450. Subsequent to the transfer of the first electron, O₂ binds to the Fe₂⁺-P450 substrate complex to form Fe₃⁺ -P450-substrate complex. This complex is then reduced by a second electron from *CPR,* or, in some cases, NADH via cytochrome b5 and NADH-cytochrome b5 reductase as described, e.g., in Guengerich et al., Arch. Biochem. Biophys. 205:365 (1980). One atom of this reactive oxygen is introduced into the substrate, while the other is reduced to water. The oxygenated substrate then dissociates, regenerating the oxidized form of the cytochrome P450 as described, e.g., in Klassen, Amdur and Doull, Casarett and Doull's Toxicology, Macmillan, New York (1986).

The P450 reaction cycle can be short-circuited in such a way that O₂ is reduced to O₂⁻ and/or H₂O₂ instead of being utilized for substrate oxygenation. This side reaction is often referred to as the "uncoupling" of cytochrome P450 as described, e.g., in Kuthen et al., Eur. J. Biochem. 126:583 (1982) and Poulos et al., FASEB J. 6:674 (1992). The formation of these oxygen radicals may lead to oxidative cell damage as described, e.g., in Mukhopadhyay, J. Biol. Chem. 269(18):13390-13397 (1994) and Ross et al., Biochem. Pharm. 49(7):979-989 (1995). It has been proposed that cytochrome b5's effect on P450 binding to the *CPR* results in a more stable complex which is less likely to become "uncoupled" as described, e.g., in Yamazaki et al., Arch. Biochem. Biophys. 325(2):174-182 (1996).

P450 families are assigned based upon protein sequence comparisons. Notwithstanding a certain amount of heterogeneity, a practical classification of P450s into families can be obtained based on deduced amino acid sequence similarity. P450s with amino acid sequence similarity of between about 40 - 80% are considered to be in the same family, with sequences of about > 55% belonging to the same subfamily. Those with sequence similarity of about < 40% are generally listed as members of different P450 gene families (Nelson, *supra).* A value of about > 97% is taken to indicate allelic variants of the same gene, unless proven otherwise based on catalytic activity, sequence divergence in non-translated regions of the gene sequence, or chromosomal mapping.

The most highly conserved region is the HR2 consensus containing the invariant cysteine residue near the carboxyl terminus which is required for heme binding as described, e.g., in Gotoh et al. J. Biochem. 93:807-817 (1983) and Motohashi et al., J. Biochem. 101:879-997 (1987). Additional consensus regions, including the central region of helix I and the transmembrane region, have also been identified, as described, e.g, in Goeptar et al., *supra* and Kalb et al., PNAS. 85:7221-7225 (1988), although the HR2 cysteine is the only invariant amino acid among P450s.

Short chain (≤C12) aliphatic dicarboxylic acids (diacids) are important industrial intermediates in the manufacture of diesters and polymers, and find application as thermoplastics, plasticizing agents, lubricants, hydraulic fluids, agricultural chemicals, pharmaceuticals, dyes, surfactants, and adhesives. The high price and limited availability of short chain diacids are due to constraints imposed by the existing chemical synthesis.

Long-chain diacids (aliphatic α, ω-dicarboxylic acids with carbon numbers of 12 or greater, hereafter also referred to as diacids) (HOOC-(CH₂)ₙ-COOH) are a versatile family of chemicals with demonstrated and potential utility in a variety of chemical products including plastics, adhesives, and fragrances. Unfortunately, the full market potential of diacids has not been realized because chemical processes produce only a limited range of these materials at a relatively high price. In addition, chemical processes for the production of diacids have a number of limitations and disadvantages. All the chemical processes are restricted to the production of diacids of specific carbon chain lengths. For example, the dodecanedioic acid process starts with butadiene. The resulting product diacids are limited to multiples of four-carbon lengths and, in practice, only dodecanedioic acid is made. The dodecanedioic process is based on nonrenewable petrochemical feedstocks. The multireaction conversion process produces unwanted byproducts, which result in yield losses, NOₓ pollution and heavy metal wastes.

Long-chain diacids offer potential advantages over shorter chain diacids, but their high selling price and limited commercial availability prevent widespread growth in many of these applications. Biocatalysis offers an innovative way to overcome these limitations with a process that produces a wide range of diacid products from renewable feedstocks. However, there is no commercially viable bioprocess to produce long chain diacids from renewable resources.

WO 91/14781 A1 discloses a process for increasing the omega-hydroxylase activity in *Candida tropicalis* comprising amplifying one or more P450 genes in said *Candida tropicalis.* Seghezzi et al., DNA AND CELL BIOLOGY, vol. 11, 1992, pages 767 to 780 disclose additional members of the cytochrome P450 multigene family of CYP52 of *Candida tropicalis.*

### SUMMARY OF THE INVENTION

An isolated nucleic acid encoding a *CYP52A5B* protein is provided having the amino acid sequence as set forth in SEQ ID NO: 101. An isolated nucleic acid is provided which includes a coding region defined by nucleotides 1142-2695 as set forth in SEQ ID NO: 91. An isolated protein is provided which includes an amino acid sequence as set forth in SEO ID NO: 101. A vector is provided which includes a nucleotide sequence encoding a *CYPS2A5B* protein having the amino acid sequence as set forth in SEQ ID NO: 101. A method of producing a *CYP52A5B* protein having an amino acid sequence as set forth in SEQ ID NO: 101 is provided which includes a) transforming a suitable host cell with a DNA sequence that encodes the protein having the amino acid sequence as set forth in SEQ ID NO: 101; and b) culturing the cell under conditions favoring the expression of the protein.

A method for increasing production of a dicarboxylic acid is provided which includes a) providing a host cell having a naturally occurring number of *CYP52ASB* genes; b) increasing, in the host cell, the number of *CYP52A58* genes which encode a *CYP52A5B* protein having the amino acid sequence as set forth in SEQ ID NO: 101; c) culturing the host cell in media containing an organic substrate which upregulates the *CYP52A5B* gene, to effect increased production of dicarboxylic acid.

A method for increasing the production of a *CYP52A5B* protein having an amino acid sequence as set forth in SEQ ID NO: 101 is provided which includes a) transforming a host cell having a naturally occurring amount of *CYP52A5B* protein with an increased copy number of *a CYP52A3B* gene that encodes the *CYP52A5B* protein having the amino acid sequence as set forth in SEQ ID NO: 101; and b) culturing the cell and thereby increasing expression of the protein compared with that of a host cell containing a naturally occurring copy number of the *CYP52A5B* gene.

### BRIEF DESCR1PTION OF THE DRAWINGS

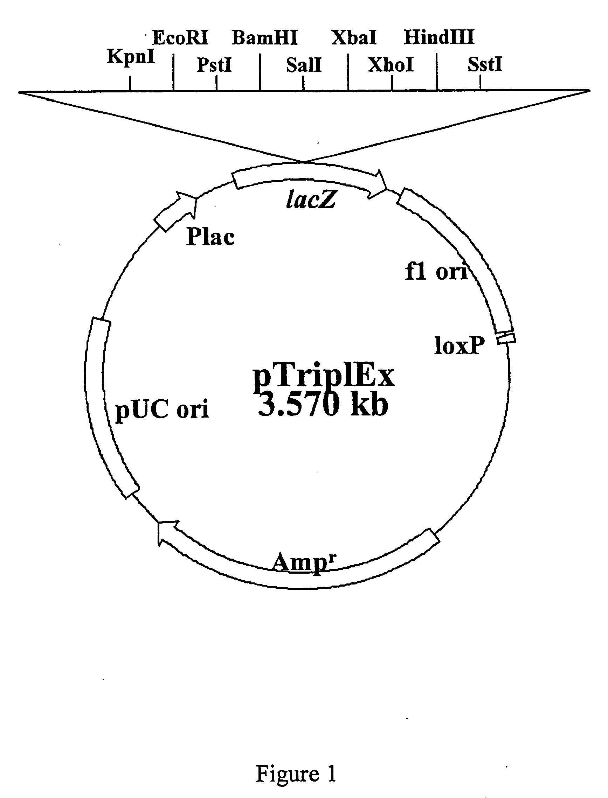

Figure 1 is a schematic representation of cloning vector pTriplEx from Clontech™ Laboratories, Inc. Selected restriction sites within the multiple cloning site are shown.

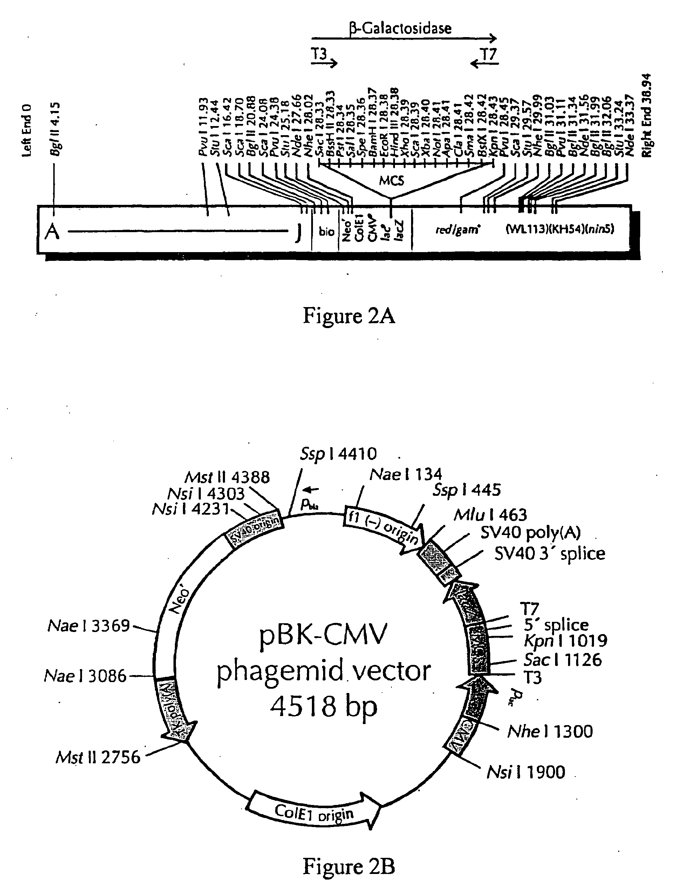

Figure 2A is a map of the ZAP Express^{™} vector.
Figure 2B is a schematic representation of cloning phagemid vector pBK-CMV.

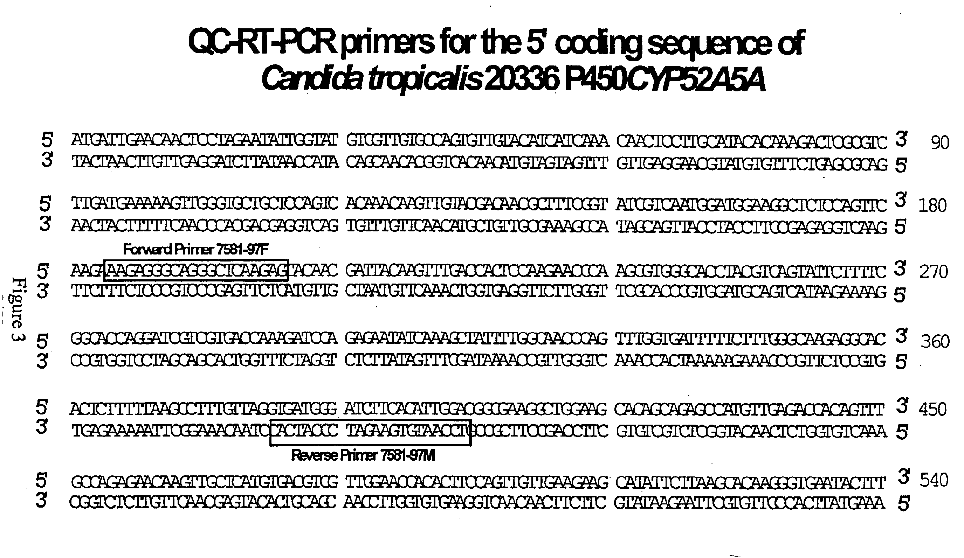

Figure 3 is a double stranded DNA sequence of a portion of the 5 prime coding - region of the *CYP52A5A* gene (SEQ ID NO: 36).

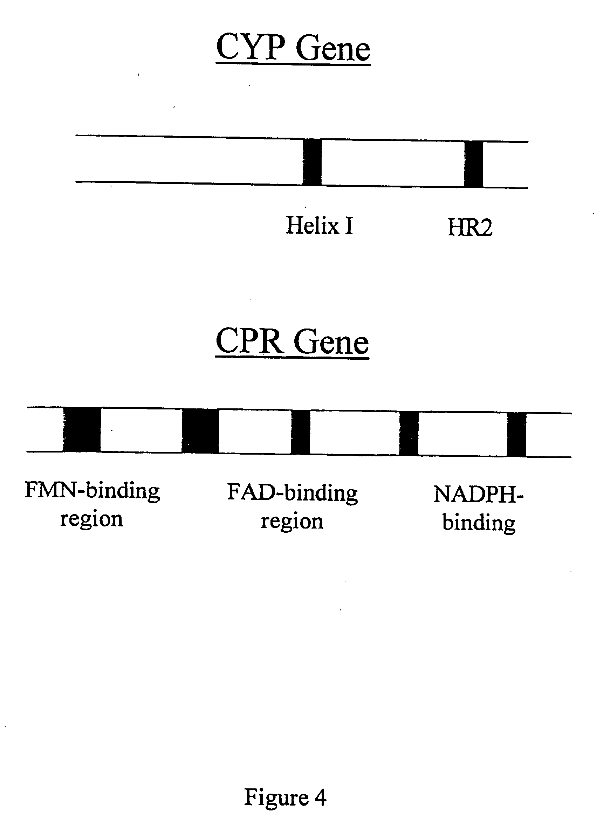

Figure 4 is a diagrammatic representation of highly conserved regions of *CYP* and *CPR* gene protein sequences. Helix I represents the putative substrate binding site and HR2 represents the heme binding region. The FMN, FAD and NADPH binding regions are indicated below the *CPR* gene.

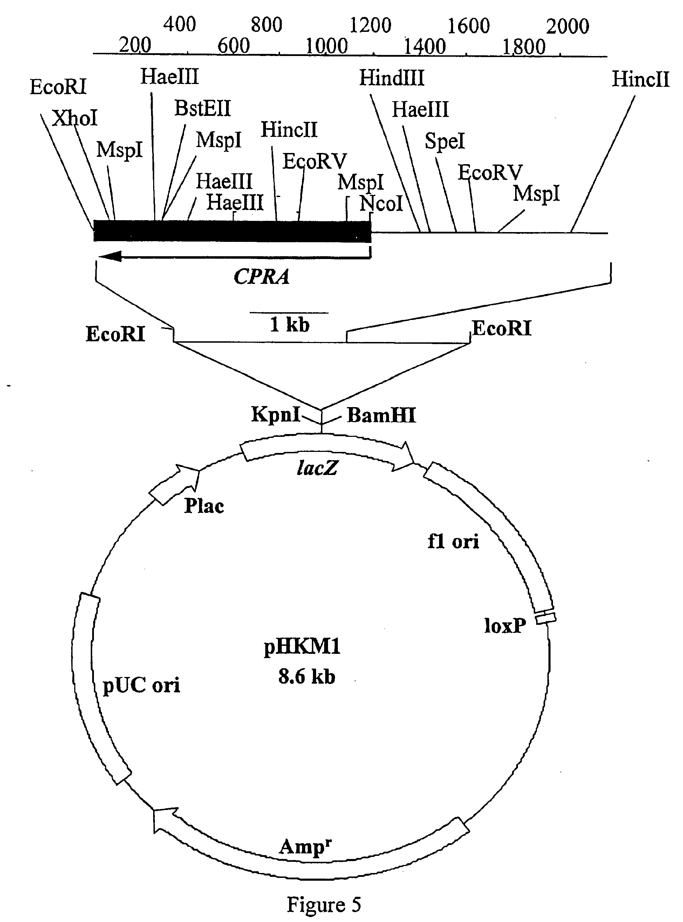

Figure 5 is a diagrammatic representation of the plasmid pHKM1 containing the truncated *CPRA* gene present in the pTriplEx vector. A detailed restriction map of only the sequenced region is shown at the top. The bar indicates the open reading frame. The direction of transcription is indicated by an arrow under the open reading frame.

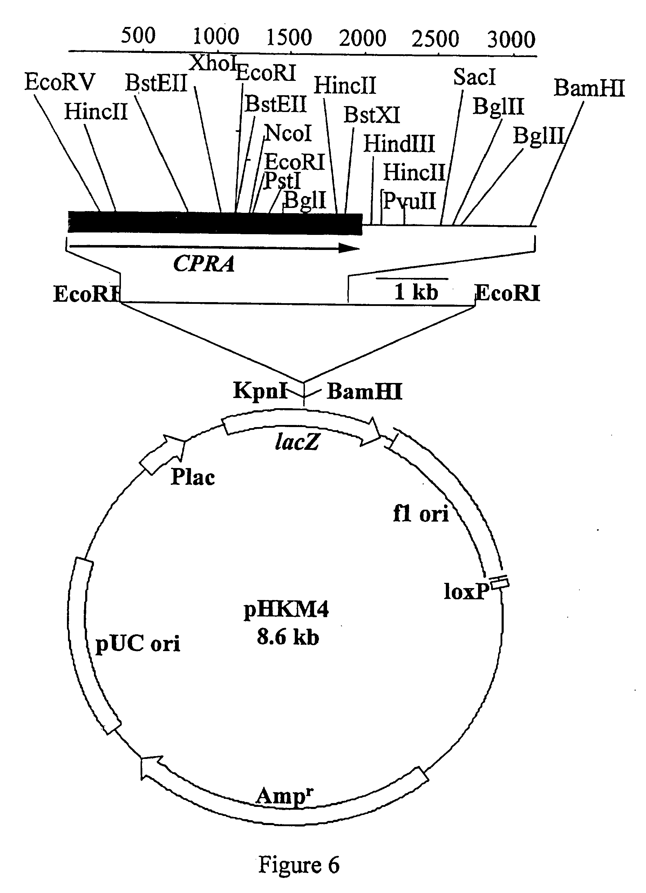

Figure 6 is a diagrammatic representation of the plasmid pHKM4 containing the truncated *CPRA* gene present in the pTriplEx vector. A detailed restriction map of only the sequenced region is shown at the top. The bar indicates the open reading frame. The direction of transcription is indicated by an arrow under the open reading frame.

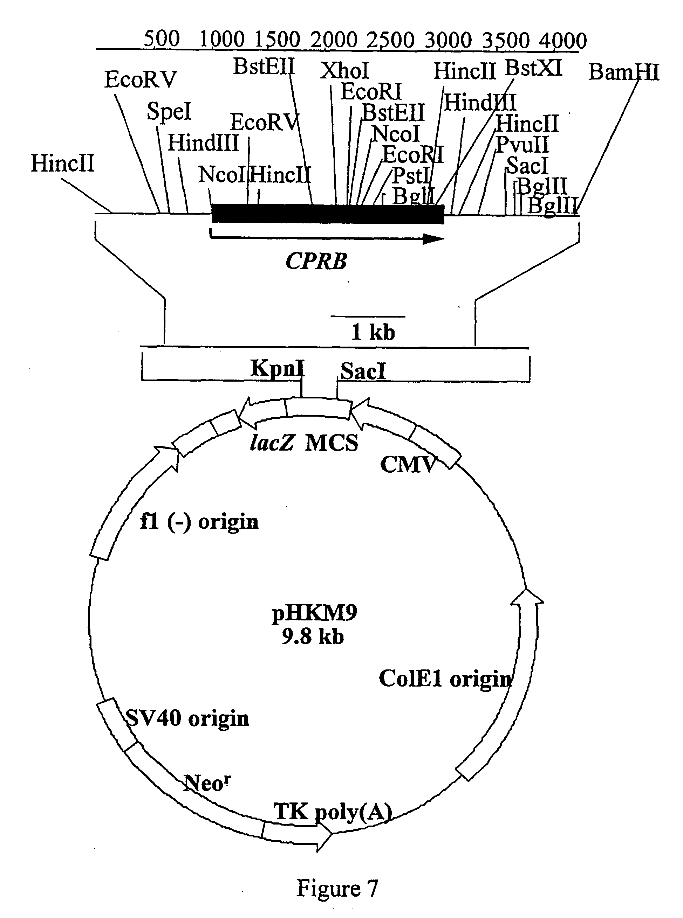

Figure 7 is a diagrammatic representation of the plasmid pHKM9 containing the *CPRB* gene (SEQ ID NO: 82) present in the pBK-CMV vector. A detailed restriction map of only the sequenced region is shown at the top. The bar indicates the open reading frame. The directions of transcription is indicated by an arrow under the open reading frame.

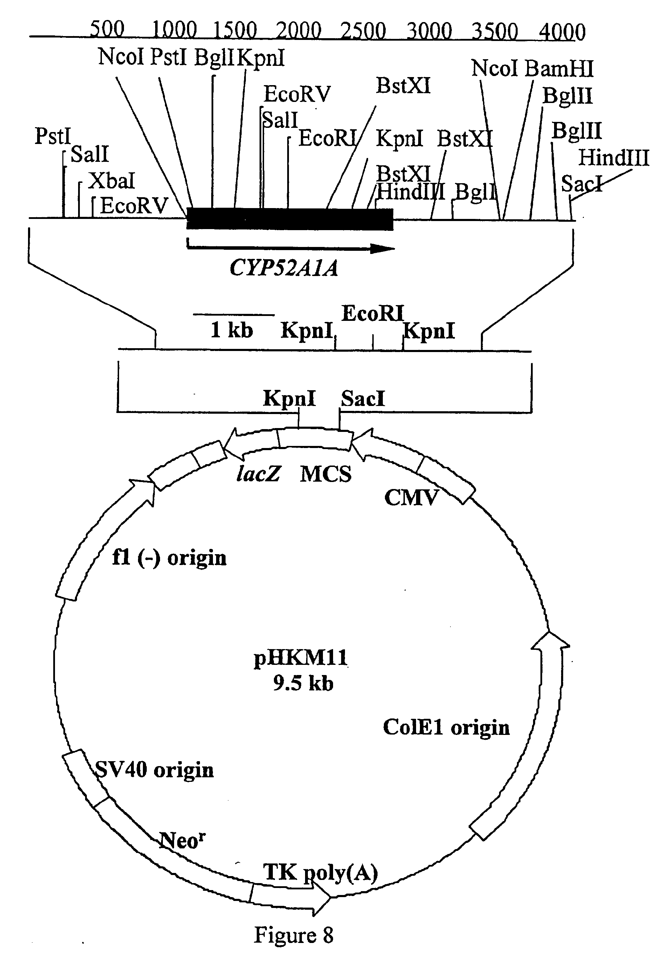

Figure 8 is a diagrammatic representation of the plasmid pHKM1 containing the *CYP52A1A* gene (SEQ ID NO: 85) present in the pBK-CMV vector. A detailed restriction map of only the sequenced region is shown at the top. The bar indicates the open reading frame. The direction of transcription is indicated by an arrow under the open reading frame.

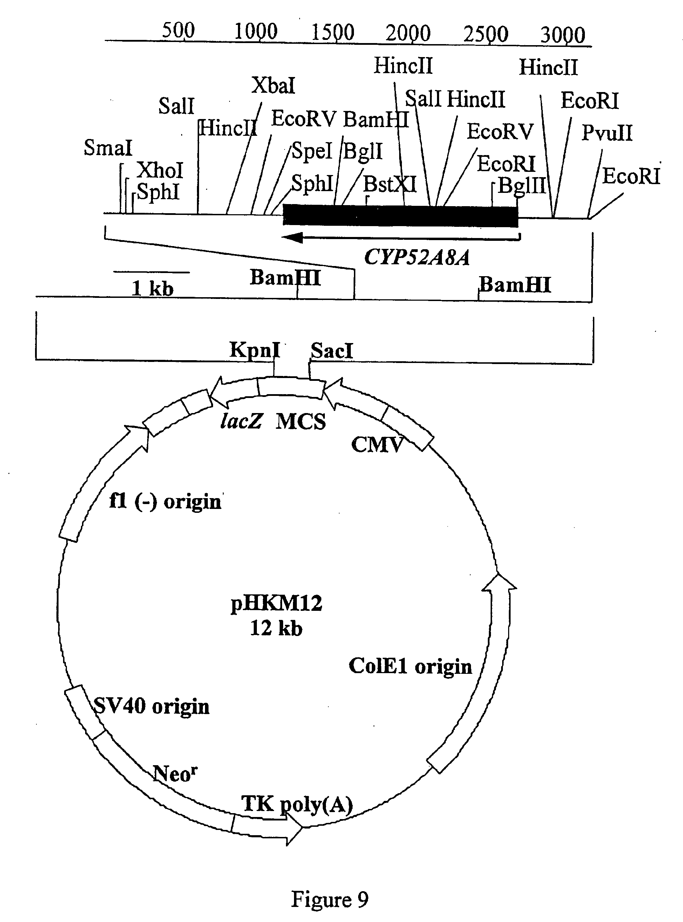

Figure 9 is a diagrammatic representation of the plasmid pHKM12 containing the *CYP52A8A* gene (SEQ ID NO: 92) present in the pBK-CMV vector. A detailed restriction map of only the sequenced region is shown at the top. The bar indicates the open reading frame. The direction of transcription is indicated by an arrow under the open reading frame.

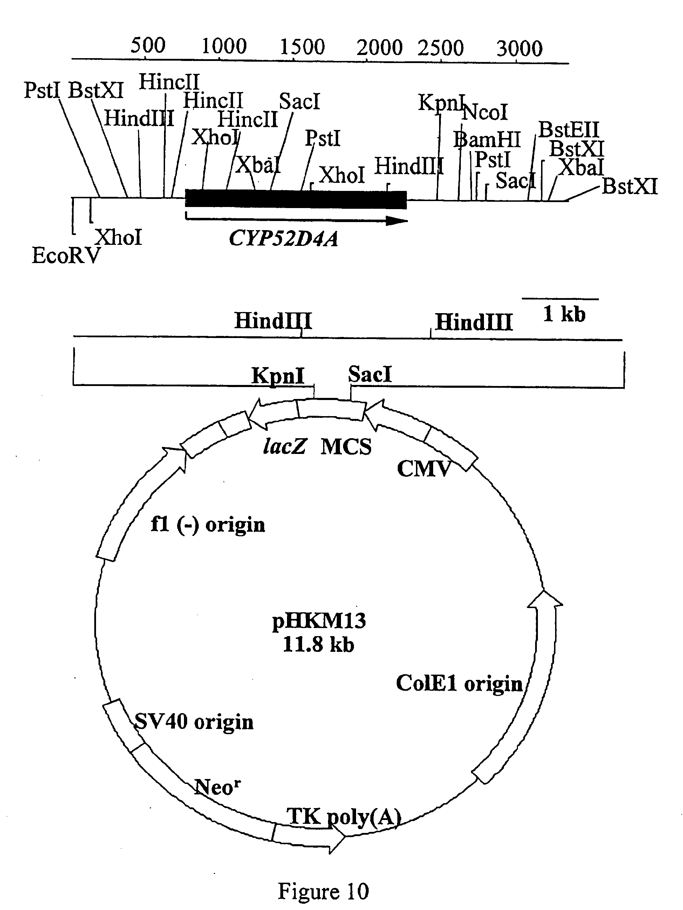

Figure 10 is a diagrammatic representation of the plasmid pHKM213 containing the *CYP52D4A* gene (SEQ ID NO: 94) present in the pBK-CMV vector. A detailed restriction map of only the sequenced region is shown at the top. The bar indicates the open reading frame. The direction of transcription is indicated by an arrow under the open reading frame.

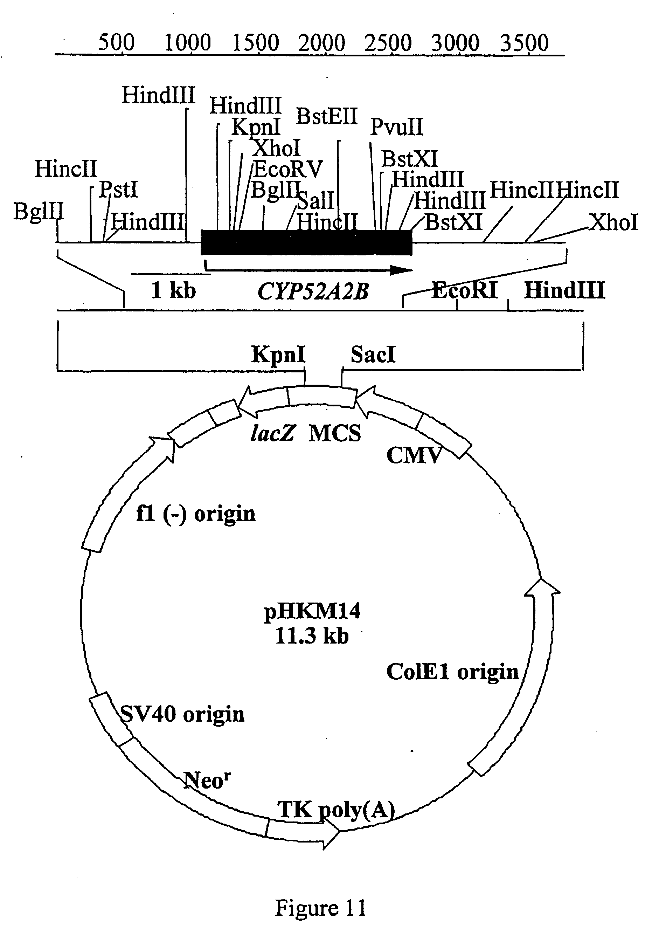

Figure 11 is a diagrammatic representation of the plasmid pHKM14 containing the *CYP52A2B* gene (SEQ ID NO: 87) present in the pBK-CMV vector. A detailed restriction map of only the sequenced region is shown at the top. The bar indicates the open reading frame. The direction of transcription is indicated by an arrow under the open reading frame.

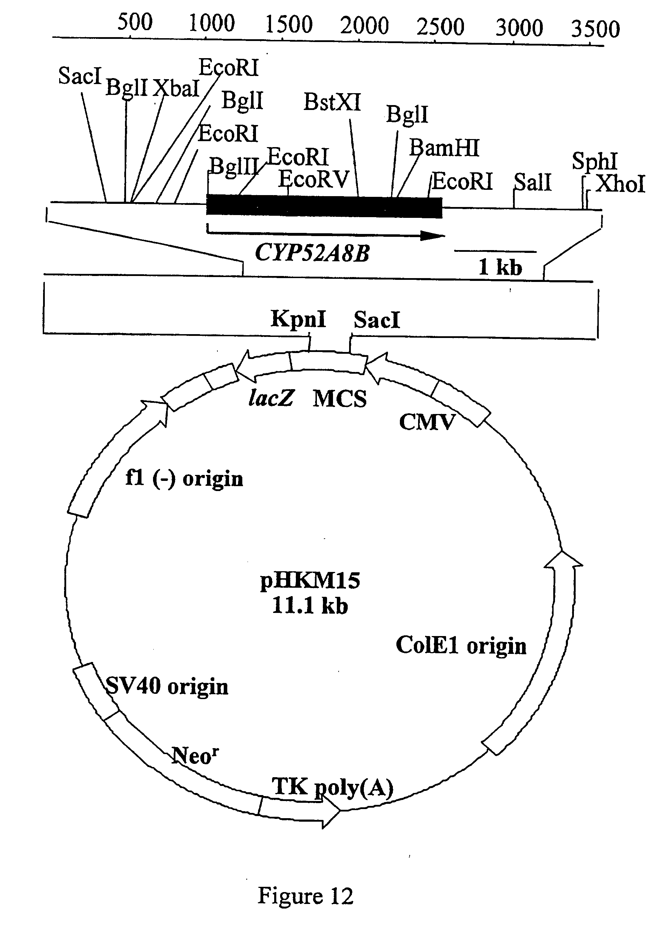

Figure 12 is a diagrammatic representation of the plasmid pHKM15 containing the *CYP52A8B* gene (SEQ ID NO: 93) present in the pBK-CMV vector. A detailed restriction map of only the sequenced region is shown at the top. The bar indicates the open reading frame. The direction of transcription is indicated by an arrow under the open reading frame.

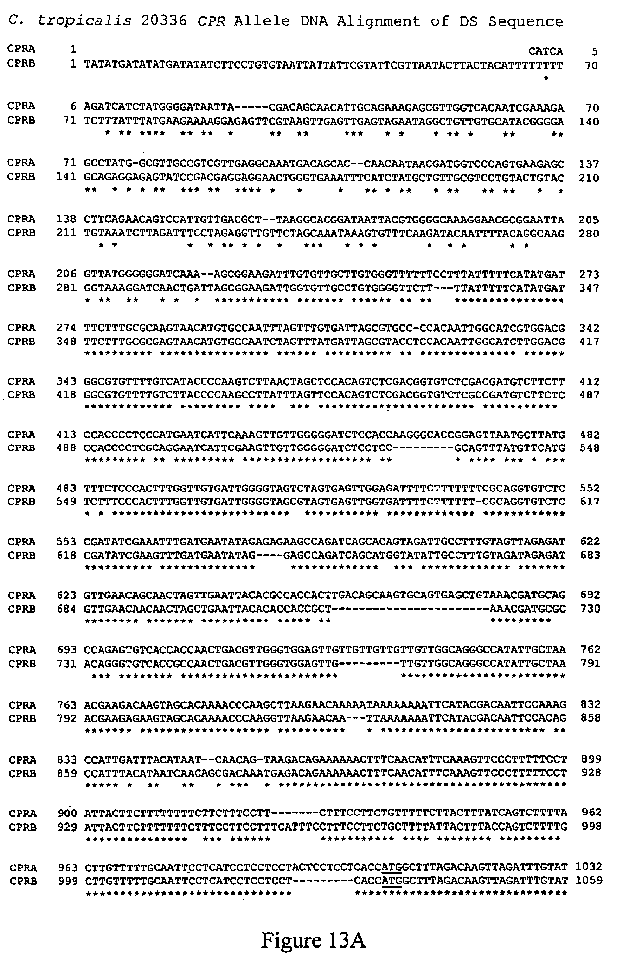

Figures 13A-13D show the complete DNA sequences including regulatory and coding regions for the *CPRA* gene (SEQ ID NO: 81) and *CPRB* gene (SEQ ID NO: 82) from C. *tropicalis* ATCC 20336. Figures 13A-13D show regulatory and coding region alignment of these sequences. Asterisks indicate conserved nucleotides. Bold indicates protein coding nucleotides; the start and stop codons are underlined.

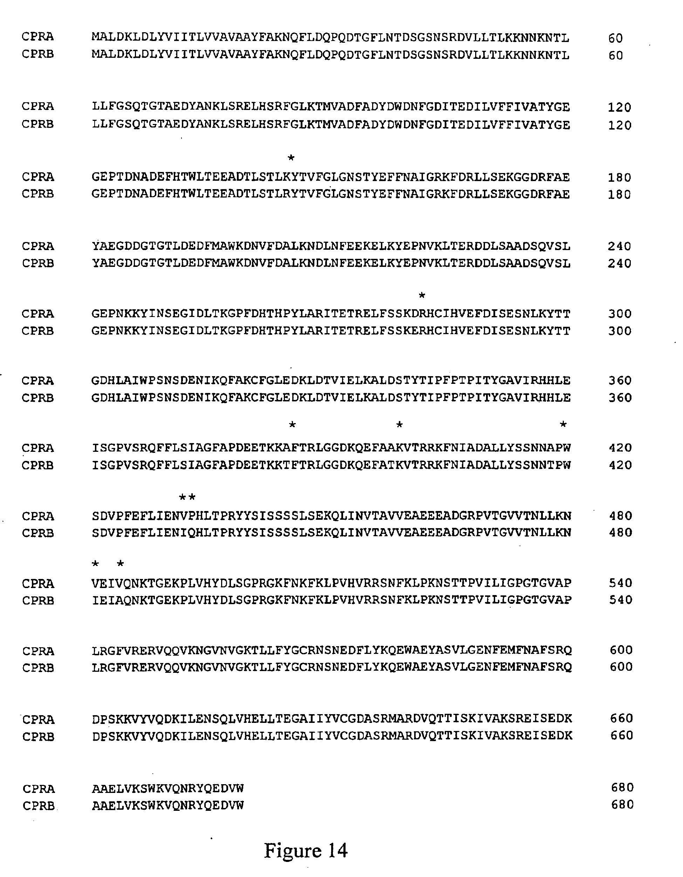

Figure 14 shows the amino acid sequence of the *CPRA* (SEQ ID NO: 83) and *CPRB* (SEQ ID NO: 84) proteins from *C*. *tropicalis* ATCC 20336 and alignment of these amino acid sequences. Asterisks indicate residues which are not conserved.

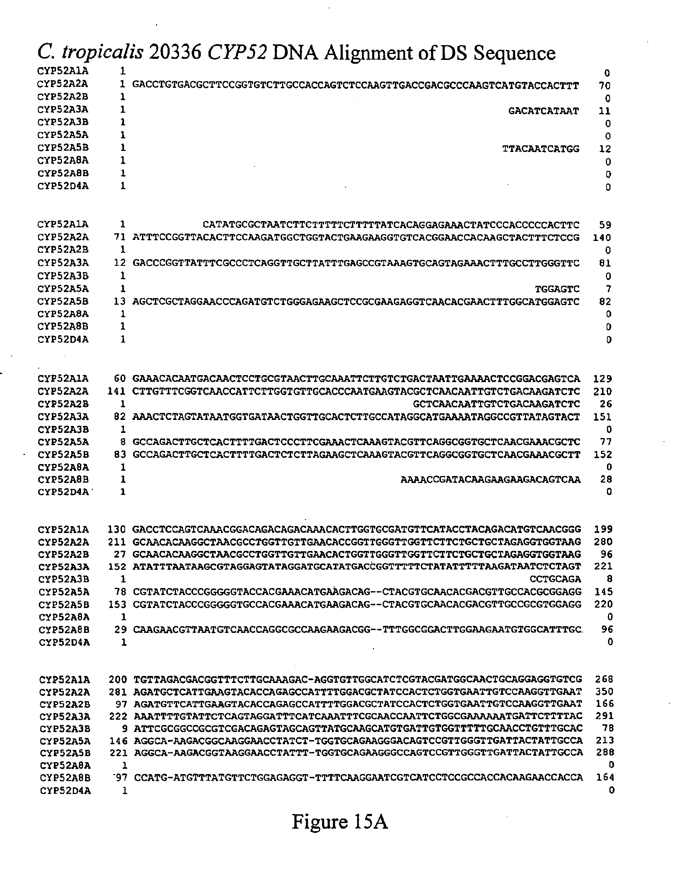

Figures 15A-15M show the complete DNA sequences including regulatory and coding regions for the following genes from *C*. *tropicalis* ATCC *20366:CYP52A1A* (SEQ ID NO: 85), *CYP52A2A* (SEQ ID NO: 86), *CYP52A2B* (SEQ ID NO: 87), *CYP52A3A* (SEQ ID NO: 88), *CYP52A3B* (SEQ ID NO: 89), *CYP52A5A* (SEQ ID NO. 90), *CYP52A5B* (SEQ ID NO: 91), *CYP52A8A* (SEQ ID NO: 92), *CYP52A8B* (SEQ ID NO: 93), and *CYP52D4A* (SEQ ID NO: 94). Figures 15A-15M show regulatory and coding region alignment of these sequences. Asterisks indicate conserved nucleotides. Bold indicates protein coding nucleotides; the start and stop codons are underlined.

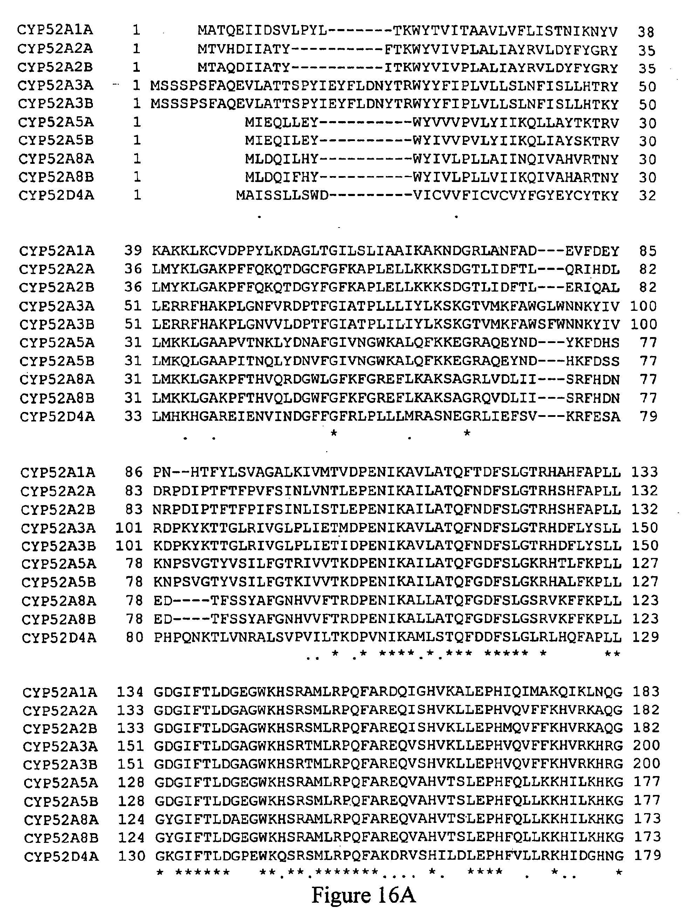

Figures 16A-16C show the amino acid sequences encoding the *CYP52A1A* (SEQ ID NO: 95), *CYP52A2A* (SEQ ID NO: 96), *CYP52A2B* (SEQ ID NO: 97), *CYP52A3A* (SEQ ID NO: 98), *CYP52A3B* (SEQ ID NO: 99), *CYP52A5A* (SEQ ID NO: 100), *CYP52A5B* (SEQ ID NO: 101), *CYP52A8A* (SEQ ID NO: 102), *CYP52A8B* (SEQ ID NO: 103) and *CYP52D4A* (SEQ ID NO. 104) proteins from *C*. *tropicalis* ATCC 20336. Asterisks indicate identical residues and dots indicate conserved residues.

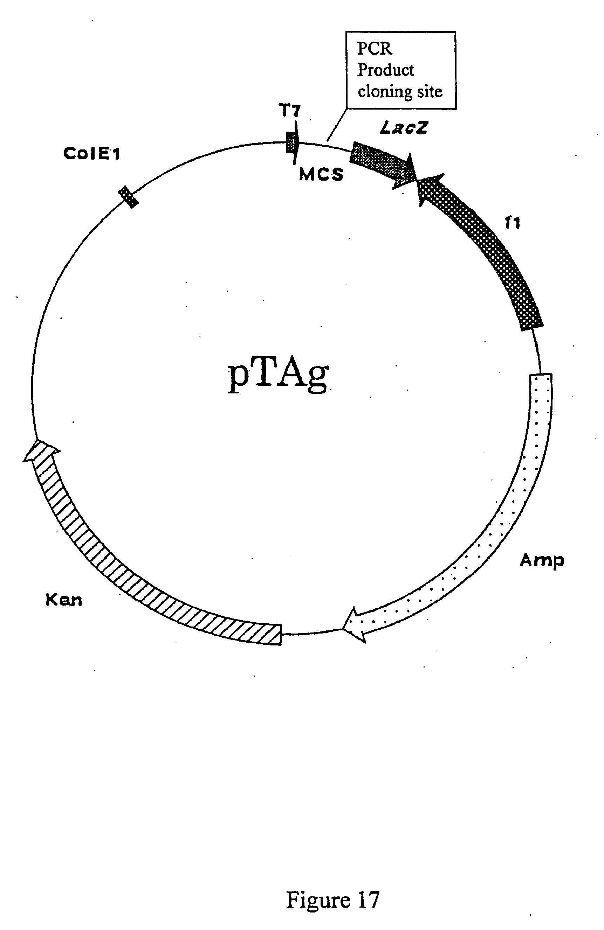

Figure 17 is a diagrammatic representation of the pTAg PCR product cloning vector (commercially available from R&D Systems, Minneapolis, MN).

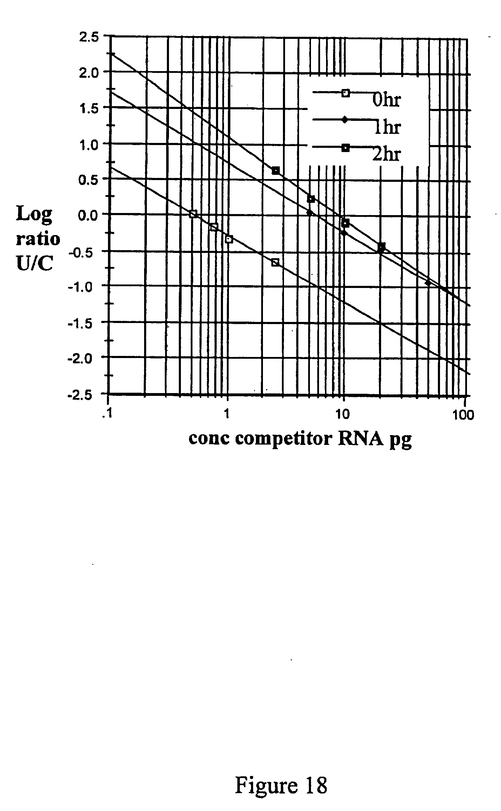

Figure 18 is a plot of the log ratio (U/C) of unknown target DNA product to competitor DNA product versus the concentration of competitor mRNA. The plot is used to calculate the target messenger RNA concentration in a quantitative competitive reverse transcription polymerase chain reaction (QC-RT-PCR).

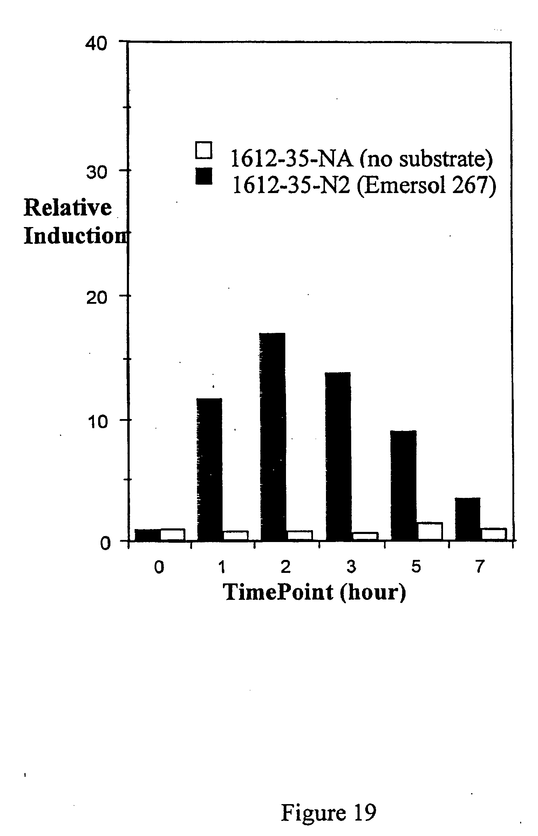

Figure 19 is a graph showing the relative induction of *C*. *tropicalis* ATCC 20962 *CYP52A5A* (SEQ ID NO: 90) by the addition of the fatty acid substrate Emersol® 267 to the growth medium.

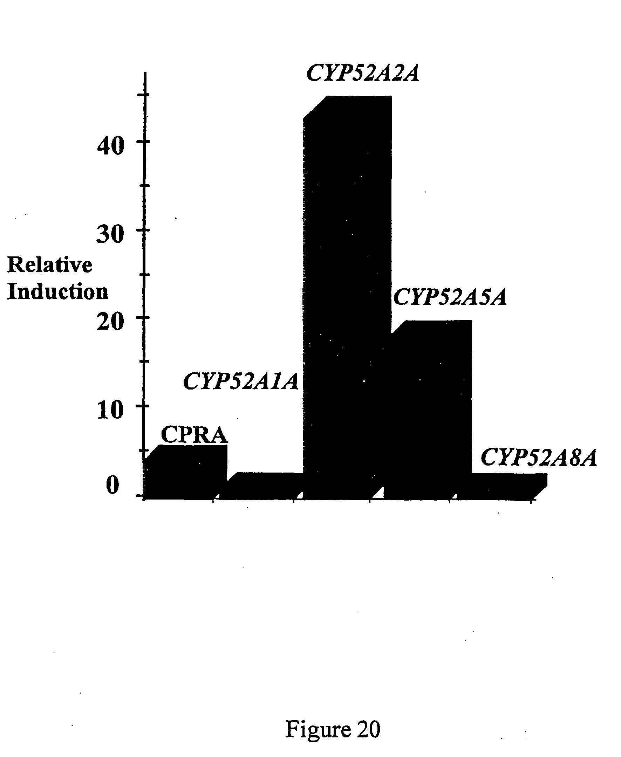

Figure 20 is a graph showing the induction of *C. tropicalis* ATCC 20962 *CYP52* and *CPR* genes by Emersol® 267. P450 genes *CYP52A3A* (SEQ ID NO: 88), *CYP52A3B* (SEQ ID NO: 89), and *CYP52D4A* (SEQ ID NO: 94) are expressed at levels below the detection level of the QC-RT-PCR assay.

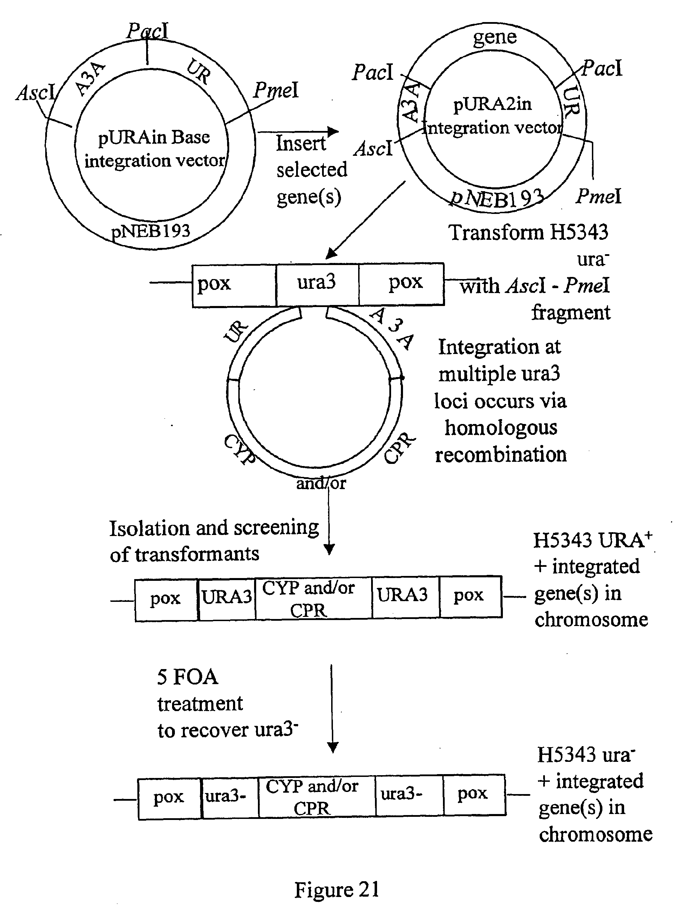

Figure 21 is a scheme to integrate selected genes into the genome of *Candida tropicalis* strains and recovery of *URA3A* selectable marker.

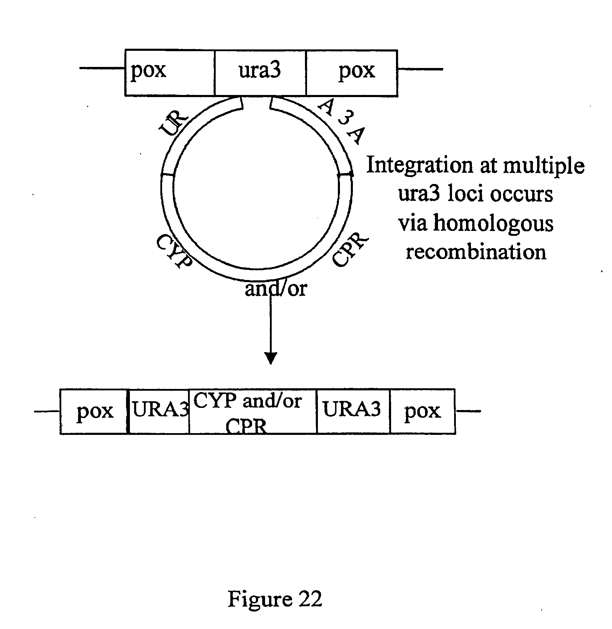

Figure 22 is a schematic representation of the transformation of *C*. *tropicalis* H5343 ura3⁻ with *CYP* and/or *CPR* genes. Only one *URA3* locus needs to be functional. There are a total of 6 possible *ura3* targets (*5ura*3A loci-2 pox4 disruptions, 2 pox 5 disruptions, 1 *ura3A* locus; and 1 *ura3B* locus).

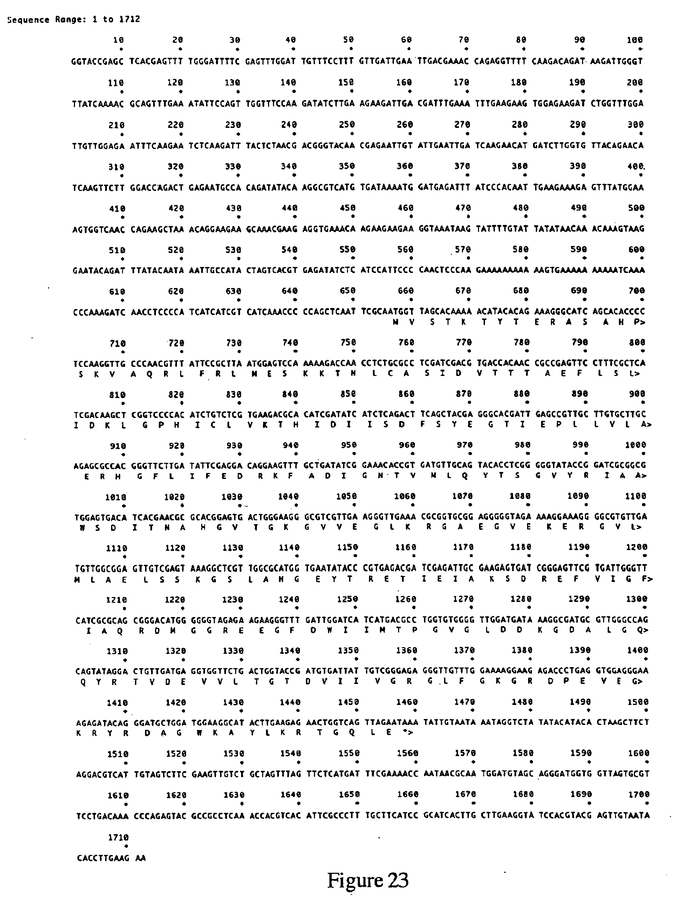

Figure 23 is the complete DNA sequence (SEQ ID NO: 105) encoding *URA3A* from *C*. *tropicalis* ATCC 20336 and the amino acid sequence of the encoded protein (SEQ ID NO:106).

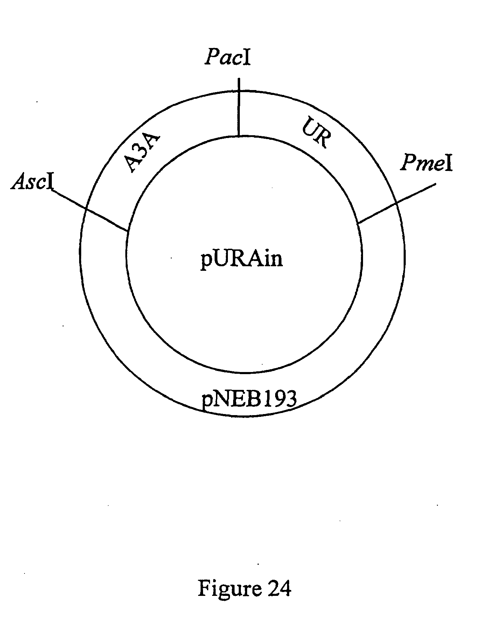

Figure 24 is a schematic representation of the plasmid pURAin, the base vector for integrating selected genes into the genome of *C*. *tropicalis.* The detailed construction of pURAin is described in the text.

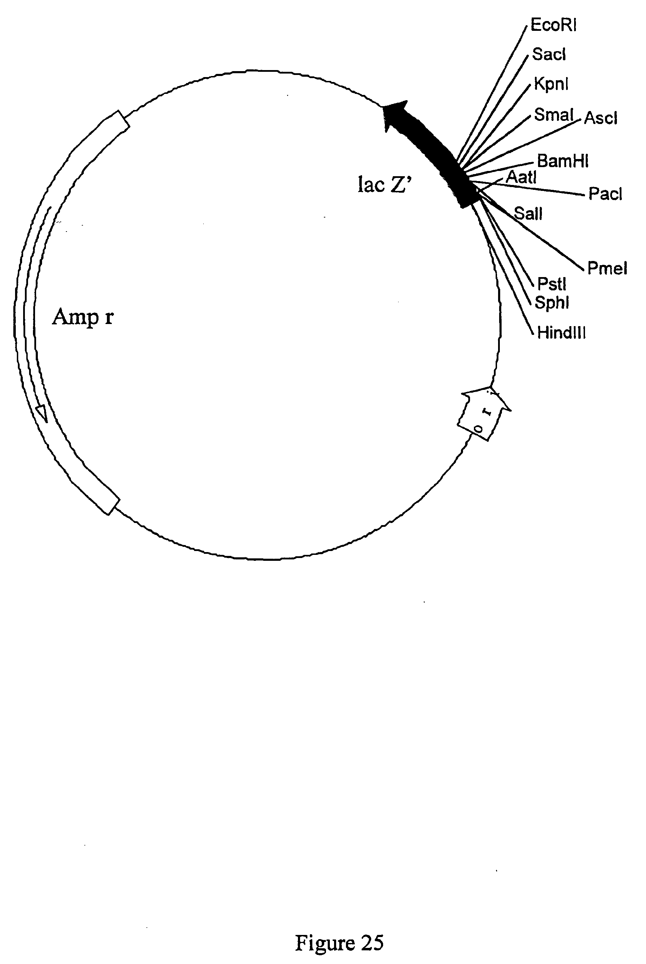

Figure 25 is a schematic representation of the plasmid pNEB193 cloning vector (commercially available from New England Biolabs, Beverly, MA).

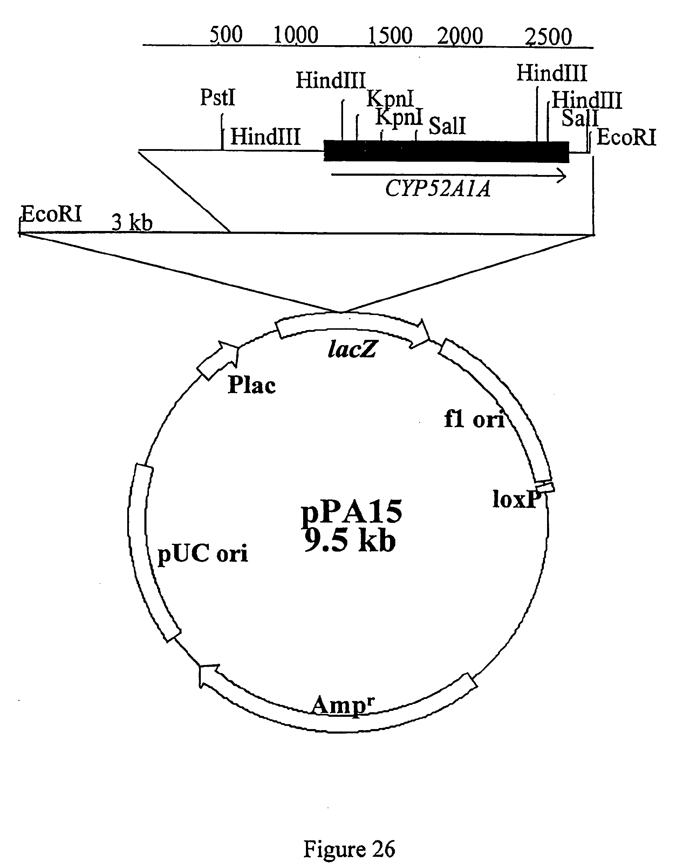

Figure 26 is a diagrammatic representation of the plasmid pPA15 containing the truncated *CYP52A2A* gene present in the pTriplEx vector. A detailed restriction map of only the sequenced region is shown at the top. The bar indicates the open reading frame. The direction of transcription is indicated by an arrow under the open reading frame.

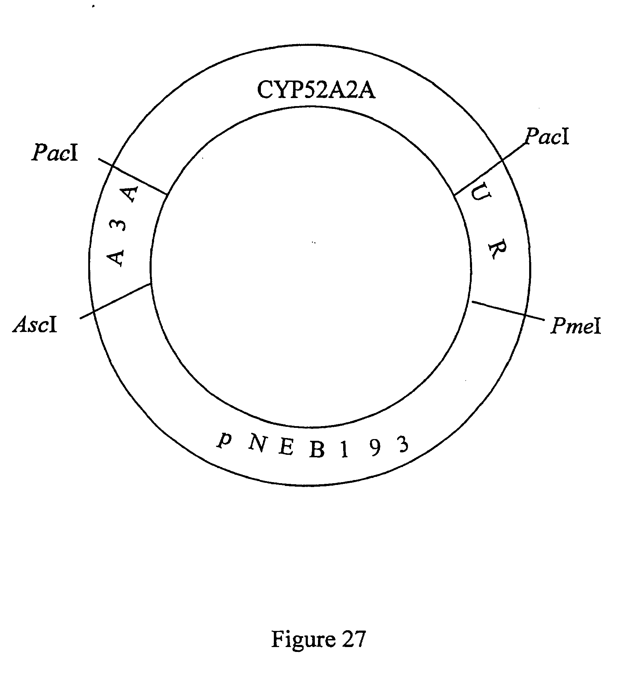

Figure 27 is a schematic representation of pURA2in, the base vector is constructed in pNEB 193 which contains the 8 bp recognition sequences for *Asc I, Pac I* and *Pme I. URA3A* (SEQ ID NO: 105) and *CYP52A2A* (SEQ ID NO: 86) do not contain these 8 bp recognition sites. *URA3A* is inverted so that the transforming fragment will attempt to recircularize prior to integration. An *Asc I*/*Pme I* fragment was used to transform H5343 ura⁻.

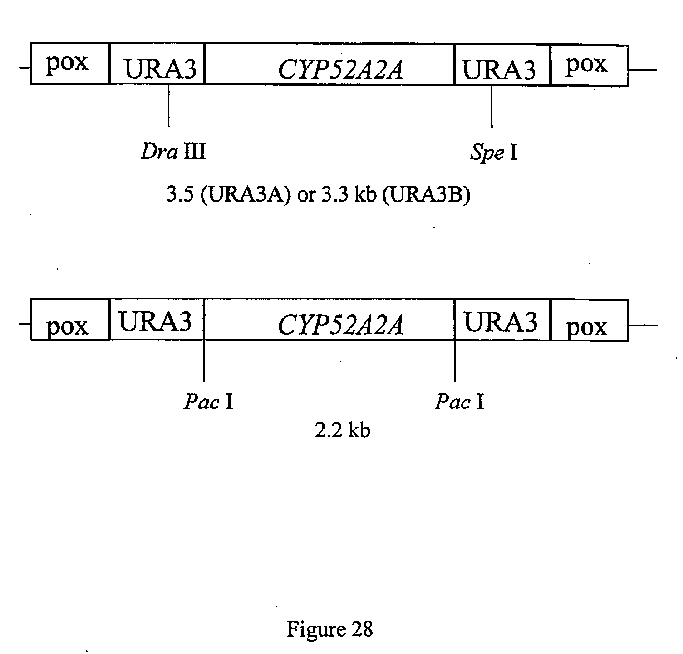

Figure 28 shows a scheme to detect integration of *CYP52A2A* gene (SEQ ID NO: 86) into the genome of H5343 ura⁻. In all cases, hybridization band intensity could reflect the number of integrations.

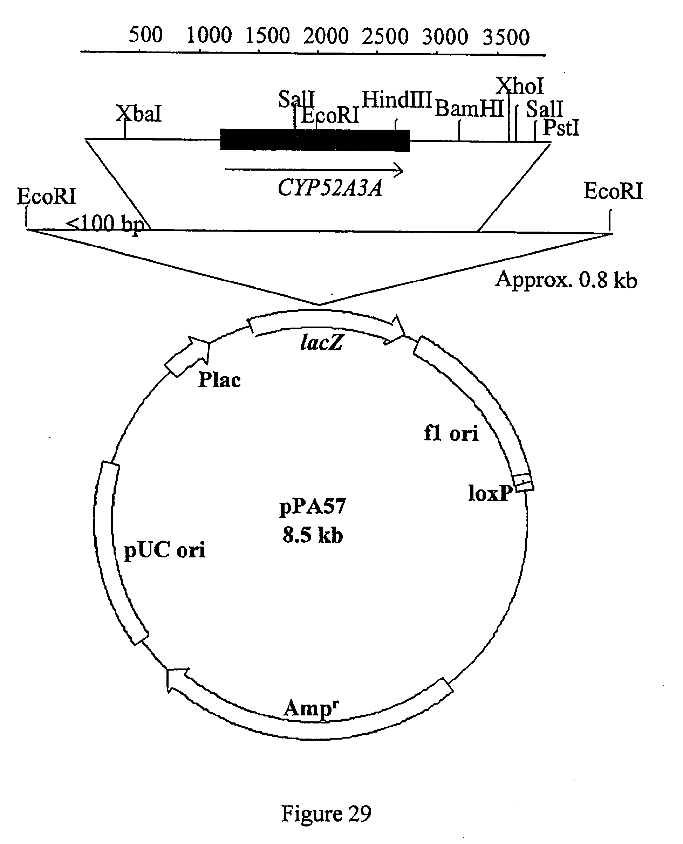

Figure 29 is a diagrammatic representation of the plasmid pPA57 containing the truncated *CYP52A3A* gene present in the pTriplEx vector. A detailed restriction map of only the sequenced region is shown at the top. The bar indicates the open reading frame. The direction of transcription is indicated by an arrow under the open reading frame.

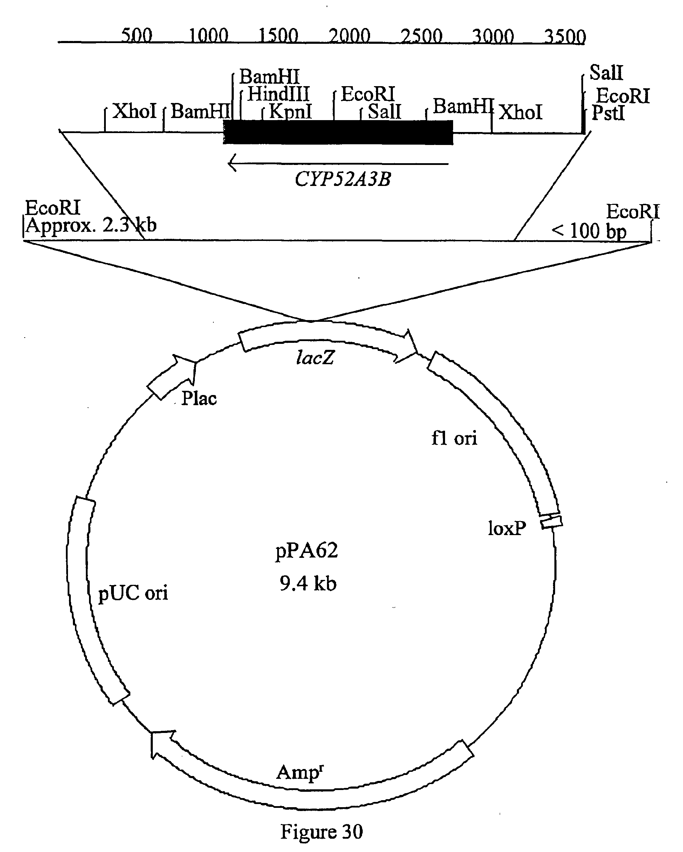

Figure 30 is a diagrammatic representation of the plasmid pPA62 containing the truncated *CYP52A3B* gene present in the pTriplEx vector. A detailed restriction map of only the sequenced region is shown at the top. The bar indicates the open reading frame. The direction of transcription is indicated by an arrow under the open reading frame.

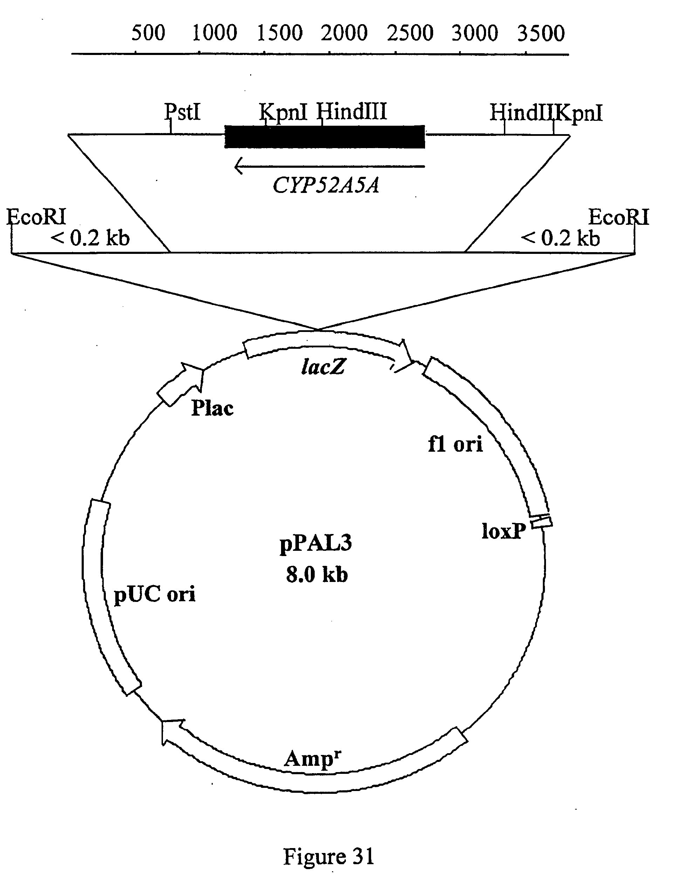

Figure 31 is a diagrammatic representation of the plasmid pPAL3 containing the truncated *CYP52A5A* gene present in the pTriplEx vector. A detailed restriction map of only the sequenced region is shown at the top. The bar indicates the open reading frame. The direction of transcription is indicated by an arrow under the open reading frame.

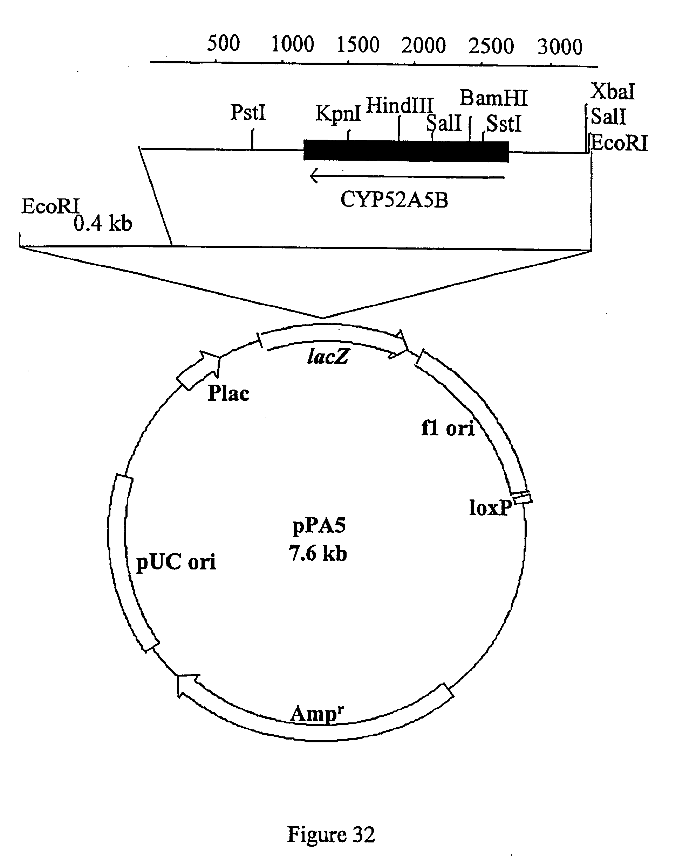

Figure 32 is a diagrammatic representation of the plasmid pPA5 containing the truncated *CYP52A5A* gene present in the pTriplEx vector. A detailed restriction map of only the sequenced region is shown at the top. The bar indicates the open reading frame. The direction of transcription is indicated by an arrow under the open reading frame.

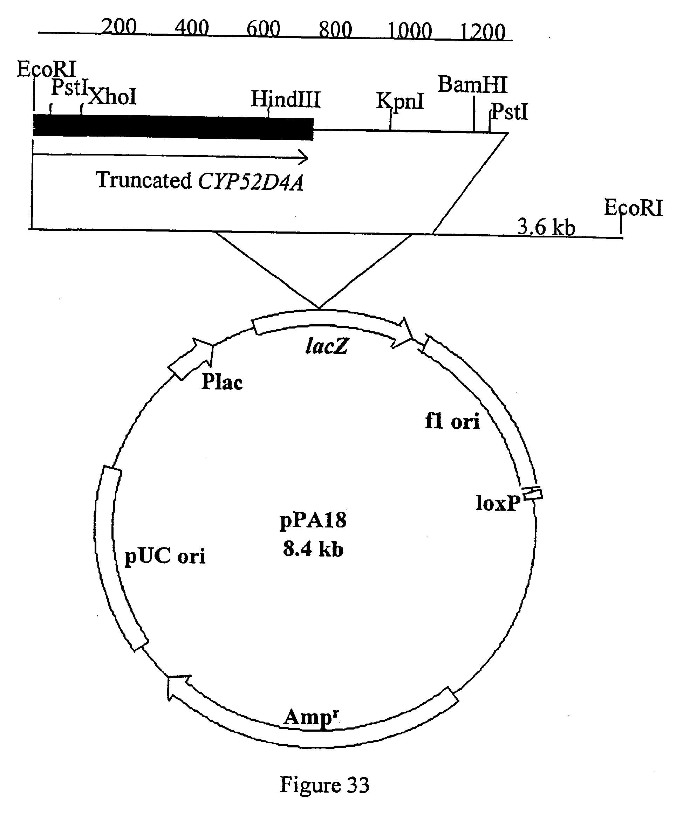

Figure 33 is a diagrammatic representation of the plasmid pPA18 containing the truncated *CYP52D4A* gene present in the pTriplEx vector. A detailed restriction map of only the sequenced region is shown at the top. The bar indicates the open reading frame. The direction of transcription is indicated by an arrow under the open reading frame.

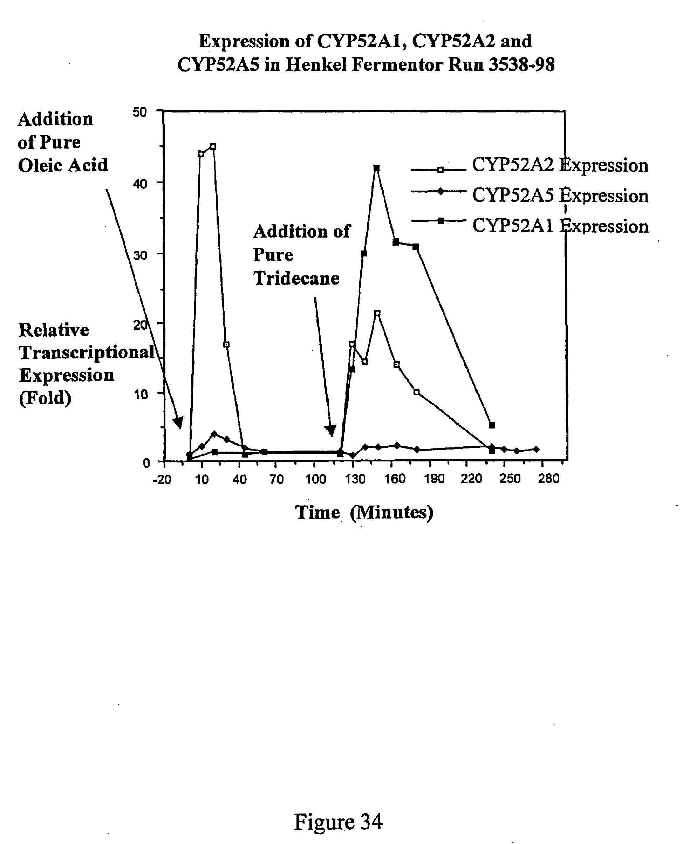

Figure 34 is a graph showing the expression of *CYP52A1* (SEQ ID NO: 85), *CYP52A2* (SEQ ID NO: 86) and *CYP52A5* genes (SEQ ID NOS: 90 and 91) from *C*. *tropicalis* 20962 in a fermentor run upon the addition of amounts of the substrate oleic acid or tridecane in a spiking experiment.

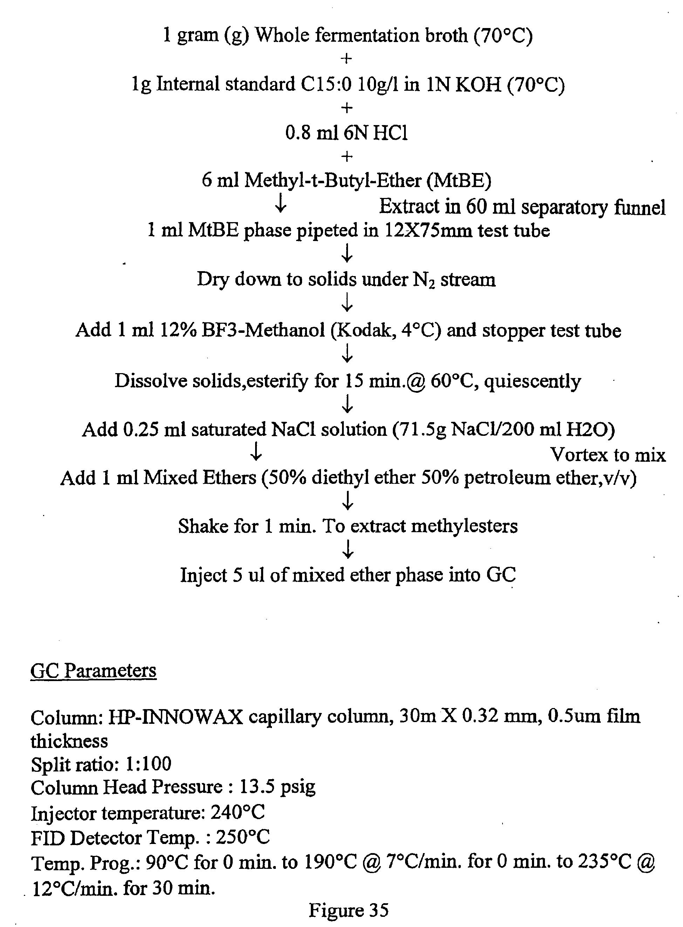

Figure 35 depicts a scheme used for the extraction and analysis of diacids and monoacids from fermentation broths.

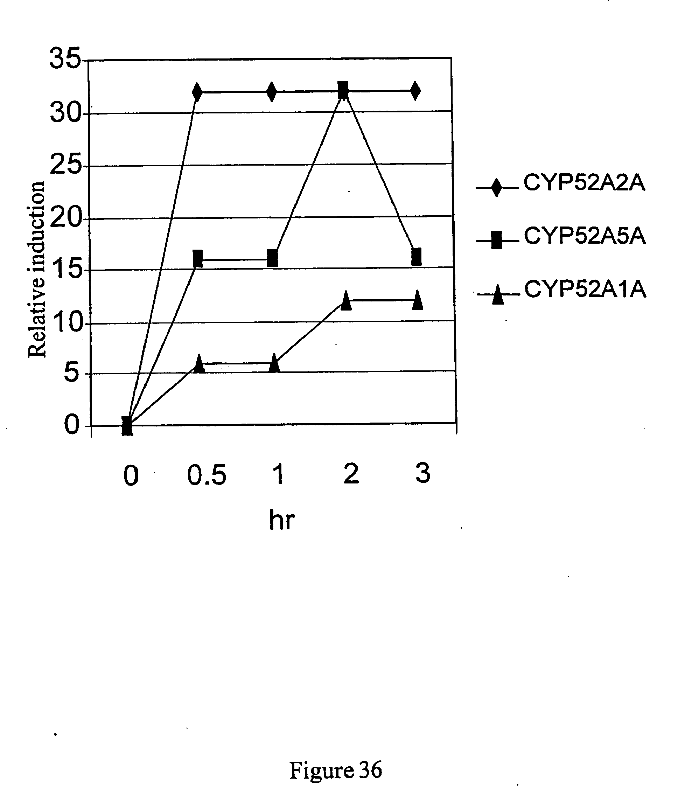

Figure 36 is a graph showing the induction of expression of *CYP52A1A*, *CYP52A2A* and *CYP52A5A* in a fermentor run upon addition of the substrate octadecane. No induction of *CYP52A3A* or *CYP52A3B* was observed under these conditions.

### DESCRIPTION OF THE PREFERRED EMBODIMENTS

Diacid productivity is improved according to the present invention by selectively increasing enzymes which are known to be important to the oxidation of organic substrates such a as fatty acids composing the desired feed. According to the present invention, a *CYP* gene of *C*. *tropicalis* has been identified and characterized that relates to participation in the ω-hydroxylase complex catalyzing the first step in the ω-oxidation pathway. In addition, a novel quantitative competitive reverse transcription polymerase chain reaction (QC-RT-PCR) assay is used to measure gene expression in the fermentor under conditions of induction by one or more organic substrates as defined herein. Based upon QC-RT-PCR results, three *CYP* genes, *CYP32A1*, *CYP52A2* and *CYP52A5*, have been identified as being of greater importance for the ω-oxidation of long chain fatty acids. Amplification of the *CPR* gene copy - number improves productivity.. The QC-RT-PCR assay indicates that both *CYP* and *CPR* genes appear to be under tight regulatory control.

Disclosed is a method for discriminating members of a gene family by quantifying the amount of target mRNA in a sample is provided which includes a) providing an organism containing a target gene; b) culturing the organism with an organic substrate which causes upregulation in the activity of the target gene; c) obtaining a sample of total RNA from the organism at a first point in time; d) combining at least a portion of the sample of the total RNA with a known amount of competitor RNA to form an RNA mixture, wherein the competitor RNA is substantially similar to the target mRNA but has a lesser number of nucleotides compared to the target mRNA; e) adding reverse transcriptase to the RNA mixture in a quantity sufficient to form corresponding target DNA and competitor DNA; (f) conducting a polymerase chain reaction in the presence of at least one primer specific for at least one substantially non-homologous region of the target DNA within the gene family, the primer also specific for the competitor DNA; g) repeating steps (c-f) using increasing amounts of the competitor RNA while maintaining a substantially constant amount of target RNA; h) determining the point at which the amount of target DNA is substantially equal to the amount of competitor DNA; i) quantifying the results by comparing the ratio of the concentration of unknown target to the known concentration of competitor, and j) obtaining a sample of total RNA from the organism at another point in time and repeating steps (d-i).

In addition, modification of existing promoters and/or the isolation of alternative promoters provides increased expression of *CYP* and *CPR* genes. Strong promoters are obtained from at least four sources: random or specific modifications of the *CYP52A2* promoter, *CYP52A5* promoter, *CYP52A1* promoter, the selection of a strong promoter from available *Candida* β-oxidation genes such as *POX4* and *POX5,* or screening to select another suitable *Candida* promoter.

Promoter strength can be directly measured using QT-RT-PCR to measure *CYP* and *CPR* gene expression in *Candida* cells isolated from fermentors. Enzymatic assays and antibodies specific for *CYP* and *CPR* proteins are used to verify that increased promoter strength is reflected by increased synthesis of the corresponding enzymes. Once a suitable promoter is identified, it is fused to the selected *CYP* and *CPR* genes and introduced into *Candida* for construction of a new improved production strain. It is contemplated that the coding region of the *CYP* and *CPR* genes can be fused to suitable promoters or other regulatory sequences which are well known to those skilled in the art.

Studies on *C*. *tropicalis* ATCC 20336 have identified six unique *CYP* genes and four potential alleles. QC-RT-PCR analyses of cells isolated during the course of the fermentation bioconversions indicate that at least three of the *CYP* genes are induced by fatty acids and at least two of the *CYP* genes are induced by alkanes. See Figure 34. Two of the *CYP* genes are highly induced indicating participation in the ω-hydroxylase complex which catalyzes the rate limiting step in the oxidation of fatty acids to the corresponding diacids.

The biochemical characterizations of each P450 enzyme herein is used to tailor the *C*. *tropicalis* host for optimal diacid productivity and is used to select P450 enzymes to be amplified based upon the fatty acid content of the feedstream. *CYP* gene(s) encoding P450 enzymes that have a low specific activity for the fatty acid or alkane substrate of choice are targeted for inactivation, thereby reducing the physiological load on the cell.

Since it has been demonstrated that *CPR* can be limiting in yeast systems, the removal of non-essential P450s from the system can free electrons that are being used by non-essential P450s and make them available to the P450s important for diacid productivity. Moreover, the removal of non-essential P450s can make available other necessary but potentially limiting components of the P450 system (i.e., available membrane space, heme and/or NADPH).

Diacid productivity is thus improved by selective integration, amplification, and over expression of *CYP* and *CPR* genes in the C. *tropicalis* production host.

It should be understood that host cells into which one or more copies of desired *CYP* and/or *CPR* genes have been introduced can be made to include such genes by any technique known to those skilled in the art. For example, suitable host cells include procaryotes such as *Bacillus sp*., *Pseudomous sp., Actinomycetes sp., Eschericia sp., Mycobacterium sp.,* and eukaryotes such as yeast, algae, insect cells, plant cells and and filamentous fungi. Suitable host cells are preferably yeast cells such as *Yarrowia*, *Bebaromyces*, *Saccharomyces*, *Schizosaccharomyces*, and *Pichia* and more preferably those of the *Candida* genus. Preferred species of *Candida* are *tropicalis*, *maltosa*, *apicola*, *paratropicalis*, *albicans*, *cloacae*, *guillermondii, intermedia, lipolytica, parapsilosis* and *zeylenoides.* Certain preferred stains of *Candida tropicalis* are listed in U.S. Patent No. 5,254,466.

Vectors such as plasmids, phagemids, phages or cosmids can be used to transform or transfect suitable host cells. Host cells may also be transformed by introducing into a cell a linear DNA vector(s) containing the desired gene sequence. Such linear DNA may be advantageous when it is desirable to avoid introduction of non-native (foreign) DNA into the cell. For example, DNA consisting of a desired target gene(s) flanked by DNA sequences which are native to the cell can be introduced into the cell by electroporation, lithium acetate transformation, spheroplasting and the like. Flanking DNA sequences can include selectable markers and/or other tools for genetic engineering.

A suitable organic substrate herein can be any organic compound that is biooxidizable to a mono- or polycarboxylic acid. Such a compound can be any saturated or unsaturated aliphatic compound or any carbocyclic or heterocyclic aromatic compound having at least one terminal methyl group, a terminal carboxyl group and/or a terminal functional group which is oxidizable to a carboxyl group by biooxidation. A terminal functional group which is a derivative of a carboxyl group may be present in the substrate molecule and may be converted to a carboxyl group by a reaction other than biooxidation. For example, if the terminal group is an ester that neither the wild-type *C*. t*ropicalis* nor the genetic modifications described herein will allow hydrolysis of the ester functionality to a carboxyl group, then a lipase can be added during the fermentation step to liberate free fatty acids. Suitable organic substrates include, but are not limited to, saturated fatty acids, unsaturated fatty acids, alkanes, alkenes, alkynes and combinations thereof.

Alkanes are a type of saturated organic substrate which are useful herein. The alkanes can be linear or cyclic, branched or straight chain, substituted or unsubstituted. Particularly preferred alkanes are those having from about 4 to about 25 carbon atoms, examples of which include but are not limited to butane, hexane, octane, nonane, dodecane, tridecane, tetradecane, octadecane and the like.

Examples of unsaturated organic substrates which can be used herein include but are not limited to internal olefins such as 2-pentene, 2-hexene, 3-hexene, 9-octadecene and the like; unsaturated carboxylic acids such as 2-hexenoic acid and esters thereof, oleic acid and esters thereof including triglyceryl esters having a relatively high oleic acid content, erucic acid and esters thereof including triglyceryl esters having a relatively high erucic acid content, ricinoleic acid and esters thereof including triglyceryl esters having a relatively high ricinoleic acid content, linoleic acid and esters thereof including triglyceryl esters having a relatively high linoleic acid content; unsaturated alcohols such as 3-hexen-1-ol, 9-octadecen-1-ol and the like; unsaturated aldehydes such as 3-hexen-1-al, 9-octadecen-1-al and the like. In addition to the above, an organic substrate which can be used herein include alicyclic compounds having at least one internal carbon-carbon double bond and at least one terminal methyl group, a terminal carboxyl group and/or a terminal functional group which is oxidizable to a carboxyl group by biooxidation. Examples of such compounds include but are not limited to 3,6-dimethyl, 1,4-cyclohexadiene; 3-methylcyclohexene; 3-methyl-1, 4-cyclohexadiene and the like.

Examples of the aromatic compounds that can be used herein include but are not limited to arenes such as o-, m-, p-xylene; o-, m-, p-methyl benzoic acid; dimethyl pyridine, and the like. The organic substrate can also contain other functional groups that are biooxidizable to carboxyl groups such as an aldehyde or alcohol group. The organic substrate can also contain other functional groups that are not biooxidizable to carboxyl groups and do not interfere with the biooxidation such as halogens, ethers, and the like.

Examples of saturated fatty acids which may be applied to cells incorporating the present *CYP* and *CPR* genes include caproic, enanthic, caprylic, pelargonic, capric, undecylic, lauric, myristic, pentadecanoic, palmitic, margaric, stearic, arachidic, behenic acids and combinations thereof. Examples of unsaturated fatty acids which may be applied to cells incorporating the present *CYP* and *CPR* genes include palmitoleic, oleic, erucic, linoleic, linolenic acids and combinations thereof. Alkanes and fractions of alkanes may be applied which include chain links from C12 to C24 in any combination. An example of a preferred fatty acid mixtures are Emersol® 267 and Tallow, both commercially available from Henkel Chemicals Group, Cincinnati, OH. The typical fatty acid composition of Emersol® 267 and Tallow is as follows:

| | TALLOW | E267 |
|---|---|---|
| C14:0 | 3.5% | 2.4% |
| C14:1 | 1.0% | 0.7% |
| C15:0 | 0.5% | -------- |
| C16:0 | 25.5% | 4.6% |
| C16:1 | 4.0% | 5.7% |
| C17:0 | 2.5% | -------- |
| C17:1 | --------- | 5.7% |
| C18:0 | 19.5% | 1.0% |
| C18:1 | 41.0% | 69.9% |
| C18:2 | 2.5% | 8.8% |
| C18:3 | -------- | 0.3% |
| C20:0 | 0.5% | -------- |
| C20:1 | -------- | 0.9% |

The following examples are meant to illustrate but not to limit the invention. All relevant microbial strains and plasmids are described in Table 1 and Table 2, respectively.

**Table 1. List of Escherichia coli and Candida tropicalis strains**

| *E. Coli* **STRAIN** | **GENOTYPE** | **SOURCE** |
|---|---|---|
| XL1Blue-MRF' | *endA1, gyrA96, hsdR17, lac*⁻, *recA1*, *relA1*, *supE44*, *thi-l, [F' lacf^{q}Z M15. proAB,* Tn*10*] | Stratagene, La Jolla, CA |
| BM25.8 | *SupE44, thi (lac-pro4B)* [F' *traD36, proAB⁺, lacF^{q}Z M15*] *λimm434 (kan^{R})P1 (cam^{R}) hsdR (rₖ₁₂-mₖ₁₂-)* | Clontech, Palo Alto, CA |
| XLOLR | *(mcrA)183 (mcrCB-hsdSMR-mrr)173 endA1 thi-1 recA1 gyrA96 relA1 lac* [F'*proAB lacl^{q}Z M15* Tn*10*(Tet^{r}) Su⁻ (nonsuppressing λ^{r}(lambda resistant) | Stratagene, La Jolla, CA |
| | | |

| *C tropicalis* **STRAIN** | **GENOTYPE** | **SOURCE** |
|---|---|---|
| ATCC20336 | Wild-type | American Type Culture Collection, Rockville, MD |
| ATCC750 | Wild-type | American Type Culture Collection, Rockville, MD |
| ATCC 20962 | *ura3A*/*ura3B,* | Henkel |
| | *pax4A::ura3A*/*pox4B::ura3A,* | |
| | *pox5::ura3A*/*pox5:: URA3A* | |
| H5343 ura- | *ura3A*/*ura3B,* | Henkel |
| | *pox4A::ura3A*/*pox4B::ura3A,* | |
| | *pox5::ura3A*/*pox5::URA3A, ura3-* | |
| HDC1 | *ura3A*/*ura3B,* | Henkel |
| | *pox4A::ura3A*/*pox4B::ura3A.* | |
| | *pox5::ura3A*/*pox5::URA3A, .* | |
| | *ura3::URA3A-CYP52A2A* | |
| HDC5 | *ura3A*/*ura3B,* | Henkel |
| | *pox4A::ura3A*/*pox4B::ura3A,* | |
| | *pox5::ura3A*/*pox5::URA3A,* | |
| | *ura3::URA3A-CYP52A3A* | |
| HDC10 | *ura3A*/*ura3B,* | Henkel |
| | *pox4A::ura3A*/*pox4B::ura3A,* | |
| | *pox5::ura3A*/*pox5::URA3A,* | |
| | *ura3::URA3A-CPRB* | |
| HDC15 | *ura3A*/*ura3B,* | Henkel |
| | *pox4A::ura3A*/*pox4B::ura3A,* | |
| | *par5::ura3A*/*pox5::URA3A,* | |
| | *ura3::URA3A-CYP52A5A* | |
| HDC20 | *ura3A*/*ura3B,* | Henkel |
| | *pox4A::wa3A*/*pox4B::ura3A,* | |
| | *pox5::ura3A*/*pox5::URA3A,* | |
| | *ura3::URA3A-CYP52A2A + CPR B* | |
| | *(CYP* and *CPR* have opposite 5' to 3' orientation with respect to each other) | |
| HDC23 | *ura3A*/*ura3B,* | Henkel |
| | *pox4A::ura3A*/*pox4B::ura3A,* | |
| | *pox5::ura3A*/*pox5:: URA3A,* | |
| | *ura3::URA3A-CYP52A2A* + *CPR B* | |
| | (*CYP* and *CPR* have same 5' to 3' orientation with respect to each other) | |

**Table 2. List of plasmids isolated from genomic libraries and constructed for use in gene integrations.**

| **Plasmid** | **Base vector** | **Insert** | **Insert Size** | **Plasmid size** | **Description** |
|---|---|---|---|---|---|
| pURAin | pNEB193 | *URA3A* | 1706 bp | 4399 bp | pNEB193 with the *URA3A* gene inserted in the *Asc*I - *Pme*I site, generating a *Pac*I site |
| pURA 2in | pURAin | *CYP52A2A* | 2230 bp | 6629 bp | pURAin containing a PCR *CYP52A2A* allele containing *Pac*I restriction sites |
| pURA REDB in | pURAin | *CPRB* | 3266 bp | 7665 bp | pURAin containing a PCR *CPRB* allele containing *Pac*I restriction sites |
| pHKM1 | pTriplEx | Truncated *CPRA* gene | Approx. 3.8 kb | Approx. 7.4 kb | A truncated *CPRA* gene obtained by first screening library containing the 5' untranslated region and 1.2 kb open reading frame |
| pHKM4 | PTriplEx | Truncated *CPRA* gene | Approx. 5 kb | Approx. 8.6 kb | A truncated *CPRA* gene obtained by screening second library containing the 3' untranslated region end sequence |
| pHKM9 | pBC-CMV | *CPRB* gene | Approx. 5.3 kb | Approx. 9.8 kb | *CPRB* allele isolated from the third library |
| pHKM11 | pBC-CMV | *CYP52A1A* | Approx. 5kb | Approx. 9.5 kb | *CYP52A1A* isolated from the third library |
| pHKM12 | pBC-CMV | *CYP52A8A* | Approx. 7.5 kb | Approx. 12 kb | *CYP52A8A* isolated from the third library |
| pHKM13 | pBC-CMV | *CYP52D4A* | Approx. 7.3 kb | Approx. 11.8 kb | *CYP52D4A* isolated from the third library |
| pHKM14 | pBC-CMV | *CYP52A2B* | Approx. 6 kb | Approx. 10.5 kb | *CYP52A2B* isolated from the third library |
| pHKM15 | pBC-CMV | *CYP52A8B* | Approx. 6.6 kb | Approx. 11.1 kb | *CYP52A8B* isolated from the third library |
| pPAL3 | pTriplEx | *CYP52ASA* | 4.4 kb | Approx. 8.1 kb | *CYP52A5A* isolated from the 1st library |
| pPA5 | pTriplEx | *CYP52A5B* | 4.1 kb | Approx. 7.8 kb | *CYP52A5B* isolated from the 2nd library |
| pPA15 | pTriplEx | *CYP52A2A* | 6.0 kb | Approx. 9.7 kb | *CYP52A2A* isolated from the 2nd library |
| pPA57 | pTriplEx | *CYP52A3A* | 5.5 kb | Approx. 9.2 kb | *CYP52A3A* isolated from the 2nd library |
| pPA62 | pTriplEx | *CYP52A3B* | 6.0 kb | Approx. 9.7 kb | *CYP52A3B* isolated from the 2nd library |

### EXAMPLE 1

### Purification of Genomic DNA from Candida tropicalis ATCC 20336

### A. Construction of Genomic Libraries

50 ml of YEPD broth (see Chart) was inoculated with a single colony of *C*. *tropicalis* 20336 from YEPD agar plate and grown overnight at 30°C. 5 ml of the overnight culture was inoculated into 100 ml of fresh YEPD broth and incubated at 30°C for 4 to 5 hr with shaking. Cells were harvested by centrifugation, washed twice with sterile distilled water and resuspended in 4 ml of spheroplasting buffer (1 M Sorbitol, 50 mM EDTA, 14 mM mercaptoethanol) and incubated for 30 min at 37°C with gentle shaking. 0.5 ml of 2 mg/ml zymolyase (ICN Pharmaceuticals, Inc., Irvine, CA) was added and incubated at 37°C with gentle shaking for 30 to 60 min. Spheroplast formation was monitored by SDS lysis. Spheroplasts were harvested by brief centrifugation (4,000 rpm, 3 min) and were washed once with the spheroplast buffer without mercaptoethanol. Harvested spheroplasts were then suspended in 4 ml of lysis buffer (0.2 M Tris/pH 8.0, 50 mM EDTA, 1% SDS) containing 100 µg/ml RNase (Qiagen Inc., Chatsworth, CA) and incubated at 37°C for 30 to 60 min.

Proteins were denatured and extracted twice with an equal volume of chloroform/isoamyl alcohol (24:1) by gently mixing the two phases by hand inversions. The two phases were separated by centrifugation at 10,000 rpm for 10 min and the aqueous phase containing the high-molecular weight DNA was recovered. To the aqueous layer NaCl was added to a final concentration of 0.2 M and the DNA was precipitated by adding 2 vol of ethanol. Precipitated DNA was spooled with a clean glass rod and resuspended in TE buffer (10 mM Tris/pH 8.0, 1 mM EDTA) and allowed to dissolve overnight at 4°C. To the dissolved DNA, RNase free of any DNase activity (Qiagen Inc., Chatsworth, CA) was added to a final concentration of 50 µg/ml and incubated at 37°C for 30 min. Then protease (Qiagen Inc., Chatsworth, CA) was added to a final concentration of 100 µg/ml and incubated at 55 to 60°C for 30 min. The solution was extracted once with an equal volume of phenol/chloroform/isoamyl alcohol (25:24:1) and once with equal volume of chloroform/isoamyl alcohol (24:1). To the aqueous phase 0.1 vol of 3 M sodium acetate and 2 volumes of ice cold ethanol (200 proof) were added and the high molecular weight DNA was spooled with a glass rod and dissolved in 1 to 2 ml of TE buffer.

### B. Genomic DNA Preparation for PCR Amplification of CYP and CPR Genes

- Five 5 ml of YPD medium was inoculated with a single colony and grown at 30°C overnight. The culture was centrifuged for 5 min at 1200 x g. The supernatant was removed by aspiration and 0.5 ml of a sorbitol solution (0.9 M sorbitol, 0.1 M Tris-Cl pH 8.0, 0.1 M EDTA) was added to the pellet. The pellet was resuspended by vortexing and 1 µl of 2-mercaptoethanol and 50 µl of a 10 µg/ml zymolyase solution were added to the mixture. The tube was incubated at 37 °C for 1 hr on a rotary shaker (200 rpm). The tube was then centrifuged for 5 min at 1200 x g and the supernatant was removed by aspiration. The protoplast pellet was resuspended in 0.5 ml 1 × TE (10 mM Tris-Cl pH 8.0, 1 mM EDTA) and transferred to a 1.5 ml microcentrifuge tube. The protoplasts were lysed by the addition of 50 µl 10% SDS followed by incubation at 65°C for 20 min. Next, 200 µl of 5M potassium acetate was added and after mixing, the tube was incubated on ice for at least 30 min. Cellular debris was removed by centrifugation at 13,000 x g for 5 min. The supernatant was carefully removed and transferred to a new microfuge tube. The DNA was precipitated by the addition of 1 ml 100% (200 proof) ethanol followed by centrifugation for 5 min at 13,000 x g. The DNA pellet was washed with 1 ml 70 % ethanol followed by centrifugation for 5 min at 13,000 x g. After partially drying the DNA under a vacuum, it was resuspended in 200 µl of 1 × TE. The DNA concentration was determined by ratio of the absorbance at 260 nm / 280 nm (A_{260/280}).

### EXAMPLE 2

### . Construction of Candida tropicalis 20336 Genomic Libraries

Three genomic libraries of *C*. *tropicalis* were constructed, two at Clontech Laboratories, Inc., (Palo Alto, CA) and one at Henkel Corporation (Cincinnati, OH).

### A. Clontech Libraries

The first Clontech library was made as follows: Genomic DNA was prepared from C. *tropicalis* 20336 as described above, partially digested with *Eco*RI and size fractionated by gel electrophoresis to eliminate fragments smaller than 0.6 kb. Following size fractionation, several ligations of the *Eco*RI genomic DNA fragments and lambda (λ) Triplex^{™} vector (Figure 1) arms with *Eco*RI sticky ends were packaged into λ phage heads under conditions designed to obtain one million independent clones. The second genomic library was constructed as follows: Genomic DNA was digested partially with *Sau3A1* and size fractionated by gel electrophoresis. The DNA fragments were blunt ended using standard protocols as described, e.g., in Sambrook et al, Molecular Cloning: A Laboratory Manual, 2ed. Cold Spring Harbor Press, USA (1989). The strategy was to fill in the *Sau3A1* overhangs with Klenow polymerase (Life Technologies, Grand Island, NY) followed by digestion with S1 nuclease (Life Technologies, Grand Island, NY). After S1 nuclease digestion the fragments were end filled one more time with Klenow polymerase to obtain the final blunt-ended DNA fragments. *Eco*RI linkers were ligated to these blunt-ended DNA fragments followed by ligation into the λTriplEx vector. The resultant library contained approximately 2 X 10⁶ independent clones with an average insert size of 4.5 kb.

### B. Henkel Library

The third genomic library was constructed at Henkel Corporation using λZAP Express^{™} vector (Stratagene, La Jolla, CA) (Figure 2). Genomic DNA was partially digested with *Sau3A1* and fragments in the range of 6 to 12 kb were purified from an agarose gel after electrophoresis of the digested DNA. These DNA fragments were then ligated to *Bam*HI digested λZAP Express^{™} vector arms according to manufacturers protocols. Three ligations were set up to obtain approximately 9.8 X 10⁵ independent clones. All three libraries were pooled and amplified according to manufacturer instructions to obtain high-titre (>10⁹ plaque forming units/ml) stock for long-term storage. The titre of packaged phage library was ascertained after infection of *E*. *coli* XL1Blue-MRF' cells. *E. coli* XL1Blue-MRF' were grown overnight in either in LB medium or NZCYM (Chart) containing 10 mM MgSO₄ and 0.2% maltose at 37°C or 30°C, respectively with shaking. Cells were then centrifuged and resuspended in 0.5 to 1 volume of 10 mM MgSO₄. 200 µl of this *E. coli* culture was mixed with several dilutions of packaged phage library and incubated at 37°C for 15 min. To this mixture 2.5 ml of LB top agarose or NZCYM top agarose (maintained at 60°C ) (see Chart) was added and plated on LB agar or NCZYM agar (see Chart) present in 82 mm petri dishes. Phage were allowed to propagate overnight at 37°C to obtain discrete plaques and the phage titre was determined.

### EXAMPLE 3

### Screening of Genomic Libraries

Both λTriplEx^{™} and λZAP Express^{™} vectors are phagemid vectors that can be propagated either as phage or plasmid DNA (after conversion of phage to plasmid). Therefore, the genomic libraries constructed in these vectors can be screened either by plaque hybridization (screening of lambda form of library) or by colony hybridization (screening plasmid form of library after phage to plasmid conversion). Both vectors are capable of expressing the cloned genes and the main difference is the mechanism of excision of plasmid from the phage DNA. The cloning site in λTriplEx^{™} is located within a plasmid which is present in the phage and is flanked by *loxP* site (Figure 1). When λTriplEx^{™} is introduced into *E. coli* strain BM25.8 (supplied by Clontech), the *Cre* recombinase present in BM25.8 promotes the excision and circularization of plasmid pTriplEx from the phage λTriplEx^{™} at the *loxP* sites. The mechanism of excision of plasmid pBK-CMV from phage λZAP Express^{™} is different. It requires the assistance of a helper phage such as ExAssist^{™} (Stratagene) and an *E. coli* strain such as XLOR (Stratagene). Both pTriplEx and pBK-CMVcan replicate autonomously in *E. coli.*

### A. Screening Genomic Libraries (Plasmid Form)

### - 1) Colony Lifts

A single colony of *E. coli* BM25.8 was inoculated into 5 ml of LB containing 50 µg/ml kanamycin, 10 mM MgSO₄ and 0.1% maltose and grown overnight at 31 °C, 250 rpm. To 200 µl of this overnight culture (∼4 × 10⁸ cells) 1 µl of phage library (2 - 5 × 10⁶ plaque forming units) and 150 µl LB broth were added and incubated at 31°C for 30 min after which 400 µl of LB broth was added and incubated at 31°C, 225 rpm for 1 h. This bacterial culture was diluted and plated on LB agar containing 50 µg/ml ampicillin (Sigma Chemical Company, St. Louis, MO) and kanamycin (Sigma Chemical Company) to obtain 500 to 600 colonies/plate. The plates were incubated at 37°C for 6 to 7 hrs until the colonies became visible. The plates were then stored at 4°C for 1.5 h before placing a Colony/Plaque Screen^{™} Hybridization Transfer Membrane disc (DuPont NEN Research Products, Boston, MA) on the plate in contact with bacterial colonies. The transfer of colonies to the membrane was allowed to proceed for 3 to 5 min. The membrane was then lifted and placed on a fresh LB agar (see Chart) plate containing 200 µg/ml of chloramphenicol with the side exposed to the bacterial colonies facing up. The plates containing the membranes were then incubated at 37°C overnight in order to allow full development of the bacterial colonies. The LB agar plates from which colonies were initially lifted were incubated at 37°C overnight and stored at 4°C for future use. The following morning the membranes containing bacterial colonies were lifted and placed on two sheets of Whatman 3M (Whatman, Hillsboro, OR) paper saturated with 0.5 N NaOH and left at room temperature (RT) for 3 to 6 min to lyse the cells. Additional treatment of membranes was as described in the protocol provided by NEN Research Products.

### 2) DNA Hybridizations

Membranes were dried overnight before hybridizing to oligonucleotide probes prepared using a non-radioactive ECL^{™} 3'-oligolabelling and detection system from Amersham Life Sciences (Arlington Heights, IL). DNA labeling, prehybridization and hybridizations were performed according to manufacturer's protocols. After hybridization, membranes were washed twice at room temperature in 5 X SSC, 0.1% SDS (in a volume equivalent to 2 ml/cm² of membrane) for 5 min each followed by two washes at 50°C in 1X SSC, 0.1% SDS (in a volume equivalent to 2 ml/cm² of membrane) for 15 min each. The hybridization signal was then generated and detected with Hyperfilm ECL^{™} (Amersham) according to manufacturer's protocols. Membranes were aligned to plates containing bacterial colonies from which colony lifts were performed and colonies corresponding to positive signals on X-ray were then isolated and propagated in LB broth. Plasmid DNA's were isolated from these cultures and analyzed by restriction enzyme digestions and by DNA sequencing.

### B. Screening Genomic Libraries (Plaque Form)

### 1) λ Library Plating

*E. coli* XL1Blue-MRF' cells were grown overnight in LB medium (25 ml) containing 10 mM MgSO₄ and 0.2% maltose at 37°C, 250 rpm. Cells were then centrifuged (2,200 x g for 10 min) and resuspended in 0.5 volumes of 10 mM MgSO₄. 500 µl of this *E. coli* culture was mixed with a phage suspension containing 25,000 amplified lambda phage particles and incubated at 37°C for 15 min. To this mixture 6.5 ml of NZCYM top agarose (maintained at 60°C) (see Chart) was added and plated on 80 - 100 ml NCZYM agar (see Chart) present in a 150 mm petridish. Phage were allowed to propagate overnight at 37°C to obtain discrete plaques. After overnight growth plates were stored in a refrigerator for 1-2 hr before plaque lifts were performed.

### 2) Plaque Lift and DNA Hybridizations

Magna Lift^{™} nylon membranes (Micron Separations, Inc., Westborough, MA) were placed on the agar surface in complete contact with λ, plaques and transfer of plaques to nylon membranes was allowed to proceed for 5 min at RT. After plaque transfer the membrane was placed on 2 sheets of Whatman 3M^{™} (Whatman, Hillsboro, OR) filter paper saturated with a 0.5 N NaOH, 1.0 M NaCl solution and left for 10 min at RT to denature DNA. Excess denaturing solution was removed by blotting briefly on dry Whatman 3M paper. Membranes were then transferred to 2 sheets of Whatman 3M^{™} paper saturated with 0.5 M Tris-HCl (pH 8.0), 1.5 M NaCl and left for 5 min to neutralize. Membranes were then briefly washed in 200 - 500 ml of 2 X SSC, dried by air and baked for 30 - 40 min at 80°C. The membranes were then probed with labelled DNA.

Membranes were prewashed with a 200 - 500 ml solution of 5 X SSC, 0.5% SDS, 1 mM EDTA (pH 8.0) for 1- 2 hr at 42°C with shaking (60 rpm) to get rid of bacterial debris from the membranes. The membranes were prehybridized for 1 - 2 hr at 42°C with (in a volume equivalent to 0.125 - 0.25 ml/cm² of membrane) ECL Gold^{™} buffer (Amersham) containing 0.5 M NaCl and 5% blocking reagent. DNA fragments that were used as probes were purified from agarose gel using a QIAEX II^{™} gel extraction kit (Qiagen Inc., Chatsworth, CA) according to manufacturers protocol and labeled using an Amersham ECL^{™} direct nucleic acid labeling kit (Amersham). Labeled DNA (5 - 10 ng/ml hybridization solution) was added to the prehybridized membranes and the hybridization was allowed to proceed overnight. The following day membranes were washed with shaking (60 rpm) twice at 42°C for 20 min each time in (in a volume equivalent to 2 ml/cm² of membrane) a buffer containing either 0.1 (high stringency) or 0.5 (low stringency) X SSC, 0.4% SDS and 360 g/l urea. This was followed by two 5 min washes at room temperature in (in a volume equivalent to 2 ml/cm² of membrane) 2 X SSC. Hybridization signals were generated using the ECL^{™} nucleic acid detection reagent and detected using Hyperfilm ECL^{™} (Amersham).

Agar plugs which contained plaques corresponding to positive signals on the X-ray film were taken from the master plates using the broad-end of Pasteur pipet. Plaques were selected by aligning the plates with the x-ray film. At this stage, multiple plaques were generally taken. Phage particles were eluted from the agar plugs by soaking in 1 ml SM buffer (Sambrook et al., *supra*) overnight. The phage eluate was then diluted and plated with freshly grown *E*. *coli* XL1Blue-MRF' cells to obtain 100 - 500 plaques per 85 mm NCZYM agar plate. Plaques were transferred to Magna Lift nylon membranes as before and probed again using the same probe. Single well-isolated plaques corresponding to signals on X - ray film were picked by removing agar plugs and eluting the phage by soaking overnight in 0.5 ml SM buffer.

### C. Conversion of λ Clones to Plasmid Form

The lambda clones isolated were converted to plasmid form for further analysis. Conversion from the plaque to the plasmid form was accomplished by infecting the plaques into *E. coli* strain BM25.8. The *E*. *coli* strain was grown overnight at 31 °C, 250 rpm in LB broth containing 10 mM MgSO₄ and 0.2% maltose until the OD₆₀₀ reached 1.1 - 1.4. Ten milliliters of the overnight culture was removed and mixed with 100 µl of 1 M MgCl₂. A 200 µl volume of cells was removed, mixed with 150 µl of eluted phage suspension and incubated at 31°C for 30 min. LB broth (400 µl) was added to the tube and incubation was continued at 31 °C for 1 hr with shaking, 250 rpm. 1 - 10 µl of the infected cell suspension was plated on LB agar containing 100 µg/ml ampicillin (Sigma, St. Louis, MO). Well-isolated colonies were picked and grown overnight in 5 ml LB broth containing 100 µg/ml ampicillin at 37°C, 250 rpm. Plasmid DNA was isolated from these cultures and analyzed. To convert the λZAP Express^{™} vector to plasmid form *E*. *coli* strains XL1 Blue-MRF' and XLOR were used. The conversion was performed according to the manufacturer's (Stratagene) protocols for single-plaque excision.

### EXAMPLE 4

### Transformation of C. tropicalis H5343 ura-

### A. Transformation of C. tropicalis H5343 by Electroporation

5 ml of YEPD was inoculated with *C*. *tropicalis* H5343 *ura-* from a frozen stock and incubated overnight on a New Brunswick shaker at 30°C and 170 rpm. The next day, 10 µl of the overnight culture was inoculated into 100 ml YEPD and growth was continued at 30°C, 170 rpm. The following day the cells were harvested at an OD₆₀₀ of 1.0 and the cell pellet was washed one time with sterile ice-cold water. The cells were resuspended in ice-cold sterile 35 % Polyethylene glycol (4,000 MW) to a density of 5x10¹ cells/ml. A 0.1 ml volume of cells were utilized for each electroporation. The following electroporation protocol was followed: 1.0 µg of transforming DNA was added to 0.1 ml cells, along with 5 µg denatured, sheared calf thymus DNA and the mixtures was allowed to incubate on ice for 15 min. The cell solution was then transferred to an ice-cold 0.2 cm electroporation cuvette, tapped to make sure the solution was on the bottom of the cuvette and electroporated. The cells were electroporated using an Invitrogen electroporator (Carlsbad, CA) at 450 Volts, 200 Ohms and 250 µF. Following electroporation, 0.9 ml SOS media (1M Sorbitol, 30% YEPD, 10 mM CaCl₂) was added to the suspension. The resulting culture was grown for 1 hr at 30°C, 170 rpm. Following the incubation, the cells were pelleted by centrifugation at 1500 x g for 5 min. The electroporated cells were resuspended in 0.2 ml of 1M sorbitol and plated on synthetic complete media minus uracil (SC - uracil) (Nelson, *supra*). In some cases the electroporated cells were plated directly onto SC - uracil. Growth of transformants was monitored for 5 days. After three days, several transformants were picked and transferred to SC-uracil plates for genomic DNA preparation and screening.

### B. Transformation of C. tropicalis Using Lithium Acetate

The following protocol was used to transform *C*. *tropicalis* in accordance with the procedures described in Current Protocols in Molecular Biology, Supplement 5, 13.7.1 (1989).

5 ml of YEPD was inoculated with *C*. *tropicalis* H5343 *ura*- from a frozen stock and incubated overnight on a New Brunswick shaker at 30°C and 170 rpm. The next day, 10 µl of the overnight culture was inoculated into 50 ml YEPD and growth was continued at 30°C, 170 rpm. The following day the cells were harvested at an OD₆₀₀ of 1.0. The culture was transferred to a 50 ml polypropylene tube and centrifuged at 1000 X g for 10 min. The cell pellet was resuspended in 10 ml sterile TE (10mM Tris-Cl and 1mM EDTA, pH 8.0). The cells were again centrifuged at 100b X g for 10 min and the cell pellet was resuspended in 10 ml of a sterile lithium acetate solution [LiAc (0.1 M lithium acetate, 10 mM Tris-Cl, pH 8.0, 1 mM EDTA)]. Following centrifugation at 1000 X g for 10 min., the pellet was resuspended in 0.5 ml LiAc. This solution was incubated for one hour at 30°C while shaking gently at 50 rpm. A 0.1 ml aliquot of this suspension was incubated with 5 µg of transforming DNA at 30°C with no shaking for 30 min. A 0.7 ml PEG solution (40 % wt/vol polyethylene glycol 3340, 0.1 M lithium acetate, 10 mM Tris-Cl, pH 8.0, 1 mM EDTA) was added and incubated at 30°C for 45 min. The tubes were then placed at 42°C for 5 min. 2A 0.2 ml aliquot was plated on synthetic complete media minus uracil (SC - uracil) (Kaiser et al. Methods in Yeast Genetics, Cold Spring Harbor Laboratory Press, USA, 1994. Growth of transformants was monitored for 5 days. After three days, several transformants were picked and transferred to SC-uracil plates for genomic DNA preparation and screening.

### EXAMPLE 5

### Plasmid DNA Isolation

Plasmid DNA were isolated from *E. coli* cultures using Qiagen plasmid isolation kit (Qiagen Inc., Chatsworth, CA) according to manufacturer's instructions.

### EXAMPLE 6

### DNA Sequencing and Analysis

DNA sequencing was performed at Sequetech Corporation (Mountain View, CA) using Applied Biosystems automated sequencer (Perkin Elmer, Foster City, CA). DNA sequences were analyzed with MacVector and GeneWorks software packages (Oxford Molecular Group, Campbell, CA).

### EXAMPLE 7

### PCR Protocols

PCR amplification was carried out in a Perkin Elmer Thermocycler using the Ampli*Taq*Gold enzyme (Perkin Elmer Cetus, Foster City, CA) kit according to manufacturer's specifications. Following successful amplification, in some cases, the products were digested with the appropriate enzymes and gel purified using QiaexII (Qiagen, Chatsworth, CA) as per manufacturer instructions. In specific cases the Ultma *Taq* polymerase (Perkin Elmer Cetus, Foster City, CA) or the Expand Hi-Fi *Taq* polymerase (Boehringer Mannheim, Indianapolis, IN) were used per manufacturer's recommendations or as defined in Table 3.

**Table 3. PCR amplification conditions used with different primer combinations.**

| **PRIMER COMBINATION** | ***Taq*** | **TEMPLATE DENATURING CONDITION** | **ANNEALING TEMP/TIME** | **EXTENSION TEMP/TIME** | **CYCLE Number** |
|---|---|---|---|---|---|
| 3674-41-1/41-2/41-4 + 3674-41-4 | Ampli-*Taq* Gold | 94 C/30 sec | 55 C/30 sec | 72 C/1min | 30 |
| URA Primer 1a | Ampli-*Taq* Gold | 95 C/1 min | 70 C/1 min | 72 C/2 min | 35 |
| URA Primer 1b | | | | | |
| URA Primer 2a | Ampli-*Taq* Gold | 95 C/1 min | 70 C/1 min | 72 C/2 min | 35 |
| URA Primer 2b | | | | | |
| *CYP2A*#1 | Ampli-*Taq* Gold | 95 C/1 min | 70 C/1 min | 72 C/2 min | 35 |
| *CYP*2A#2 | | | | | |
| *CYP3*A# 1 | Ultma *Taq* | 95 C/1 min | 70 C/1 min | 72 C/1 min | 30 |
| *CYP3*A#2 | | | | | |
| *CPR* B#1 | Expand | 94 C/15 sec | 50 C/30 sec | 68 C/3 min | 10 |
| *CPR* B#2 | Hi-Fi *Taq* | 94 C/15 sec | 50 C/30 sec | 68 C/3 min +20 sec/cycle | 15 |
| *CYP5*A#1 | Expand | 94 C/15 sec | 50 C/30 sec | 68 C/3 min | 10 |
| *CYPS*A#2 | Hi-Fi *Taq* | 94 C/15 sec | 50 C/30 sec | 68 C/3 min +20 sec/cycle | 15 |

Table 4 below contains a list of primers (SEQ ID NOS: 1-35) used for PCR amplification to construct gene integration vectors or to generate probes for gene detection and isolation.

**Table 4. Primer table for PCR amplification to construct gene integration vectors, to generate probes for gene isolation and detection and to obtain DNA sequence of constructs. (A-deoxyadenosine triphosphate [dATP], G- deoxyguanosine triphosphate [dGTP], C-deoxycytosine triphosphate [dCTP], T- deoxythymidine triphosphate [dTTP], Y- dCTP or dTTP, R- dATP or dGTP, W- dATP or dTTP, M- dATP or dCTP, N- dATP or dCTP or dGTP or dTTP).**

| **Target gene(s)** | **Patent Primer Name** | **Lab Primer Name** | **Sequence (5' to 3')** | **PCR Product Size** |
|---|---|---|---|---|
| | | | | |
| *CYP52A2A* | CYP2A#1 | 3659-72M | | 2230 bp |
| | CYP2A#2 | 3659-72N | | |
| | | | | |
| *CYP52A3A* | CYP3A#1 | 3659-720 | | 2154 bp |
| | CYP3A#2 | 3659-72P | | |
| | | | | |
| *CYP52A5A* | CYP5A#1 | 3659-72K | | 3298 bp |
| | CYPSA#2 | 3659-72L | | |
| | | | | |
| *CPRB* | CPRB#1 | 3698-20A | | 3266 bp |
| | CPRB#2 | 3698-20B | | |
| | | | | |
| *URA3A* | URA Primer 1a | 3698-7C | | 956 bp |
| | URA Primer 1b | 3698-7D | | |
| | | | | |
| *URA3A* | URA Primer 2a | 3698-7A | | 750 bp |
| | URA Primer 2b | 3698-7B | | |
| | | | | |
| | | | *GGGTTTAAAC- Pme* I restriction site (SEQ ID NO: 13) | |
| | | | *AGGCGCGCC - Asc*I restriction site (SEQ ID NO: 14) | |
| | | | *CCTTAATTAA- Pac*I restriction site (SEQ ID NO: 15) | |
| | | | | |
| *CPR* | FMN1 | 3674-41-1 | TCYCAAACWGGTACWGCWGAA (SEQ ID NO: 16) | |
| *CPR* | FMN2 | 3674-41-2 | GGTTTGGGTAAYTCWACTTAT (SEQ ID NO: 17) | |
| *CPR* | FAD | 3674-41-3 | CGTTATTAYTCYATTTCTTC (SEQ ID NO: 18) | |
| *CPR* | NADPH | 3674-41-4 | GCMACACCRGTACCTGGACC (SEQ ID NO: 19) | |
| *CPR* | PRK1.F3 | PRK1.F3 | ATCCCAATCGTAATCAGC (SEQ ID NO: 20) | |
| *CPR* | PRK1.F5 | PRY1.F5 | ACTTGTCTTCGTTTAGCA (SEQ ID NO: 21) | |
| *CPR* | PRK4.R20 | PRK4.R20 | CTACGTCTGTGGTGATGC (SEQ ID NO: 22) | |
| *CYP* | UCup1 | UCup1 | CGNGAYACNACNGCNGG (SEQ ID NO: 23) | |
| *CYP* | UCup2 | UCup2 | AGRGAYACNACNGCNGG (SEQ ID NO: 24) | |
| *CYP* | UCdown1 | UCdown1 | AGNGCRAAYTGYTGNCC (SEQ ID NO: 25) | |
| *CYP* | UCdown2 | UCdown2 | YAANGCRAAYTGYTGNCC (SEQ ID NO: 26) | |
| *CYP* | HemeB1 | HemeB 1 | | |
| *CYP* | 2,3,5P | 2,3,5P | GAGCTATGTTGAGACCACAGTTTGC (SEQ ID NO: 28) | |
| *CYP* | 2,3,5M | 2,3,5M | CTTCAGTTAAAGCAAATTGTTTGGCC (SEQ ID NO: 29) | |
| pTriplEx vector | Triplex5' | Triplex5' | CTCGGGAAGCGCGCCATTGTGTTGG (SEQ ID NO: 30) | |
| pTriplEx vector | Triplex3' | Triplex3' | | |
| *CYP* | Cyp52a | Cyp52a | TGRYTCAAACCATCTYTCTGG (SEQ ID NO: 32) | |
| *CYP* | Cyp52b | Cyp52b | GGACCGGCGTTAAAGGG (SEQ ID NO: 33) | |
| *CYP* | Cyp52c | Cyp52c | CATAGTCGWATYATGCTTAGACC (SEQ ID NO: 34) | |
| *CYP* | Cyp52d | Cyp52d | GGACCACCATTGAATGG (SEQ ID NO: 35) | |

### EXAMPLE 8

### Yeast Colony PCR Procedure for Confirmation of Gene Integration into the Genome of C. tropicalis

Single yeast colonies were removed from the surface of transformation plates, suspended in 50 µl of spheroplasting buffer (50mM KCI, 10mM Tris-HCl, pH 8.3, 1.0 mg/ml Zymolyase, 5% glycerol) and incubated at 37°C for 30 min. Following incubation, the solution was heated for 10 min at 95°C to lyse the cells. Five µlof this solution was used as a template in PCR Expand Hi-Fi *Taq* polymerase (Boehringer Mannheim, Indianapolis, IN) was used in PCR coupled with a gene-specific primer (gene to be integrated) and a *URA3* primer. If integration did occur, amplification would yield a PCR product of predicted size confirming the presence of an integrated gene.

### EXAMPLE 9

### Fermentation Method for Gene Induction Studies

A fermentor was charged with a semi-synthetic growth medium having the composition 75 g/l glucose (anhydrous), 6.7 g/l Yeast Nitrogen Base (Difco Laboratories), 3 g/l yeast extract, 3 g/l ammonium sulfate, 2 g/l monopotassium phosphate, 0.5 g/l sodium chloride. Components were made as concentrated solutions for autoclaving then added to the fermentor upon cooling: final pH approximately 5.2. This charge was inoculated with 5-10% of an overnight culture of *C*. *tropicalis* ATCC 20962 prepared in YM medium (Difco Laboratories) as described in the methods of Examples 17 and 20 of US Patent 5,254,466. *C. tropicalis* ATCC 20962 is a POX 4 and POX 5 disrupted *C*. *tropicalis* ATCC 20336. Air and agitation were supplied to maintain the dissolved oxygen at greater than about 40% of saturation versus air. The pH was maintained at about 5.0 to 8.5 by the addition of 5N caustic soda on pH control. Both a fatty acid feedstream (commercial oleic acid in this example) having a typical composition: 2.4% C₁₄;0.7% C₁₄; 4.6% C₁₆;5.7% C_{16:1};5.7% C_{17:1}; 1.0% C₁₈;69.9% C_{18:1}; 8.8% C_{18:2}; 0.30% C_{18:3}; 0.90% C_{20:1} and a glucose co-substrate feed were added in a feedbatch mode beginning near the end of exponential growth. Caustic was added on pH control during the bioconversion of fatty acids to diacids to maintain the pH in the desired range. Typically, samples for gene induction studies were collected just prior to starting the fatty acid feed and over the first 10 hours of bioconversion. Determination of fatty acid and diacid content was determined by a standard methyl ester protocol using gas liquid chromatography (GLC). Gene induction was measured using the QC-RT-PCR protocol described in this application.

### EXAMPLE 10

### RNA Preparation

The first step of this protocol involves the isolation of total cellular RNA from cultures of *C*. *tropicalis.* The cellular RNA was isolated using the Qiagen RNeasy Mini Kit (Qiagen Inc., Chatsworth, CA) as follows: 2 ml samples of *C*. *tropicalis* cultures were collected from the fermentor in a standard 2 ml screw capped Eppendorf style tubes at various times before and after the addition of the fatty acid or alkane substrate. Cell samples were immediately frozen in liquid nitrogen or a dry-ice/alcohol bath after their harvesting from the fermentor. To isolate -total RNA from the samples, the tubes were allowed to thaw on ice and the cells pelleted by centrifugation in a microfuge for 5 minutes (min) at 4°C and the supernatant was discarded while keeping the pellet ice-cold. The microfuge tubes were filled 2/3 full with ice-cold Zirconia/Silica beads (0.5 mm diameter, Biospec Products, Bartlesville, OK) and the tube filled to the top with ice-cold RLT* lysis buffer (* buffer included with the Qiagen RNeasy Mini Kit). Cell rupture was achieved by placing the samples in a mini bead beater (Biospec Products, Bartlesville, OK) and immediately homogenized at full speed for 2.5 min. The samples were allowed to cool in a ice water bath for 1 minute and the homogenization/cool process repeated two more times for a total of 7.5 min homogenization time in the beadbeater. The homogenized cells samples were microfuged at full speed for 10 min and 700 µl of the RNA containing supernatant removed and transferred to a new eppendorf tube. 700 µl of 70% ethanol was added to each sample followed by mixing by inversion. This and all subsequent steps were performed at room temperature. Seven hundred microliters of each ethanol treated sample were transferred to a Qiagen RNeasy spin column, followed by centrifugation at 8,000 x g for 15 sec. The flow through was discarded and the column reloaded with the remaining sample (700 µl) and re-centrifuged at 8,000 x g for 15 sec. The column was washed once with 700 µl of buffer RW 1*, and centrifuged at 8,000 x g for 15 sec and the flow through discarded. The column was placed in a new 2 ml collection tube and washed with 500 µl of RPE* buffer and the flow through discarded. The RPE* wash was repeated with centrifugation at 8,000 x g for 2 min and the flow through discarded. The spin column was transferred to a new 1.5 ml collection tube and 100 µl of RNase free water added to the column followed by centrifugation at 8,000 x g for 15 seconds. An additional 75 µl of RNase free water was added to the column followed by centrifugation at 8,000 x g for 2 min. RNA eluted in the water flow through was collected for further purification.

The RNA eluate was then treated to remove contaminating DNA. Twenty microliters of 10X DNase I buffer (0.5 M tris (pH 7.5), 50 mM CaCl₂, 100 mM MgCl₂), 10 µl of RNase-free DNase I (2 Units/µl, Ambion Inc., Austin, Texas) and 40 units Rnasin (Promega Corporation, Madison, Wisconsin) were added to the RNA sample. The mixture was then incubated at 37°C for 15 to 30 min. Samples were placed on ice and 250 µl Lysis buffer RLT* and 250 µl ethanol (200 proof) added. The samples were then mixed by inversion. The samples were transferred to Qiagen RNeasy spin columns and centrifuged at 8,000 x g for 15 sec and the flow through discarded. Columns were placed in new 2 ml collection tubes and washed twice with 500 µl of RPE* wash buffer and the flow through discarded. Columns were transferred to new 1.5 ml eppendorf tubes and RNA was eluated by the addition of 100 µl of DEPC treated water followed by centrifugation at 8,000 x g for 15 sec. Residual RNA was collected by adding an additional 50 µl of RNase free water to the spin column followed by centrifugation at full speed for 2 min. 10 µl of the RNA preparation was removed and quantified by the (A_{260/280}) method. RNA was stored at
-70°C. Yields were found to be 30-100 µg total RNA per 2.0 ml of fermentation broth.

### EXAMPLE 11

### Quantitative Competitive Reverse Transcription Polymerase Chain Reaction (QC-RT-PCR) Protocol

QC-RT-PCR is a technique used to quantitate the amount of a specific RNA in a RNA sample. This technique employs the synthesis of a specific DNA molecule that is complementary to an RNA molecule in the original sample by reverse transcription and its subsequent amplification by polymerase chain reaction. By the addition of various amounts of a competitor RNA molecule to the sample one can determine the concentration of the RNA molecule of interest (in this case the mRNA transcripts of the *CYP* and *CPR* genes). The levels of specific mRNA transcripts were assayed over time in response to the addition of fatty acid and/or alkane substrates to the growth medium of fermentation grown *C*. *tropicalis* cultures for the identification and characterization of the genes involved in the oxidation of these substrates. This approach can be used to identify the *CYP* and *CPR* genes involved in the oxidation of any given substrate based upon their transcriptional regulation.

### A. Primer Design

The first requirement for QC-RT-PCR is the design of the primer pairs to be used in the reverse transcription and subsequent PCR reactions. These primers need to be unique and specific to the gene of interest. As there is a family of genetically similar *CYP* genes present in *C. tropicalis* 20336, care had to be taken to design primer pairs that would be discriminating and only amplify the gene of interest, in this example the *CYP52A5* gene. In this manner, unique primers directed to substantially non-homologous (aka variable) regions within target members of a gene family are constructed. What constitutes substantially non-homologous regions is determined on a case by case basis. Such unique primers should be specific enough to anneal the non-homologous region of the target gene without annealing to other non-target members of the gene family. By comparing the known sequences of the members of a gene family, non-homologous regions are identified and unique primers are constructed which will anneal to those regions. It is contemplated that non-homologous regions herein would typically exhibit less than about 85% homology but can be more homologous depending on the positions which are conserved and stringency of the reaction. After conducting PCR, it may be helpful to check the reaction product to assure it represents the unique target gene product. If not, the reaction conditions can be altered in terms of stringency to focus the reaction to the desired target. Alternatively a new primer or new non-homologous region can be chosen. Due to the high level of homology between the genes of the *CYP52A* family, the most variable 5 prime region of the *CYP52A5* coding sequence was targeted for the design of the primer pairs. In Figure 3, a portion of the 5 prime coding region for the *CYP52A5A* (SEQ ID NO: 36) allele of *C*. *tropicalis* 20336 is shown. The boxed sequences in Figure 3 are the sequences of the forward and backwards primers (SEQ ID NOS: 47 and 48) used to quantitate expression of both alleles of this gene. The actual reverse primer (SEQ ID NO: 48) contains one less adenine than that shown in Figure 3. Primers used to measure the expression of specific *C*. *tropicalis* 20336 genes using the QC-RT-PCR protocol are listed in Table 5 (SEQ ID NOS: 37-58).

**Table 5. Primer used to measure C. tropicalis gene expression in the QC-RT-PCR reactions.**

| **Primer Name** | **Direction** | **Target** | **Sequence** |
|---|---|---|---|
| 3737-89F | F | *CYP52A1A* | CCGATGAAGTTTTCGACGAGTACCC (SEQ ID NO: 37) |
| 3737-89B | B | *CYP52A1A* | AAGGCTTTAACGTGTCCAATCTGGTC (SEQ ID NO: 38) |
| alk2aF1 | F | *CYP52A2A* | ATTATCGCCACATACTTCACCAAATGG (SEQ ID NO: 39) |
| alk2aB5 | B | *CYP52A2A* | CGAGATCGTGGATACGCTGGAGTG (SEQ ID NO: 40) |
| 7581-178-3 | F | *CYP52A3A* | GCCACTCGGTAACTTTGTCAGGGAC (SEQ ID NO: 41) |
| 7581-178-4 | B | *CYP52A3A* | CATTGAACTGAGTAGCCAAAACAGCC (SEQ ID NO: 42) |
| 3737-50F | F | *CYP52A3A* & *CYP52A3B* | CCTACGTTTGGTATCGCTACTCCGTTG (SEQ ID NO: 43) |
| 3737-50B | B | *CYP52A3A* & *CYP52A3B* | TTTCCAGCCAGCACCGTCCAAG (SEQ ID NO: 44) |
| 3737-175F | F | *CYP52D4A* | GCAGAGCCGATCTATGTTGCGTCC (SEQ ID NO: 45) |
| 3737-175B | B | *CYP52D4A* | TCATTGAATGCTTCCAGGAACCTCG (SEQ ID NO: 46) |
| 7581-97-F | F | *CYP52A5A& CYP52A5B* | AAGAGGGCAGGGCTCAAGAG (SEQ ID NO: 47) |
| 7581-97-M | B | *CYP52A5A& CYP52A5B* | TCCATGTGAAGATCCCATCAC (SEQ ID NO: 48) |
| 4P-2 | F | *CYP52A8A* | CTTGAAGGCCGTGTTGAACG (SEQ ID NO: 49) |
| 4M-1 | B | *CYP52A8A* | CAGGATTTGTCTGAGTTGCCG (SEQ ID NO: 50) |
| 3737-52F | F | *POX4A & POX4B* | CCATTGCCTTGAGATACGCCATTGGTAG (SEQ ID NO: 51) |
| 3737-52B | B | *POX4A & POX4B* | AGCCTTGGTGTCGTTCTTTTCAACGG (SEQ ID NO: 52) |
| 3737-53F | F | *POX5A* | TTGGGTTTGTTTGTTTCCTGTGTCCG (SEQ ID NO: 53) |
| 3737-53B | B | *POX5A* | CCTTTGACCTTCAATCTGGCGTAGACG (SEQ ID NO: 54) |
| F33 | F | *CPRA* | GGTTTGCTGAATACGCTGAAGGTGATG (SEQ ID NO: 55) |
| B63 | B | *CPRA* | TGGAGCTGAACAACTCTCTCGTCTCGG (SEQ ID NO: 56) |
| 3737-133F | F | *CPRA* & *CPRB* | TTCCTCAACACGGACAGCGG (SEQ ID NO: 57) |
| 3737-133B | B | *CPRA & CPRB* | AGTCAACCAGGTGTGGAACTCGTC (SEQ ID NO: 58) |

| | | | |
|---|---|---|---|
| F=Forward B=Backward | | | |

### B. Design and Synthesis of the Competitor DNA Template

The competitor RNA is synthesized *in vitro* from a competitor DNA template that has the T7 polymerase promoter and preferably carries a small deletion of e.g., about 10 to 25 nucleotides relative to the native target RNA sequence. The DNA template for the *in-vitro* synthesis of the competitor RNA is synthesized using PCR primers that are between 46 and 60 nucleotides in length. In this example, the primer pairs for the synthesis of the *CYP52A5* competitor DNA are shown in Tables 6 and 7 (SEQ ID NOS: 59 AND 60).

**Table 6. Forward and Reverse primers used to synthesize the competitor RNA template for the QC-RT-PCR measurement of CYP52A5A gene expression.**

| | | |
|---|---|---|
| Forward Primer | *CYP52A5A* | |
| Reverse Primer | *CYP52A5A* | |

**Table 7. Primers for the synthesis of the QC-RT-PCR competitor RNA templates**

| **Primer Name** | **Direction** | **Target** | **Sequence 5'-3'** |
|---|---|---|---|
| 3737-89C | F | *CYP52A1A* | |
| 3737-89D | B | *CYP52A1A* | |
| 7581-137-A | F | *CYP52A2A* | |
| 7581-137-B | B | *CYP52A2A* | |
| 7581-137-D | B | *CYP52A3A* | |
| 7581-137-C | F | *CYP52A3A* | |
| 3737-50-D | F | *CYP52A3A* & *CYP52A3B* | |
| 3737-50-C | B | *CYP52A3A* & *CYP52A3B* | |
| 3737-175C | F | *CYP52D4A* | |
| 3737-175D | B | *CYP52D4A* | |
| 581-97-A | F | *CYP52A5A* & *CYP52A5B* | |
| 7581-97-B | B | *CYP52A5A* & *CYP52A5B* | |
| 4P-2/T7 | F | *CYP52A8A* | |
| 4M-3/4M-1 | B | *CYP52A8A* | |
| 3737-26-D | F | *CPRA* | |
| 3737-26-C | B | *CPRA* | |
| 3737-133C | F | *CPRA & CPRB* | |
| 3737-133D | B | *CPRA & CPRB* | |
| 3737-52-C | F | *POX4A & POX4B* | |
| 3737-52-D | B | *POX4A & POX4B* | |
| 3737-53-C | F | *POX5A* | |
| 3737-53-D | B | *POX5A* | |

| | | | |
|---|---|---|---|
| F=Forward B=Backword | | | |

The forward primer (SEQ ID NO: 59) contains the T7 promoter consensus sequence "GGATCCTAATACGA CTCACTATAGGG AGG" fused to the primer 7581-97-F sequence (SEQ ID NO: 47). The Reverse Primer (SEQ ID NO: 60) contains the sequence of primer 7581-97M (SEQ ID NO: 48) followed by the 20 bases of upstream sequence with a 18 base pair deletion between the two blocks of the *CYP52A5* sequence. The forward primer was used with the corresponding reverse primer to synthesize the competitor DNA template. The primer pairs were combined in a standard *Taq* Gold polymerase PCR reaction according to the manufacturer's recommended conditions (Perkin-Elmer/Applied Biosystems, Foster City, CA). The PCR reaction mix contained a final concentration of 250 nM each primer and 10 ng *C*. *tropicalis* chromosomal DNA for template. The reaction mixture was placed in a thermocycler for 25 to 35 cycles using the highest annealing temperature possible during the PCR reactions to assure a homogeneous PCR product (in this case 62°C). The PCR products were either gel purified or filtered purified to remove un-incorporated nucleotides and primers. The competitor template DNA was then quantified using the (A_{260/280}) method. Primers used in QC-RT-PCR experiments for the synthesis of various competitive DNA templates are listed in Table 7 (SEQ ID NOS: 61-80).

### C. Synthesis of the Competitor RNA

Competitor template DNA was transcribed *In-Vitro* to make the competitor RNA using the Megascript T7 kit from Ambion Biosciences (Ambion Inc., Austin, Texas). 250 nanograms (ng) of competitor DNA template and the *in-vitro* transcription reagents are mixed according to the directions provided by the manufacturer. The reaction mixture was incubated for 4 hours at 37°C. The resulting RNA preparations were then checked by gel electrophoresis for the conditions giving the highest yields and quality of competitor RNA. This often required optimization according to the manufacturer's specifications. The DNA template was then removed using DNase I as described in the Ambion kit. The RNA competitor was then quantified by the (A_{260/280}) method. Serial dilution's of the RNA (1 ng/µl to 1 femtogram (fg)/µl) were made for use in the QC-RT-PCR reactions and the original stocks stored at -70°C.

### D. QC-RT-PCR Reactions

QC-RT-PCR reactions were performed using rTth polymerase from Perkin-Elmer(Perkin-Elmer/Applied Biosystems, Foster City, CA) according to the manufacturer's recommended conditions. The reverse transcription reaction was performed in a 10 µl volume with a final concentrations of 200 µM for each dNTP, 1.25 units rTth polymerase, 1.0 mM MnCl₂, I X of the 10X buffer supplied with the Enzyme from the manufacturer, 100 ng of total RNA isolated from a fermentor grown culture of *C*. *tropicalis* and 1.25 µM of the appropriate reverse primer. To quantitate *CYP52A5* expression in *C*. *tropicalis* an appropriate reverse primer was 7581-97M (SEQ ID NO: 48). Several reaction mixes were prepared for each RNA sample characterized. To quantitate *CYP52A5* expression a series of 8 to 12 of the previously described QC-RT-PCR reaction mixes were aliquoted to different reaction tubes. To each tube 1 µl of a serial dilution containing from 100 pg to 100 fg *CYP52A5* competitor RNA per µl was added bringing the final reaction mixtures up to the final volume of 10 µl. The QC-RT-PCR reaction mixtures were mixed and incubated at 70°C for 15 min according to the manufacturer's recommended times for reverse transcription to occur. At the completion of the 15 minute incubation, the sample temperature was reduced to 4°C to stop the reaction and 40 µl of the PCR reaction mix added to the reaction to bring the total volume up to 50 µl. The PCR reaction mix consists of an aqueous solution containing 0.3125 µM of the forward primer 7581-97F (SEQ ID NO: 47), 3.125 mM MgCl, and 1X chelating buffer supplied with the enzyme from Perkin-Elmer. The reaction mixtures were placed in a thermocycler (Perkin-Elmer GeneAmp PCR System 2400, Perkin-Elmer/Applied Biosystems, Foster City, CA) and the following PCR cycle performed: 94°C for 1 min. followed by 94°C for 10 seconds followed by 58°C for 40 seconds for 17 to 22 cycles. The PCR reaction was completed with a final incubation at 58°C for 2 min followed by 4°C. In some reactions where no detectable PCR products were produced the samples were returned the thermocycler for additional cycles, this process was repeated until enough PCR products were produced to quantify using HPLC. The number of cycles necessary to produce enough PCR product is a function of the amount of the target mRNA in the 100 ng of total cellular RNA. In cultures where the *CYP52A5* gene is highly expressed there is sufficient *CYP52A5* mRNA message present and less PCR cycles (≤17) are required to produce quantifiable amount of PCR product. The lower the concentrations of the target mRNA present the more PCR cycles are required to produce a detectable amount of product. These QC-RT-PCR procedures were applied to all the target genes listed in Table 5 using the respective primers indicated therein.

### E. HPLC Quantification

Upon completion of the QC-RT-PCR reactions the samples were analyzed and quantitated by HPLC. Five to fifteen microliters of the QC-RT-PCR reaction mix was injected into a Waters Bio-Compatible 625 HPLC with an attached Waters 484 tunable detector. The detector was set to measure a wave length of 254 nm. The HPLC contained a Sarasep brand DNASep™ column (Sarasep, Inc., San Jose, CA) which was placed within the oven and the temperature set for 52 °C. The column was installed according to the manufacturer's recommendation of having 30 cm. of heated PEEK tubing installed between the injector and the column. The system was configured with a Sarasep brand Guard column positioned before the injector. In addition, there was a 0.22 µm filter disk just before the column, within the oven. Two Buffers were used to create an elution gradient to resolve and quantitate the PCR products from the QC-RT-PCR reactions. Buffer-A consists of 0.1 M tri-ethyl ammonium acetate (TEAA) and 5% acetonitrile (volume to volume). Buffer-B consists of 0.1 M TEAA and 25% acetonitrile (volume to volume). The QC-RT-PCR samples were injected into the HPLC and the linear gradient of 75% buffer-A/ 25% buffer-B to 45% buffer-A/ 55% B was run over 6 min at a flow rate of 0.85 ml per minute. The QC-RT-PCR product of the competitor RNA being 18 base pairs smaller is eluted from the HPLC column before the QC-RT-PCR product from the *CYP52A5* mRNA(U). The amount of the QC-RT-PCR products are plotted and quantitated with an attached Waters Corporation 745 data module. The log ratios of the amount of *CYP52A5* mRNA QC-RT-PCR product (U) to competitor QC-RT-PCR product (C), as measured by peak areas, was plotted and the amount of competitor RNA required to equal the amount of *CYP52A5* mRNA product determined. In the case of each of the target genes listed in Table 5, the competitor RNA contained fewer base pairs as compared to the native target mRNA and eluted before the native mRNA in a manner similar to that demonstrated by *CYP52A5.* HPLC quantification of the genes was conducted as above.

### EXAMPLE 12

### Evaluation of New Strains in Shake Flasks

The *CYP* and *CPR* amplified strains such as strains HDC10, HDC15, HDC20 and HDC23 (Table 1) and H5343 were evaluated for diacid production in shake flasks. A single colony for each strain was transferred from a YPD agar plate into 5 ml of YPD broth and grown overnight at 30°C, 250 rpm. An inoculum was then transferred into 50 ml of DCA2 medium (Chart) and grown for 24 h at 30°C, 300 rpm. The cells were centrifuged at 5000 rpm for 5 min and resuspended in 50 ml of DCA3 medium (Chart) and grown for 24 h at 30°C, 300 rpm. 3% oleic acid w/v was added after 24 h growth in DCA3 medium and the cultures were allowed to bioconvert oleic acid for 48 h. Samples were harvested and the diacid and monoacid concentrations were analyzed as per the scheme given in Figure 35. Each strain was tested in duplicate and the results shown in Table 8 represent the average value from two flasks.

**Table 8. Bioconversion of oleic acid by different recombinant strains of Candida tropicalis**

| Strain | Conversion to Oleic diacid (%) | Specific Conversion (g diacid/g biomass |
|---|---|---|
| H5343 | 41.9 | 0.53 |
| HDC 10-2 | 50.5 | 0.85 |
| HDC 15 | 54.4 | 0.85 |
| HDC 20-1 | 45.1 | 0.72 |
| HDC 20-2 | 45.3 | 0.58 |
| HDC 23-2 | 55.2 | 0.84 |
| HDC 23-3 | 58.8 | 0.89 |

### EXAMPLE 13

### Cloning and Characterization of C. tropicalis 20336 Cytochrome P450 Monooxygenase (CYP) and Cytochrome P450 NADPH Oxidoreductase (CPR) Genes

To clone *CYP* and *CPR* genes several different strategies were employed. Available CYP amino acid sequences were aligned and regions of similarity were observed (Figure 4). These regions corresponded to described conserved regions seen in other cytochrome P450 families (Goeptar et al., *supra* and Kalb et al. *supra*). Proteins from eight eukaryotic cytochrome P450 families share a segmented region of sequence similarity. One region corresponded to the HR2 domain containing the invariant cysteine residue near the carboxyl terminus which is required for heme binding while the other region corresponded to the central region of the I helix thought to be involved in substrate recognition (Figure 4). Degenerate oligonucleotide primers corresponding to these highly conserved regions of the *CYP52* gene family present in *Candida maltosa* and *Candida tropicalis* ATCC 750 were designed and used to amplify DNA fragments of *CYP* genes from *C tropicalis* 20336 genomic DNA. These discrete PCR fragments were then used as probes to isolate full-length *CYP* genes from the C. *tropicalis* 20336 genomic libraries. In a few instances oligonucleotide primers corresponding to highly conserved regions were directly used as probes to isolate full-length *CYP* genes from genomic libraries. In the case of *CPR* a heterologous probe based upon the known DNA sequence for the *CPR* gene from *C*. *tropicalis* 750 was used to isolate the *C*. *tropicalis* 20336 *CPR* gene.

### A. Cloning of the CPR Gene from C tropicalis 20336

### 1) Cloning of the CPRA Allele

Approximately 25,000 phage particles from the first genomic library of *C*. *tropicalis* 20336 were screened with a 1.9 kb *Bam*HI-*Nde*I fragment from plasmid pCU3RED (See Picattagio et al., Bio/Technology 10:894-898 (1992) containing most of the *C*. *tropicalis* 750 *CPR* gene. Five clones that hybridized to the probe were isolated and the plasmid DNA from these lambda clones was rescued and characterized by restriction enzyme analysis. The restriction enzyme analysis suggested that all five clones were identical but it was not clear that a complete *CPR* gene was present.

PCR analysis was used to determine if a complete *CPR* gene was present in any of the five clones. Degenerate primers were prepared for highly conserved regions of known *CPR* genes (See Sutter et al., J. Biol. Chem. 265:16428-16436 (1990)) ( Figure 4). Two Primers were synthesized for the FMN binding region (FMN1, SEQ ID NO: 16 and FMN2, SEQ ID NO: 17). One primer was synthesized for the FAD binding region (FAD, SEQ ID NO: 18), and one primer for the NADPH binding region (NADPH, SEQ - ID NO: 19) (Table 4). These four primers were used in PCR amplification experiments using as a template plasmid DNA isolated from four of the five clones described above. The FMN (SEQ ID NOS: 16 and 17) and FAD (SEQ ID NO: 18) primers served as forward primers and the NADPH primer (SEQ ID NO: 19) as the reverse primer in the PCR reactions. When different combinations of forward and reverse primers were used, no PCR products were obtained from any of the plasmids. However, all primer combinations amplified expected size products with a plasmid containing the *C*. *tropicalis* 750 *CPR* gene (positive control). The most likely reason for the failure of the primer pairs to amplify a product, was that all four of clones contained a truncated *CPR* gene. One of the four clones (pHKM1) was sequenced using the Triplex 5' (SEQ ID NO: 30) and the Triplex 3' (SEQ ID NO: 31) primers (Table 4) which flank the insert and the multiple cloning site on the cloning vector, and with the degenerate primer based upon the NADPH binding site described above. The NADPH primer (SEQ ID NO: 19) failed to yield any sequence data and this is consistent with the PCR analysis. Sequences obtained with Triplex primers were compared with *C*. *tropicalis* 750 *CPR* sequence using the MacVector^{™} program (Oxford Molecular Group, Campbell, CA). Sequence obtained with the Triplex 3' primer-(SEQ ID NO: 31) showed similarity to an internal sequence of the *C*. *tropicalis* 750 *CPR* gene confirming that pHKM1 contained a truncated version of a 20336 *CPR* gene. pHKM1 had a 3.8 kb insert which included a 1.2 kb coding region of the *CPR* gene accompanied by 2.5 kb of upstream DNA (Figure 5). Approximately 0.85 kb of the 20336 *CPR* gene encoding the C-terminal portion of the *CPR* protein is missing from this clone.

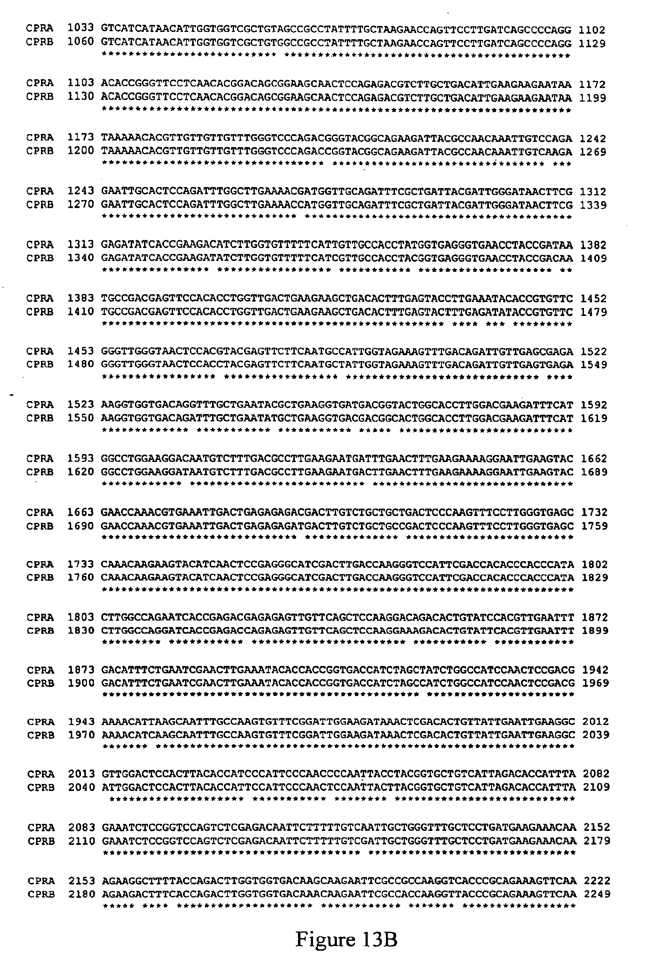

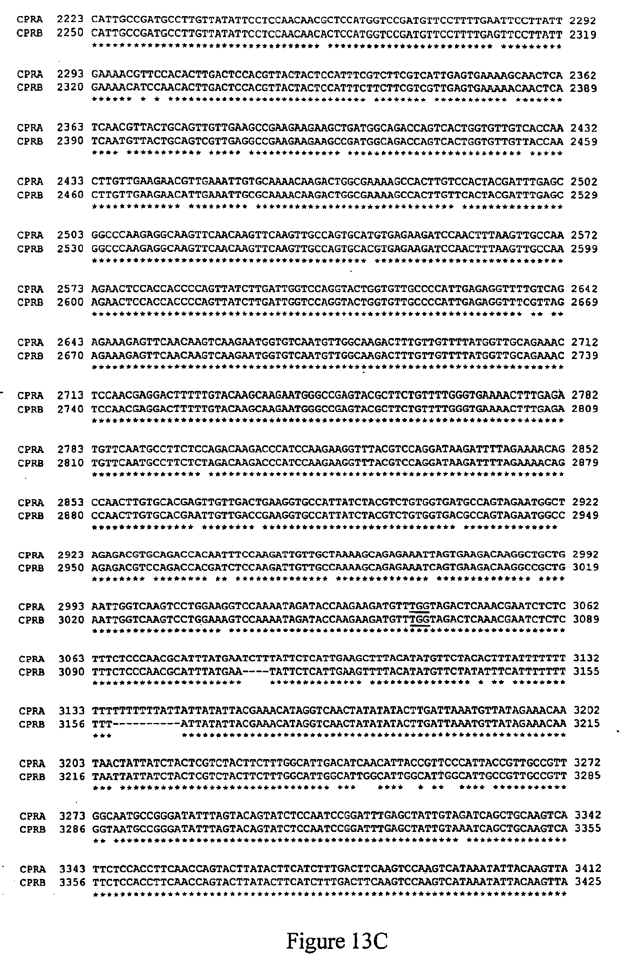

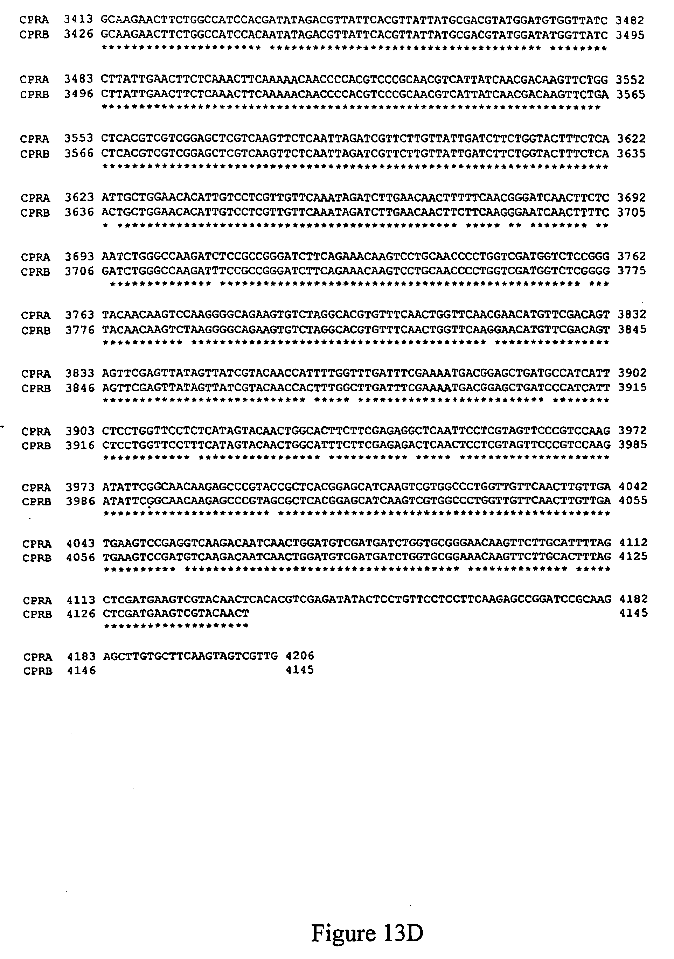

Since the first Clontech library yielded only a truncated *CPR* gene, the second library prepared by Clontech was screened to isolate a full-length *CPR* gene. Three putative *CPR* clones were obtained. The three clones, having inserts in the range of 5-7 kb, were designated pHKM2, pHKM3 and pHKM4. All three were characterized by PCR using the degenerate primers described above. Both pHKM2 and pHKM4 gave PCR products with two sets of internal primers. pHKM3 gave a PCR product only with the FAD (SEQ ID NO: 18) and NADPH (SEQ ID NO: 19) primers suggesting that this clone likely contained a truncated *CPR* gene. All three plasmids were partially sequenced using the two Triplex primers and a third primer whose sequence was selected from the DNA sequence near the truncated end of the *CPR* gene present in pHKM1. This analysis confirmed that both pHKM2 & 4 have sequences that overlap pHKM1 and that both contained the 3' region of *CPR* gene that is missing from pHKM1. Portions of inserts from pHKM1 and pHKM4 were sequenced and a full-length *CPR* gene was identified. Based on the DNA sequence and PCR analysis, it was concluded that pHKM1 contained the putative promoter region and 1.2 kb of sequence encoding a portion (5' end) of a *CPR* gene. pHKM4 had 1.1 kb of DNA that overlapped pHKM1 and contained the remainder (3' end) of a *CPR* gene along with a downstream untranslated region (Figure 6). Together these two plasmids contained a complete *CPRA* gene with an upstream promoter region. *CPRA* is 4206 nucleotides in length (SEQ ID NO: 81) and includes a regulatory region and a protein coding region (defined by nucleotides 1006-3042) which is 2037 base pairs in length and codes for a putative protein of 679 amino acids (SEQ ID NO: 83) (Figures 13 and 14). In Figure 13, the asterisks denote conserved nucleotides between *CPRA* and *CPRB,* bold denotes protein coding nucleotides, and the start and stop codons are underlined. The *CPRA* protein, when analyzed by the protein alignment program of the GeneWorks^{™} software package (Oxford Molecular Group, Campbell, CA), showed extensive homology to *CPR* proteins from C. *tropicalis* 750 and *C*. *maltosa.*

### 2) Cloning of the CPRB Allele

To clone the second *CPRB* allele, the third genomic library, prepared by Henkel, was screened using DNA fragments from pHKM1 and pHKM4 as probes. Five clones were obtained and these were sequenced with the three internal primers used to sequence *CPRA.* These primers were designated PRK1.F3 (SEQ ID NO: 20), PRK1.F5 (SEQ ID NO: 21) and PRK4.R20 (SEQ ID NO: 22) (Table 4). and the two outside primers (M13 -20 and T3 [Stratagene]) for the polylinker region present in the pBK-CMV cloning vector. Sequence analysis suggested that four of these clones, designated pHKM5 to 8, contained inserts which were identical to the *CPRA* allele isolated earlier. All four seemed to contain a full length *CPR* gene. The fifth clone was very similar to the *CPRA* allele, especially in the open reading frame region where the identity was very high. However, there were significant differences in the 5' and 3' untranslated regions. This suggested that the fifth clone was the allele to *CPRA.* The plasmid was designated pHKM9 (Figure 7) and a 4.14 kb region of this plasmid was sequenced and the analysis of this sequence confirmed the presence of the *CPRB.* allele (SEQ ID NO: 82), which includes a regulatory region and a protein coding region (defined by nucleotides 1033-3069) (Figure 13). The amino acid sequence of the *CPRB* protein is set forth in SEQ ID NO: 84 (Figure 14).

### B. Cloning of C. tropicalis 20336 (CYP) Genes

### 1) Cloning of CYP52A2A, CYP52A3A & 3B and CYP52A5A & 5B

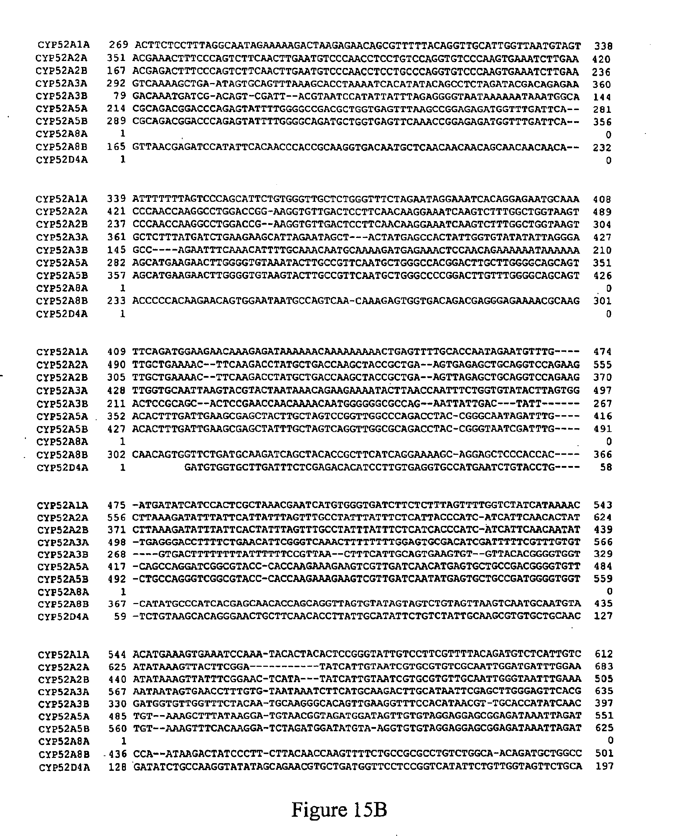

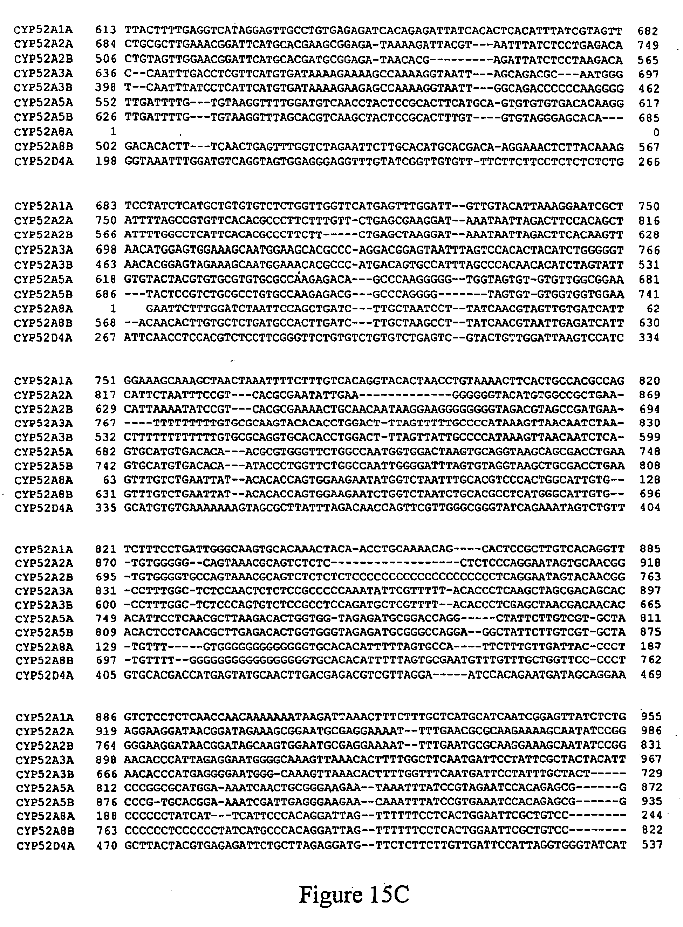

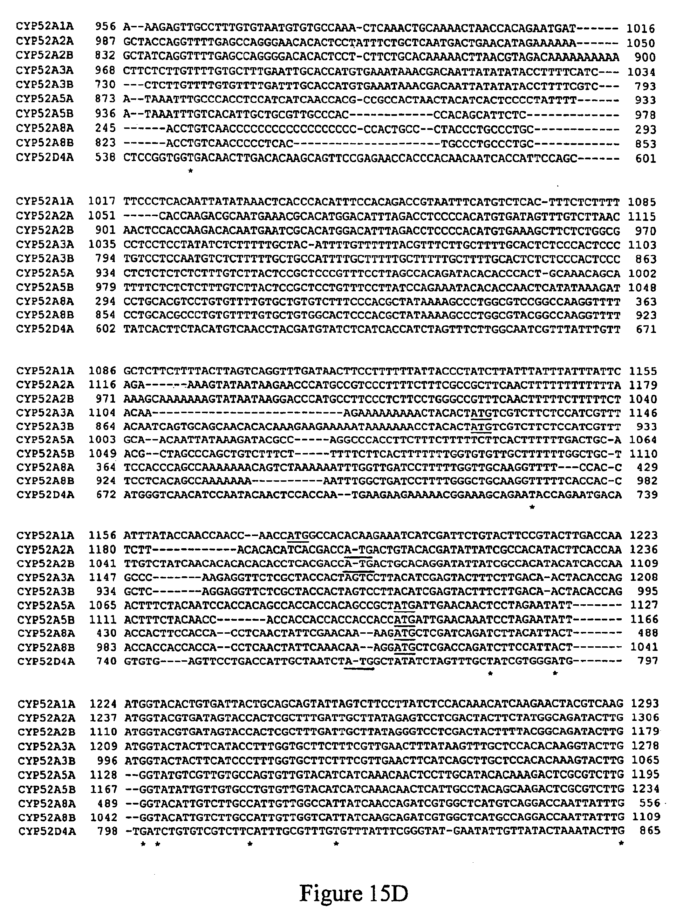

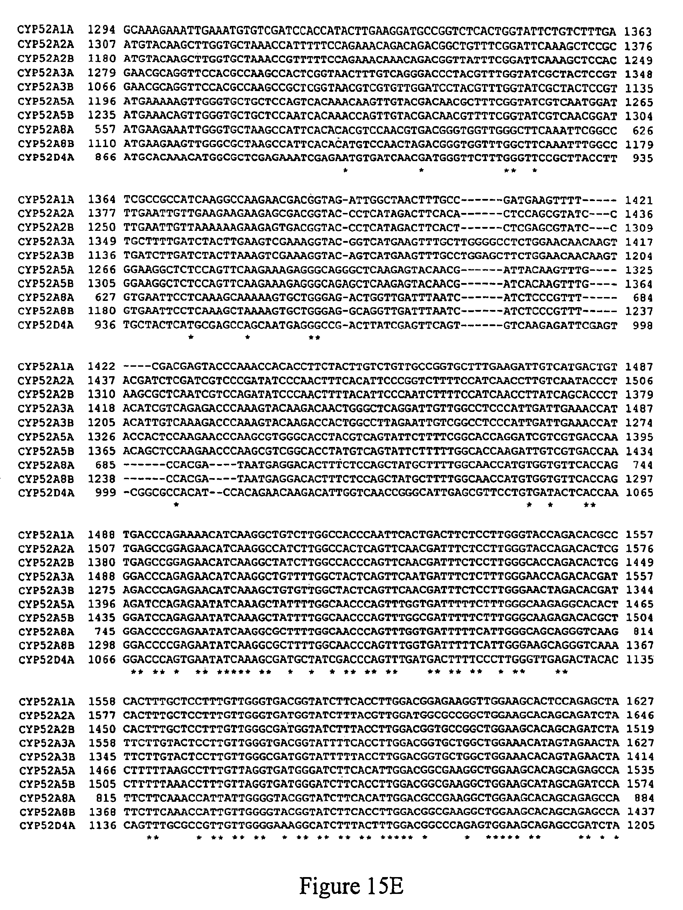

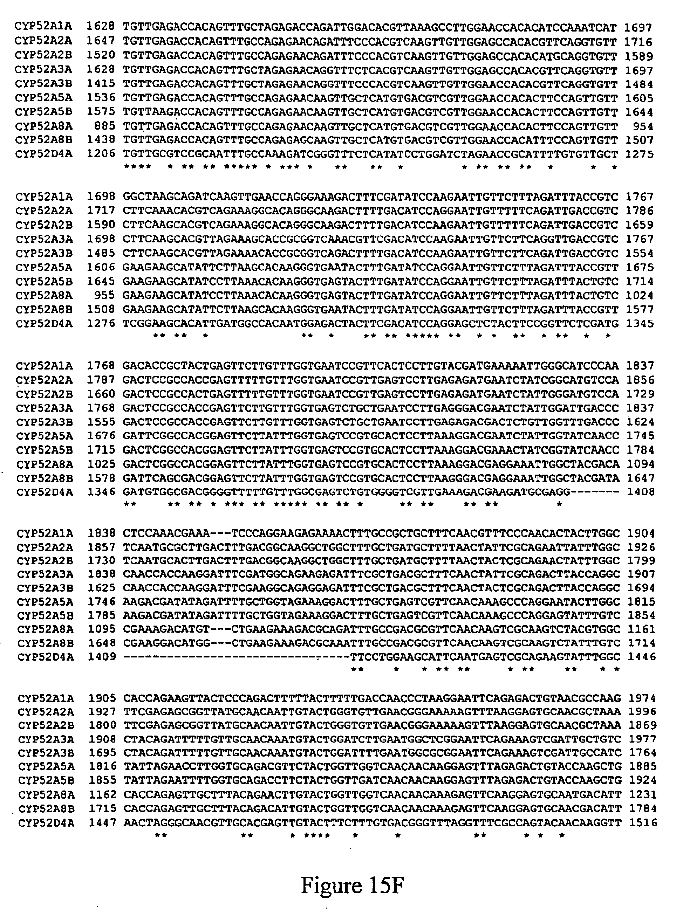

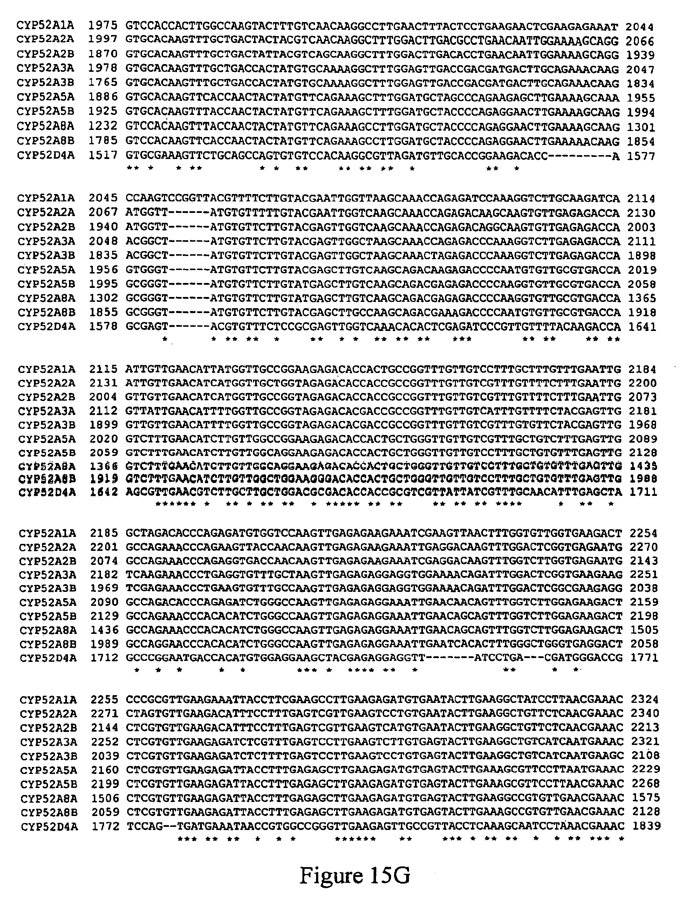

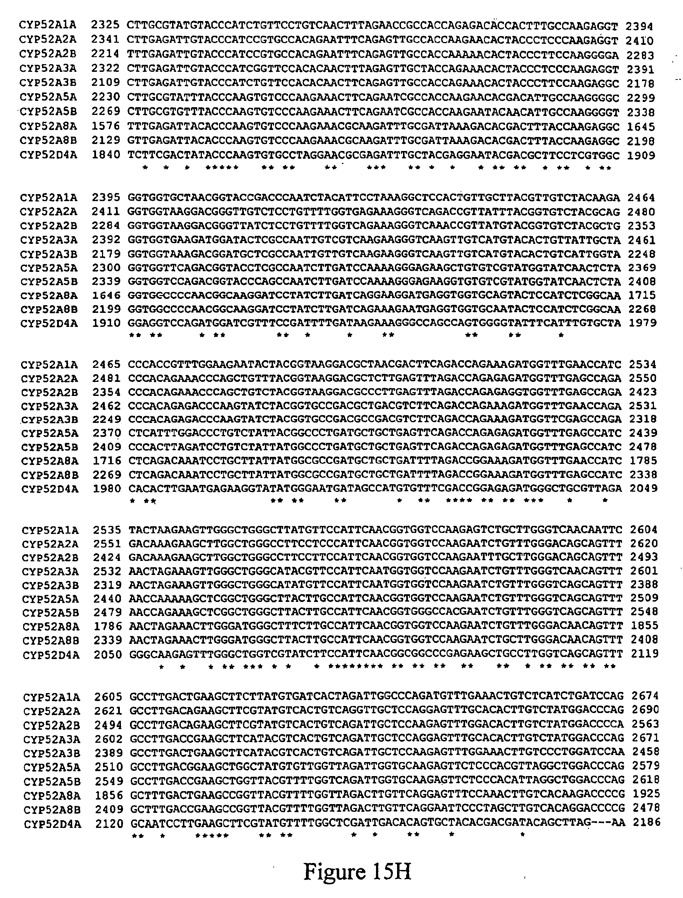

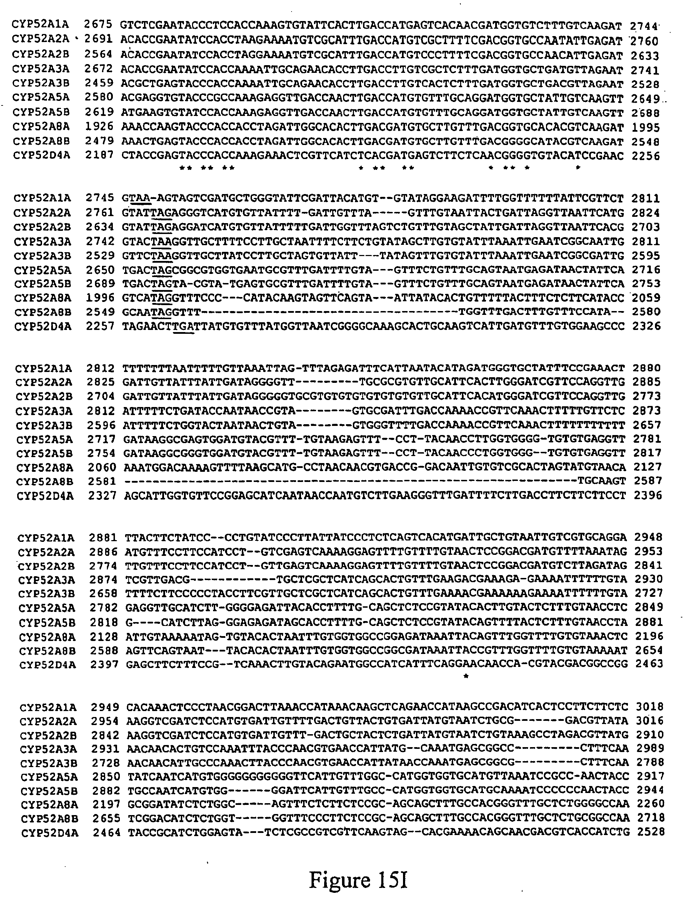

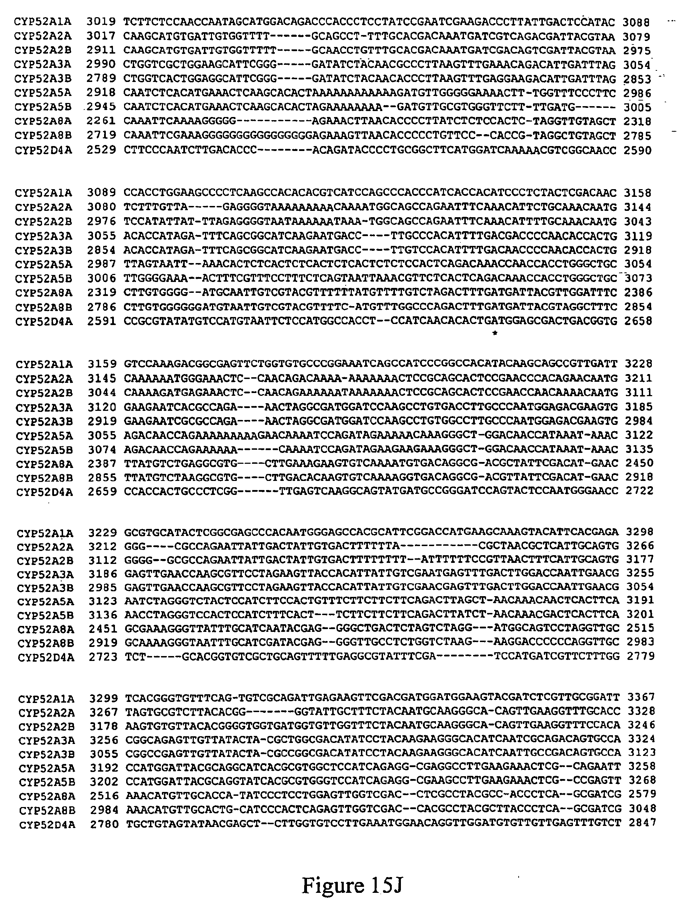

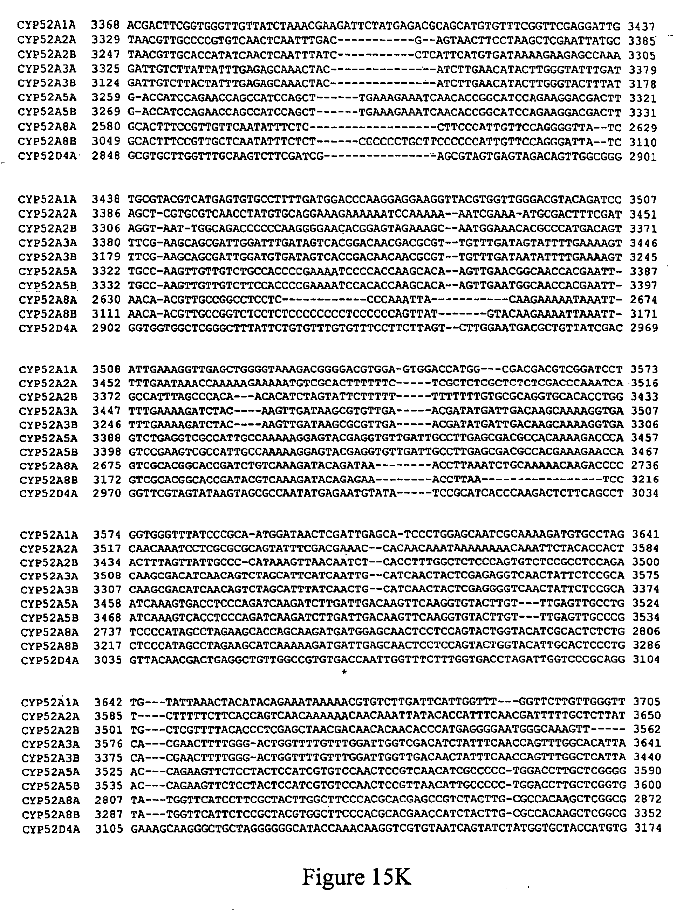

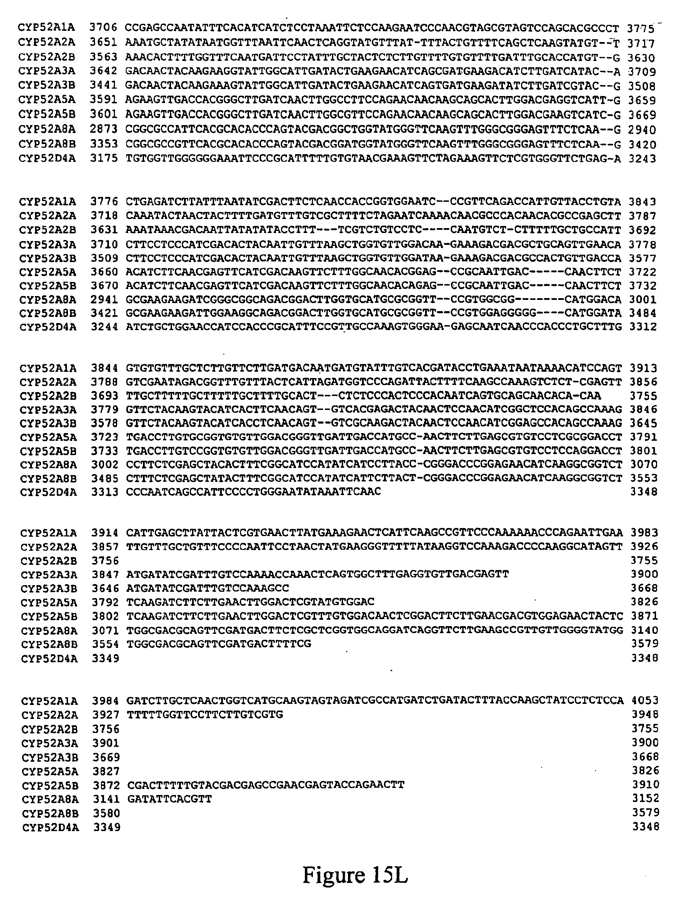

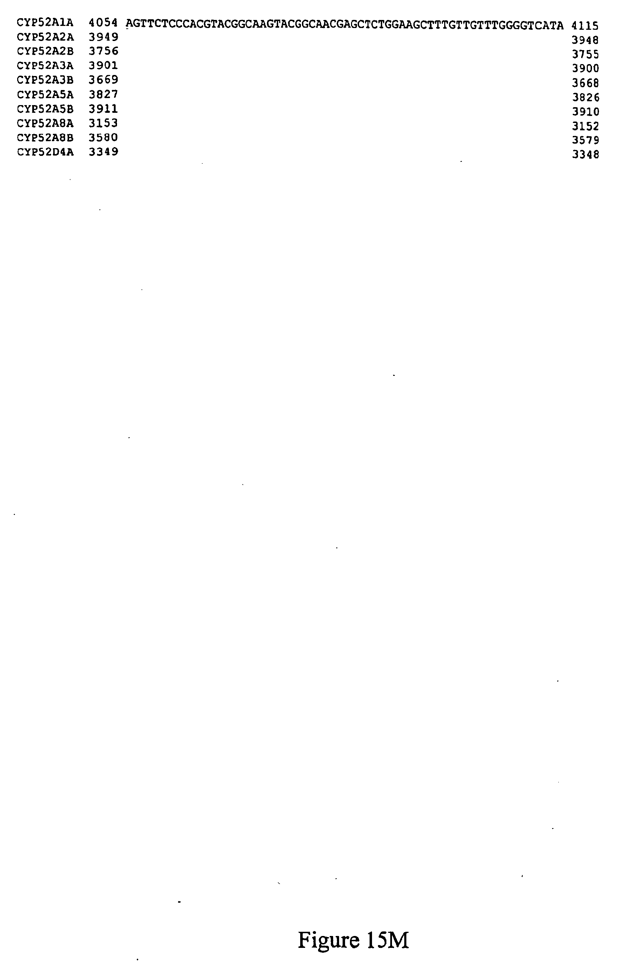

Clones carrying *CYP52A2A, A3A, A3B, A5A* and *A5B* genes were isolated from the first and second Clontech genomic libraries using an oligonucleotide probe (HemeB1, SEQ ID NO: 27) whose sequence was based upon the amino acid sequence for the highly conserved heme binding region present throughout the *CYP52* family. The first and second libraries were converted to the plasmid form and screened by colony hybridizations using the HemeB1 probe (SEQ ID NO: 27) (Table 4). Several potential clones were isolated and the plasmid DNA was isolated from these clones and sequenced using the HemeB 1 oligonucleotide (SEQ ID NO: 27) as a primer. This approach succeeded in identifying five *CYP52* genes. Three of the *CYP* genes appeared unique, while the remaining two were classified as alleles. Based upon an arbitrary choice of homology to *CYP52* genes from *Candida maltosa,* these five genes and corresponding plasmids were designated *CYP52A2A* (pPA15 [Figure 26]), *CYP52A3A* (pPA57 [Figure 29]), *CYP52A3B* (pPA62 [Figure 30]), *CYP52A5A* (pPAL3 [Figure 31]) and *CYP52A5B* (pPA5 [Figure 32]). The complete DNA sequence including regulatory and protein coding regions of these five genes was obtained and confirmed that all five were *CYP52* genes (Figure 15). In Figure 15, the asterisks denote conserved nucleotides among the *CYP* genes. Bold indicates the protein coding nucleotides of the *CYP* genes, and the start and stop codons are underlined. The *CYP52A2A* gene as represented by SEQ ID NO: 86 has a protein coding region defined by nucleotides 1199-2767 and the encoded protein has an amino acid sequence as set forth in SEQ ID NO: 96. The *CYP52A3A* gene as represented by SEQ ID NO: 88 has a protein encoding region defined by nucleotides 1126-2748 and the encoded protein has an amino acid sequence as set forth in SEQ ID NO: 98. The *CYP52A3B* gene as represented by SEQ ID NO: 89 has a protein coding defined by nucleotides 913-2535 and the encoded protein has an amino acid sequence as set forth in SEQ ID NO: 99. The *CYP52A5A* gene as represented by SEQ ID NO: 90 has a protein coding region defined by nucleotides 1103-2656 and the encoded protein has an amino acid sequence as set forth in SEQ ID NO: 100. The *CYP52A5B* gene as represented by SEQ ID NO: 91 has a protein coding region defined by nucleotides 1142-2695 and the encoded protein has an amino acid sequence as set forth in SEQ ID NO: 101.

### 2) Cloning of CYP542A1A and CYP52A8A

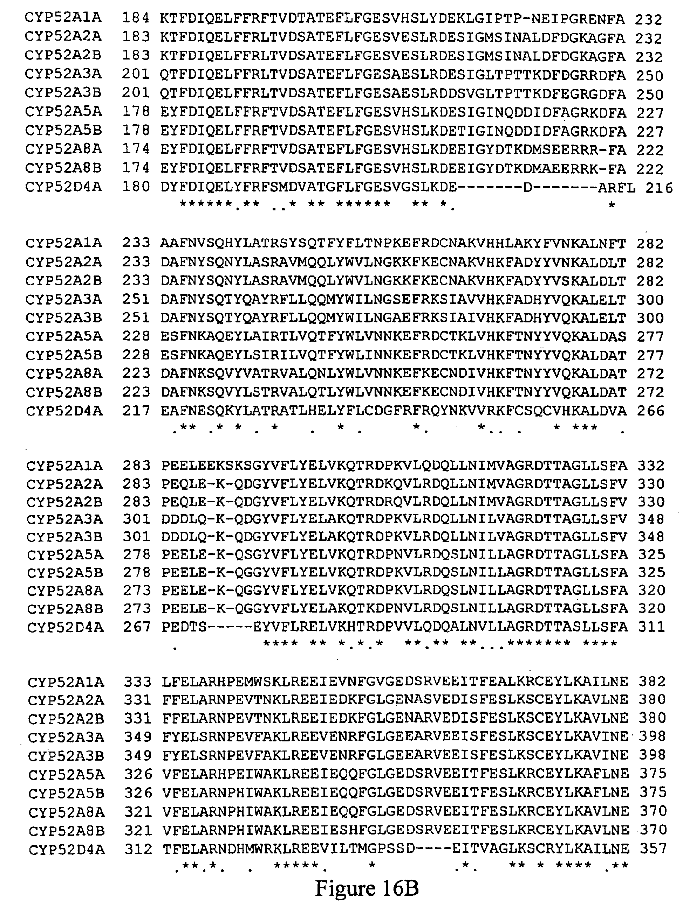

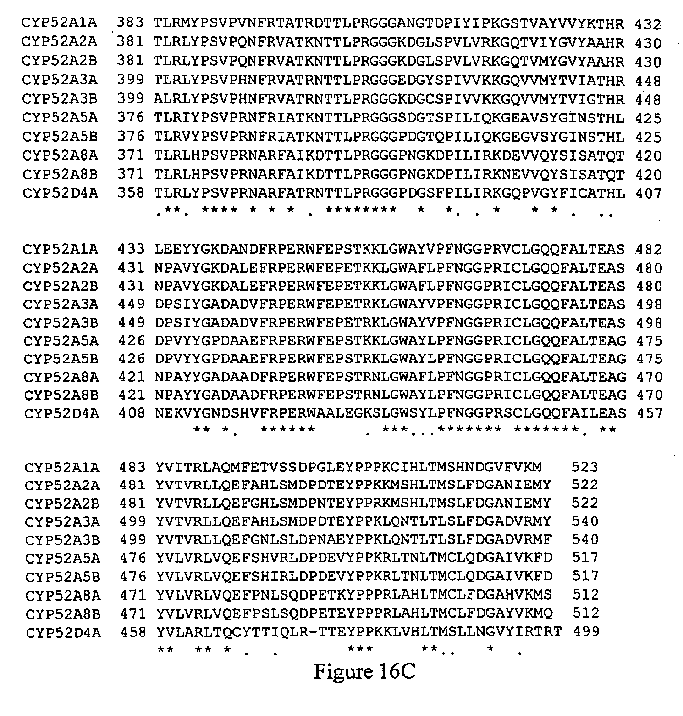

*CYP52A1A* and *CYP52A8A* genes were isolated from the third genomic library using PCR fragments as probes. The PCR fragment probe for *CYP52A1* was generated after PCR amplification of 20336 genomic DNA with oligonucleotide primers that were designed to amplify a region from the Helix I region to the HR2 region using all available *CYP52* genes from National Center for Biotechnology Information. Degenerate forward primers UCup1 (SEQ ID NO: 23) and UCup2 (SEQ ID NO: 24) were designed based upon an amino acid sequence (-RDTTAG-) from the Helix I region (Table 4). Degenerate primers UCdown1 (SEQ ID NO: 25) and UCdown2 (SEQ ID NO: 26) were designed based upon an amino acid sequence (-GQQFAL- ) from the HR2 region (Table 4). For the reverse primers, the DNA sequence represents the reverse complement of the corresponding amino acid sequence. These primers were used in pairwise combinations in a PCR reaction with Stoffel *Taq* DNA polymerase (Perkin-Elmer Cetus, Foster City, CA) according to the manufacturer's recommended procedure. A PCR product of approximately 450 bp was obtained. This product was purified from agarose gel using Gene-clean^{™} (Bio 101, LaJolla, CA) and ligated to the pTAG^{™} vector (Figure 17) (R&D systems, Minneapolis, MN) according to the recommendations of the manufacturer. No treatment was necessary to clone into pTAG because it employs the use of the TA cloning technique. Plasmids from several transformants were isolated and their inserts were characterized. One plasmid contained the PCR clone intact. The DNA sequence of the PCR fragment (designated *44CYP3,* SEQ ID NO: 107) shared homology with the DNA sequences for the *CYP52A1* gene of *C*. *maltosa* and the *CYP52A3* gene of *C*. *tropicalis 750.* This fragment was used as a probe in isolating the *C*. *tropicalis* 20336 *CYP52A1* homolog. The third genomic library was screened using the *44CYP3* PCR probe (SEQ ID NO: 107) and a clone (pHKM11) that contained a full-length *CYP52* gene was obtained (Figure 8). The clone contained a gene having regulatory and protein coding regions. An open reading frame of 1572 nucleotides encoded a *CYP52* protein of 523 amino acids (Figures 15 and 16). This *CYP52* gene was designated *CYP52A1A* (SEQ ID NO: 85) since its putative amino acid sequence (SEQ ID NO: 95) was most similar to the *CYP52A1* protein of C. *maltosa.* The protein coding region of the *CYP53A1A* gene is defined by nucleotides 1177-2748 of SEQ ID NO: 85.

A similar approach was taken to clone *CYP52A8A.* A PCR fragment probe for *CYP52A8* was generated using primers for highly conserved sequences of *CYP52A3, CYP52A2* and *CYP52A5* genes of *C*. *tropicalis 750.* The reverse primer (primer 2,3,5,M) (SEQ ID NO: 29) was designed based on the highly conserved heme binding region (Table 4). The design of the forward primer (primer 2,3,5,P) (SEQ ID NO: 28) was based upon a sequence conserved near the N-terminus of the *CYP52A3, CYP52A2* and *CYP52A5* genes from *C*. *tropicalis 750* (Table 4). Amplification of 20336 genomic DNA with these two primers gave a mixed PCR product. One amplified PCR fragment was 1006 bp long (designated DCA1002). The DNA sequence for this fragment was determined and was found to have 85% identity to the DNA sequence for the *CYP52D4* gene of *C*. *tropicalis* 750. When this PCR product was used to screen the third genomic library one clone (pHKM12) was identified that contained a full-length *CYP52* gene along with 5' and 3' flanking sequences (Figure 9). The *CYP52* gene included regulatory and protein coding regions with an open reading frame of 1539 nucleotides long which encoded a putative CYP52 protein of 512 amino acids (Figures 15 and 16). This gene was designated as *CYP52A8A* (SEQ ID.NO: 92) since its amino acid sequence (SEQ ID NO: 102) was most similar to the *CYP52A8* protein of *C*. *maltosa.* The protein coding region of the *CYP52A8A* gene is defined by nucleotides 464-2002 of SEQ ID NO: 92. The amino acid sequence of the *CYP52A8A* protein is set forth in SEQ ID NO: 102.

### 3) Cloning of CYP52D4A

The screening of the second genomic library with the HemeB1 (SEQ ID NO: 27) primer (Table 4) yielded a clone carrying a plasmid (pPA18) that contained a truncated gene having homology with the *CYP52D4* gene of *C*. *maltosa* (Figure 33). A 1.3 to 1.5-kb *Eco*RI-*Sst*I fragment from pPA18 containing part of the truncated *CYP* gene was isolated and used as a probe to screen the third genomic library for a full length *CYP52* gene. One clone (pHKM23) was isolated and found to contain a full-length *CYP* gene with extensive 5' and 3' flanking sequences (Figure 10). This gene has been designated as *CYP52D4A* (SEQ ID NO: 94) and the complete DNA including regulatory and protein coding regions (coding region defined by nucleotides 767-2266) and putative amino acid sequence (SEQ ID NO: 104) of this gene is shown in Figures 15 and 16. *CYP52D4A* (SEQ ID NO: 94) shares the greatest homology with the *CYP52D4* gene of *C. maltosa.*

### 4) Cloning of CYP52A2B and CYP5248B

A mixed probe containing *CYP52A1A, A2A, A3A, D4A, A5A* and *A8A* genes was used to screen the third genomic library and several putative positive clones were identified. Seven of these were sequenced with the degenerate primers Cyp52a (SEQ ID NO: 32), Cyp52b (SEQ ID NO: 33), Cyp52c (SEQ ID NO: 34) and Cyp52d (SEQ ID NO: 35) shown in Table 4. These primers were designed from highly conserved regions of the four *CYP52* subfamilies, namely *CYP52A, B, C & D.* Sequences from two clones, pHKM14 and pHKM15 (Figures 11 and 12), shared considerable homology with DNA sequence of the *C*. *tropicalis* 20336 *CYP52A2* and *CYP52A8* genes, respectively. The complete DNA (SEQ ID NO: 87) including regulatory and protein coding regions (coding region defined by nucleotides 1072-2640) and putative amino acid sequence (SEQ ID NO: 97) of the *CYP52* gene present in pHKM14 suggested that it is *CYP52A2B* (Figures 15 and 16). The complete DNA (SEQ ID NO: 93) including regulatory and protein coding regions (coding region defined by nucleotides 1017-2555) and putative amino acid sequence (SEQ ID NO: 103) of the *CYP52* gene present in pHKM15 suggested that it is *CYP52A8B* (Figures 15 and 16).

### EXAMPLE 14

### Identification of CYP and CPR Genes Induced by Selected Fatty Acid and Alkane Substrates

Genes whose transcription is turned on by the presence of selected fatty acid or alkane substrates have been identified using the QC-RT-PCR assay. This assay was used to measure (*CYP*) and (*CPR*) gene expression in fermentor grown cultures *C*. *tropicalis* ATCC 20962. This method involves the isolation of total cellular RNA from cultures of *C*. *tropicalis* and the quantification of a specific mRNA within that sample through the design and use of sequence specific QC-RT-PCR primers and an RNA competitor. Quantification is achieved through the use of known concentrations of highly homologous competitor RNA in the QC-RT-PCR reactions. The resulting QC-RT-PCR amplified cDNA's are separated and quantitated through the use of ion pairing reverse phase HPLC. This assay was used to characterize the expression of *CYP52* genes of C. *tropicalis* ATCC 20962 in response to various fatty acid and alkane substrates. Genes which were induced were identified by the calculation of their mRNA concentration at various times before and after induction. Figure 18 provides an example of how the concentration of mRNA for *CYP52A5* can be calculated using the QC-RT-PCR assay. The log ratio of unknown (U) to competitor product (C) is plotted versus the concentration of competitor RNA present in the QC-RT-PCR reactions. The concentration of competitor which results in a log ratio of U/C of zero, represents the point where the unknown messenger RNA concentration is equal to the concentration of the competitor. Figure 18 allows for the calculation of the amount of *CYP52A5* message present in 100 ng of total RNA isolated from cell samples taken at 0, 1, and 2 hours after the addition of Emersol® 267 in a fermentor run. From this analysis, it is possible to determine the concentration of the *CYP52A5* mRNA present in 100 ng of total cellular RNA. In the plot contained in Figure 18 it takes 0.46 pg of competitor to equal the number of mRNA's of *CYP52A5* in 100 ng of RNA isolated from cells just prior (time 0) to the addition of the substrate, Emersol® 267. In cell samples taken at one and two hours after the addition of Emersol® 267 it takes 5.5 and 8.5 pg of competitor RNA, respectively. This result demonstrates that *CYP52A5* (SEQ ID NOS: 90 and 91) is induced more than 18 fold within two hours after the addition of Emersol® 267. This type of analysis was used to demonstrate that *CYP52A5* (SEQ ID NO: 90 and 91) is induced by Emersol® 267. Figure 19 shows the relative amounts *of CYP52A5* (SEQ ID NOS: 90 and 91) expression in fermentor runs with and without Emersol® 267 as a substrate. The differences in the *CYP52A5* (SEQ. ID NOS: 90 and 91) expression patterns are due to the addition of Emersol® 267 to the fermentation medium.

This analysis clearly demonstrates that expression of *CYP52A5* (SEQ ID NOS: 90 and 91) in *C*. *tropicalis* 20962 is inducible by the addition of Emersol® 267 to the growth medium. This analysis was performed to characterize the expression of *CYP52A2A* (SEQ ID NO: 86), *CYP52A3AB* (SEQ ID NOS: 88 and 89), *CYP52A8A* (SEQ ID NO: 92), *CYP52A1A* (SEQ ID NO: 85), *CYP52D4A* (SEQ ID NO: 94) and *CPRB* (SEQ ID NO: 82) in response to the presence of Emersol® 267 in the fermentation medium (Figure 20). The results of these analysis' indicate, that like the *CYP52A5* gene (SEQ ID NOS: 90 and 91) of *C*. *tropicalis* 20962, the *CYP52A2A* gene (SEQ ID NO: 86) is inducible by Emersol® 267. A small induction is observed for *CYP52A1A* (SEQ ID NO: 85) and *CYP52A8A* (SEQ ID NO: 92). In contrast, any induction for *CYP52D4A* (SEQ ID NO, 94), *CYP52A3A* (SEQ ID NO: 88), *CYP52A3B* (SEQ ID NO: 89) is below the level of detection of the assay. *CPRB* (SEQ ID NO: 82) is moderately induced by Emersol® 267, four to five fold. The results of these analysis are summarized in Figure 20. Figure 34 provides an example of selective induction of *CYP52A* genes. When pure fatty acid or alkanes are spiked into a fermentor containing *C*. *tropicalis* 20962 or a derivative thereof, the transcriptional activation of *CYP52A* genes was detected using the QC-RT-PCR assay. Figure 34 shows that pure oleic acid (C18:1) strongly induces *CYP52A2A* (SEQ ID NO: 86) while inducing *CYP52A5* (SEQ ID NOS: 90 and 91). In the same fermentor addition of pure alkane (tridecane) shows strong induction of both *CYP52A2A* (SEQ ID NO: 86) and *CYP52A1A* (SEQ ID NO: 85). However, tridecane did not induce *CYP52A5* (SEQ ID NOS: 90 and 91). In a separate fermentation using ATCC 20962, containing pure octadecane as the substrate, induction of *CYP52A2A, CYP52A5A* and *CYP52A1A* is detected (see Figure 36). The foregoing demonstrates selective induction of particular *CYP* genes by specific substrates, thus providing techniques for selective metabolic engineering of cell strains. For example, if tridecane modification is desired, organisms engineered for high levels of *CYP52A2A* (SEQ ID NO: 86) and *CYP52A1A* (SEQ ID NO: 85) activity are indicated. If oleic acid modification is desired, organisms engineered for high levels of *CYP52A2A* (SEQ ID NO: 86) activity are indicated.

### EXAMPLE 15

### Integration of Selected CYP and CPR Genes into the Genome of Candida tropicalis

In order to integrate selected genes into the chromosome of *C*. *tropicalis* 20336 or its descendants, there has to be a target DNA sequence, which may or may not be an intact gene, into which the genes can be inserted. There must also be a method to select for the integration event. In some cases the target DNA sequence and the selectable marker are the same and, if so, then there must also be a method to regain use of the target gene as a selectable marker following the integration event. In *C*. *tropicalis* and its descendants, one gene which fits these criteria is *URA3A,* encoding orotidine-5'-phosphate decarboxylase. Using it as a target for integration, *ur*a variants of *C. tropicalis* can be transformed in such a way as to regenerate a *URA*⁺ genotype via homologous recombination (Figure 21). Depending upon the design of the integration vector, one or more genes can be integrated into the genome at the same time. Using a split *URA3A* gene oriented as shown in Figure 22, homologous integration would yield at least one copy of the gene(s) of interest which are inserted between the split portions of the *URA3A* gene. Moreover, because of the high sequence similarity between *URA3A* and *URA3B* genes, integration of the construct can occur at both the *URA3A* and *URA3B* loci. Subsequently, an oligonucleotide designed with a deletion in a portion of the *URA* gene based on the identical sequence across both the *URA3A* and *URA3B* genes, can be utilized to yield *C*. *tropicalis* transformants which are once again ura but which still carry one or more newly integrated genes of choice (Figure 21). *ura*⁻ variants of *C*. *tropicalis* can also be isolated via other methods such as classical mutagenesis or by spontaneous mutation. Using well established protocols, selection of *ura* strains can be facilitated by the use of 5-fluomorotic acid (5-FOA) as described, e.g., in Boeke et al., Mol. Gen. Genet. 197:345-346, (1984). The utility of this approach for the manipulation of C. *tropicalis* has been well documented as described, e.g., in Picataggio et al., Mol. and Cell. Biol. 11:4333-4339 (1991); Rohrer et al., Appl. Microbiol. Biotechnol. 36:650-654 (1992); Picataggio et al., Bio/Technology 10:894-898 (1992); U.S. Patent No. 5,648,247; U.S. Patent No. 5,620,878; U.S. Patent No. 5,204,252; U.S. Patent No. 5,254,466.

### A. Construction of a URA Integration Vector, pURAin.

Primers were designed and synthesized based on the 1712 bp sequence of the *URA3A* gene of *C*. *tropicalis* 20336 (see Figure 23). The nucleotide sequence of the *URA3A* gene of *C*. *tropicalis* 20336 is set forth in SEQ ID NO: 105 and the amino acid sequence of the encoded protein is set forth in SEQ ID NO: 106. *URA3A* Primer Set #1a (SEQ ID NO: 9) and #1b (SEQ ID NO: 10) (Table 4) was used in PCR with *C*. *tropicalis* 20336 genomic DNA to amplify *URA3A* sequences between nucleotide 733 and 1688 as shown in Figure 23. The primers are designed to introduce unique 5' *AscI* and 3' *Pac*I restriction sites into the resulting amplified *URA3A* fragment. *Asc*I and *Pac*I sites were chosen because these sites are not present within *CYP* or *CPR* genes identified to date. *URA3A* Primer Set #2 was used in PCR with C. *tropicalis* 20336 genomic DNA as a template, to amplify *URA3A* sequences between nucleotide 9 and 758 as shown in Figure 23. *URA3A* Primer set #2a (SEQ ID NO: 11) and #2b (SEQ ID NO: 12) (Table 4) was designed to introduce unique 5' *Pac*I and 3' *Pme*I restriction sites into the resulting amplified *URA3A* fragment. The *Pme*I site is also not present within *CYP* and *CPR* genes identified to date. PcR fragments of the *URA3A* gene were purified, restricted with *AscI, Pac*I and *Pme*I restriction enzymes and ligated to a gel purified, QiaexII cleaned *Asc*I*-Pme*I digest of plasmid pNEB193 (Figure 25) purchased from New England Biolabs (Beverly, MA). The ligation was performed with an equimolar number of DNA termini at 16°C for 16 hr using T4 DNA ligase (New England Biolabs). Ligations were transformed into *E. coli* XL1-Blue cells (Stratagene, LaJolla, CA) according to manufacturers recommendations. White colonies were isolated, grown, plasmid DNA isolated and digested with *Asc*I*-Pme*I to confirm insertion of the modified *URA3A* into pNEB193. The resulting base integration vector was named pURAin (Figure 24).

### B. Amplification of CYP52A2A, CYP52A3A, CYP52A5A and CPRB from C. tropicalis 20336 Genomic DNA

The genes encoding *CYP52A2A,* (SEQ ID NO: 86) and *CYP52A3A* (SEQ ID NO: 88) from *C*. *tropicalis* 20336 were amplified from genomic clones (pPA15 and pPA57, respectively) (Figures 26 and 29) via PCR using primers (Primer *CYP* 2A#1, SEQ ID NO: 1 and Primer *CYP* 2A#2, SEQ ID NO: 2 for CYP52A2A) (Primer *CYP* 3A#1, SEQ ID NO: 3 and Primer *CYP* 3A#2, SEQ ID NO: 4 for CYP52A3A) to introduce *Pac*I cloning sites. These PCR primers were designed based upon the DNA sequence determined for *CYP52A2A* (SEQ ID NO: 86) (Figure 15). The Ampli*Taq* Gold PCR kit (Perkin Elmer Cetus, Foster City, CA) was used according to manufacturers specifications. The *CYP52A2A* PCR amplification product was 2,230 base pairs in length, yielding 496 bp of DNA upstream of the *CYP52A2A* start codon and 168 bp downstream of the stop codon for the *CYP52A2A* ORF. The *CYP52A3A* PCR amplification product was 2154 base pairs in length, yielding 437bp of DNA upstream of the *CYP52A3A* start codon and 97bp downstream of the stop codon for the *CYP52A3A ORF.* The *CYP52A3A* PCR amplification product was 2154 base pairs in length, yielding 437bp of DNA upstream of the *CYP52A3A* start codon and 97bp downsteam of the stop codon for the *CYP52A3A* ORF.

The gene encoding *CYP52A5A* (SEQ ID NO: 90) from *C*. *tropicalis* 20336 was amplified from genomic DNA via PCR using primers (Primer *CYP* 5A#1, SEQ ID NO: 5 and Primer *CYP* 5A#2, SEQ ID NO: 6) to introduce *Pac*I cloning sites. These PCR primers were designed based upon the DNA sequence determined for *CYP52A5A* (SEQ ID NO: 90). The Expand Hi-Fi *Taq* PCR kit (Boehringer Mannheim, Indianapolis, IN) was used according to manufacturers specifications. The *CYP52A5A* PCR amplification product was 3,298 base pairs in length.

The gene encoding *CPRB* (SEQ ID NO: 82) from *C*. *tropicalis* 20336 was amplified from genomic DNA via PCR using primers (*CPR* B#1, SEQ ID NO: 7 and *CPR* B#2, SEQ ID NO: 8) based upon the DNA sequence determined for *CPRB* (SEQ ID NO: 82) (Figure 13). These primers were designed to introduce unique *Pac*I cloning sites. The Expand Hi-Fi *Taq* PCR kit (Boehringer Mannheim, Indianapolis, IN) was used according to manufacturers specifications. The *CPRB* PCR product was 3266 bp in length, yielding 747 bp pf DNA upstream of the *CPRB* start codon and 493 bp downstream of the stop codon for the *CPRB* ORF. The resulting PCR products were isolated via agarose gel electrophoresis, purified using QiaexII and digested with *Pac*I. The PCR fragments were purified, desalted and concentrated using a Microcon 100 (Amicon, Beverly, MA).

The above described amplification procedures are applicable to the other genes listed in Table 5 using the respectively indicated primers.

### C. Cloning of CYP and CPR Genes into pURAin

The next step was to clone the selected *CYP* and *CPR* genes into the pURAin integration vector. In particular , no foreign DNA other than that specifically provided by synthetic restriction site sequences are incorporated into the DNA which was cloned into the genome of *C*. *tropicalis,* i.e., with the exception of restriction site DNA only native *C. tropicalis* DNA sequences are incorporated into the genome. pURAin was digested with *Pac*I*,* Qiaex II cleaned, and dephosphorylated with Shrimp Alkaline Phosphatase (SAP) (United States Biochemical, Cleveland, OH) according the manufacturer's recommendations. Approximately 500 ng of *Pac*I linearized pURAin was dephosphorylated for 1 hr at 37°C using SAP at a concentration of 0.2 Units of enzyme per 1 pmol of DNA termini. The reaction was stopped by heat inactivation at 65°C for 20 min.

The *CYP52A2A Pac*I fragment derived using the primer shown in Table 4 was ligated to plasmid pURAin which had also been digested with *Pac*I. *Pac*I digested pURAin was dephosphorylated, and ligated to the *CYP52A2A* ULTMA PCR product as described previously. The ligation mixture was transformed into *E. coli* XL1 Blue MRF' (Stratagene) and 2 resistant colonies were selected and screened for correct constructs which should contain vector sequence, the inverted *URA3A* gene, and the amplified *CYP52A2A* gene (SEQ ID NO: 86) of 20336. *Asc*I-*Pme*I digestion identified one of the two constructs, plasmid pURA2in, as being correct (Figure 27). This plasmid was sequenced and compared to *CYP52A2A* (SEQ ID NO: 86) to confirm that PCR did not introduce DNA base changes that would result in an amino acid change.

Prior to its use, the *CPRB Pac*I fragment derived using the primers shown in Table 4 was sequenced and compared to *CPRB* (SEQ ID NO: 82) to confirm that PCR did not introduce DNA base pair changes that would result in an amino acid change. Following confirmation, *CPRB* (SEQ ID NO: 82) was ligated to plasmid pURAin which had also been digested with *Pac*I*. Pac*I digested pURAin was dephosphorylated, and ligated to the *CPR* Expand Hi-Fi PCR product as described previously. The ligation mixture was transformed into *E. coli* XL1 Blue MRF' (Stratagene) and several resistant colonies were selected and screened for correct constructs which should contain vector sequence, the inverted *URA3A* gene, and the amplified *CPRB* gene (SEQ ID NO: 82) of 20336. AscI*-Pme*I digestion confirmed a successful construct, pURAREDBin.

In a manner similar to the above, each of the other *CYP* and *CPR* genes disclosed herein are cloned into pURAin. *Pac*I fragments of these genes, whose sequences are given in Figures 13 and 15, are derivable by methods known to those skilled in the art.

### 1) Construction of Vectors Used to Generate HDC 20 and HDC 23

A previously constructed integration vector containing *CPRB* (SEQ ID NO: 82), pURAREDBin, was chosen as the starting vector. This vector was partially digested with *Pac*I and the linearized fragment was gel-isolated. The active *Pac*I was destroyed by treatment with T4 DNA polymerase and the vector was re-ligated. Subsequent isolation and complete digestion of this new plasmid yielded a vector now containing only one active *Pac*I site. This fragment was gel-isolated, dephosphorylated and ligated to the *CYP52A2A Pac*I fragment. Vectors that contain the *CYP52A2A* (SEQ ID NO: 86) and *CPRB* (SEQ ID NO: 82) genes oriented in the same direction, pURAin *CPR* 2A S, as well as opposite directions (5' ends connected), pURAin *CPR* 2A O, were generated.

### D. Confirmation of CYP Integration (Figure 21 for Integration Scheme) into the Genome of C. tropicalis

Based on the construct, pURA2in, used to transform H5343 *ura*⁻*,* a scheme to detect integration was devised. Genomic DNA from transformants was digested with *Dra* III and *Spe* I which are enzymes that cut within the *URA3A,* and *URA3B* genes but not within the integrated *CYP52A2A* gene. Digestion of genomic DNA where an integration had occurred at the *URA3A* or *URA3B* loci would be expected to result in a 3.5 kb or a 3.3 kb fragment, respectively (Figure 28). Moreover, digestion of the same genomic DNA with *Pac*I would yield a 2.2 kb fragment characteristic for the integrated *CYP52A2A* gene (Figure 28). Southern hybridizations of these digests with fragments of the *CYP52A2A* gene were used to screen for these integration events. Intensity of the band signal from the Southern using *Pac*I digestion was used as a measure of the number of integration events, ((i.e. the more copies of the *CYP52A2A* gene (SEQ ID NO: 86) which are present, the stronger the hybridization signal)).

*C. tropicalis* H5343 transformed URA prototrophs were grown at 30°C, 170 rpm, in 10 ml SC-uracil media for preparation of genomic DNA. Genomic DNA was isolated by the method described previously. Genomic DNA was digested with *Spe*I and DraIII. A 0.95% - agarose gel was used to prepare a Southern hybridization blot. The DNA from the gel was transferred to a MagnaCharge nylon filter membrane (MSI Technologies, Westboro, MA) according to the alkaline transfer method of Sambrook et al., *supra.* For the Southern hybridization, a 2.2 kb *CYP52A2A* DNA fragment was used as a hybridization probe. 300 ng of *CYP52A2A* DNA was labeled using a ECL Direct labeling and detection system (Amersham) and the Southern was processed according to the ECL kit specifications. The blot was processed in a volume of 30 ml of hybridization fluid corresponding to 0.125 ml/cm². Following a prehybridization at 42°C for 1 hr, 300 ng of *CYP52A2A* probe was added and the hybridization continued for 16 hr at 42°C. Following hybridization, the blots were washed two times for 20 min each at 42 °C in primary wash containing urea. Two 5 min secondary washes at RT were conducted, followed by detection according to directions. The blots were exposed for 16 hours (hr) as recommended.

Integration was confirmed by the detection of a *Spe*I*-Dra*III 3.5 kb fragment from the genomic DNA of the transformants but not with the *C. tropicalis* 20336 control. Subsequently, a *Pac*I digestion of the genomic DNA of the positive transformants, followed by a Southern hybridization using an *CYP52A2A* gene probe, confirmed integration by the detection of a 2.2 kb fragment. The resulting *CYP52A2A* integrated strain was named HDC1 (see Table 1).

In a manner similar to the above, each of the genes contained in the *Pac*I fragments which are described in Section 3c above were confirmed for integration into the genome of *C*. *tropicalis.*

Transformants generated by transformation with the vectors, pURAin *CPR* 2A S or pURAin *CPR* 2A O, were analyzed by Southern hybridization for integration of both the *CYP52A2A* (SEQ ID NO: 86) and *CPRB* (SEQ ID NO: 82) genes tandemly. Three strains were generated in which the *CYP52A2A* (SEQ ID NO: 86) and *CPRB* (SEQ ID NO: 82) genes integrated are in the opposite orientation (HDC 20-1, HDC 20-2 and HDC 20-3) and three were generated with the *CYP52A2A* (SEQ ID NO: 86) and *CPRB* (SEQ ID NO: 82) genes integrated in the same orientation (HDC 23-1, HDC 23-2 and HDC 23-3), Table 1.

### E. Confirmation of CPRB Integration into H5343 ura⁻

Seven transformants were screened by colony PCR using *CPRB* primer #2 (SEQ ID NO: 8) and a *URA3A-* specific primer. In five of the transformants, successful integration was detected by the presence of a 3899 bp PCR product. This 3899 bp PCR product represents the *CPRB* gene adjacent to the *URA3A* gene in the genome of H5343 thereby confirming integration. The resulting *CPRB* integrated strains were named HDC10-1 and HDC10-2 (see Table 1).

### F. Strain Evaluation.

As determined by quantitative PCR, when compared to parent H5343, HDC10-1 contained three additional copies of the reductase gene and HDC10-2 contained four additional copies of the reductase gene. Evaluations of HDC20-1, HDC20-2 and HDC20-3 based on Southern hybridization data indicates that HDC20-1 contained multiple integrations, i.e., 2 to 3 times that of HDC20-2 or HDC20-3. Evaluations of HDC23-1, HDC23-2, and HDC23-3 based on Southern hybridization data indicates that HDC23-3 contained multiple integrations, i.e., 2 to 3 times that of HDC23-1 or HDC23-2. The data in Table 8 indicates that the integration of components of the ω-hydroxylase complex have a positive effect on the improvement of *Candida tropicalis* ATCC 20962 as a biocatalyst. The results indicate that *CYP52A5A* (SEQ ID NO: 90) is an important gene for the conversion of oleic acid to diacid. Surprisingly, tandem integrations of *CYP* and *CPR* genes oriented in the opposite direction (HDC 20 strains) seem to be less productive than tandem integrations oriented in the same direction (HDC 23 strains), Tables 1 and 8.

**CHART**

| | | | |
|---|---|---|---|
| Media Composition | | Magnesium Sulfate (anhydrous) | 0.98 g |
| LB Broth | | Agar | 15 g |
| Bacto Tryptone | 10 g | Distilled Water | 1,000 ml |
| Bacto Yeast Extract | 5 g | NZCYM Top Agarose | |
| Sodium Chloride | 10 g | Bacto Casein Digest | 10 g |
| Distilled Water | 1,000 ml | Bacto Casamino Acids | 1 g |
| | | Bacto Yeast Extract | 5 g |
| LB Agar | | Sodium Chloride | 5 g |
| Bacto Tryptone | 10 g | Magnesium Sulfate | 0.98 g |
| Bacto Yeast Extract | 5 g | (anhydrous) | |
| Sodium Chloride | 10 g | Agarose | 7 g |
| Agar | 15 g | Distilled Water | 1,000 ml |
| Distilled Water | 1,000 ml | | |
| | | YEPD Broth | |
| LB Top Agarose | | Bacto Yeast Extract | 10 g |
| Bacto Tryptone | 10 g | Bacto Peptone | 20 g |
| Bacto Yeast Extract | 5 g | Glucose | 20 g |
| Sodium Chloride | 10 g | Distilled Water | 1,000 ml |
| Agarose | 7 g | | |
| Distilled Water | 1,000 ml | YEPD Agar* | |
| | | Bacto Yeast Extract | 10 g |
| NZCYM Broth | | Bacto Peptone | 20 g |
| Bacto Casein Digest | 10 g | Glucose | 20 g |
| Bacto Casamino Acids | 1 g | Agar | 20 g |
| Bacto Yeast Extract | 5 g | Distilled Water | 1,000 ml |
| Sodium Chloride | 5 g | | |
| Magnesium Sulfate | 0.98 g | SC - uracil* | |
| (anhydrous) | | Bacto-yeast nitrogen base without amino acids | 6.7g |
| Distilled Water | 1,000 ml | Glucose | 20g |
| | | Bacto-agar | 20g |
| NZCYM Agar | | Drop-out mix | 2g |
| Bacto Casein Digest | 10 g | Distilled water | 1,000ml |
| Bacto Casamino Acids | 1 g | | |
| Bacto Yeast Extract | 5 g | | |
| Sodium Chloride | 5 g | | |

| DCA2 medium | | g/l | |
|---|---|---|---|
| Peptone | | 3.0 | |
| Yeast Extract | | 6.0 | |
| Sodium Acetate | | 3.0 | |
| Yeast Nitrogen Base (Difco) | | 6.7 | |
| Glucose (anhydrous) | | 50.0 | |
| Potassium Phosphate (dibasic, trihydr | | ate) 7.2 | |
| Potassium Phosphate (monobasic, anh | | hydrous) 9.3 | |
| | | | |

| DCA3 medium | | g/l | |
|---|---|---|---|
| 0.3 M Phosphate buffer containing, pH 7.5 | | | |
| Glycerol | | 50 | |
| Yeast Nitrogen base (Difco) | | 6.7 | |
| | | | |

| Drop-out mix | | | |
|---|---|---|---|
| Adenine | 0.5g | Alanine | 2g |
| Arginine | 2g | Asparagine | 2g |
| Aspartic acid | 2g | Cysteine | 2g |
| Glutamine | 2g | Glutamic acid | 2g |
| Glycine | 2g | Histidine | 2g |
| Inositol | 2g | Isoleucine | 2g |
| Leucine | 10g | Lysine | 2g |
| Methionine | 2g | para-Aminobenzoic acid | 0.2g |
| Phenylalanine | 2g | Proline | 2g |
| Serine | 2g | Threonine | 2g |
| Tryptophan | 2g | Tyrosine | 2g |
| Valine | 2g | | |

| | | | |
|---|---|---|---|
| *See Kaiser et al., Methods in Yeast Genetics, Cold Spring Harbor Laboratory Press, USA (1994). | | | |

## Claims

1. Isolated nucleic acid encoding a *CYP52A5B* protein having the amino acid sequence as set forth in SEQ ID NO: 101.

2. Isolated nucleic acid comprising a coding region defined by nucleotides 1142-2695 as set forth in SEQ ID NO: 91.

3. Isolated nucleic acid according to claim 2 comprising the nucleotide sequence as set forth in SEQ ID NO: 91.

4. Isolated protein comprising an amino acid sequence as set forth in SEQ ID NO: 101.

5. A vector comprising a nucleotide sequence encoding a *CYP52A5B* protein having the amino acid sequence as set forth in SEQ ID NO: 101.

6. A vector according to claim 5 wherein the nucleotide sequence is set forth in nucleotides 1142-2695 of SEQ ID NO: 91.

7. A vector according to claim 5 wherein the vector is selected from the group consisting of plasmid, phagemid, phage and cosmid.

8. A method of producing a *CYP52A5B* protein having an amino acid sequence as set forth in SEQ ID NO: 101 comprising:
a) transforming a suitable host cell with a DNA sequence that encodes the protein having the amino acid sequence as set forth in SEQ ID NO: 101; and
b) culturing the cell under conditions favoring the expression of the protein.

9. The method according to claim 8 wherein the step of culturing the cell comprises adding an organic substrate to media containing the cell.

10. A method for increasing production of a dicarboxylic acid comprising:
a) providing a host cell having a naturally occurring number of *CYP52A5B* genes;
b) increasing, in the host cell, the number of *CYP52A5B* genes which encode a *CYP52A5B* protein having the amino acid sequence as set forth in SEQ ID NO: 101;
c) culturing the host cell in media containing an organic substrate which upregulates the *CYP52A5B* gene, to effect increased production of dicarboxylic acid.

11. A method for increasing the production of a *CYP52A5B* protein having an amino acid sequence as set forth in SEQ ID NO: 101 comprising:
a) transforming a host cell having a naturally occurring amount of *CYP52A5B* protein with an increased copy number of a *CYP52A5B* gene that encodes the *CYP52A5B* protein having the amino acid sequence as set forth in SEQ ID NO: 101; and
b) culturing the cell and thereby increasing expression of the protein compared with that of a host cell containing a naturally occurring copy number of the *CYP52A5B* gene.

## Patentansprüche

1. Isolierte Nukleinsäure, welche für ein CYP52A5B-Protein mit der in SEQ ID Nr.: 101 wiedergegebenen Aminosäuresequenz kodiert.

2. Isolierte Nukleinsäure, umfassend einen kodierenden Bereich, welcher durch die in SEQ ID Nr.: 91 wiedergegebenen Nukleotide 1142-2695 definiert wird.

3. Isolierte Nukleinsäure nach Anspruch 2, umfassend die in SEQ ID Nr.: 91 wiedergegebene Nukleotidsequenz.

4. Isoliertes Protein, umfassend die in (SEQ ID Nr. 101 wiedergegebene Aminosäuresequenz.

5. Ein Vektor, umfassend eine Nukleotidsequenz, welche ein *CYP52ASB-*Protein mit der in SEQ ID Nr.: 101 wiedergegebenen Aminosäuresequenz kodiert.

6. Ein Vektor nach Anspruch 5, wobei die Nukleotidsequenz durch die Nukleotide 1142-2695 in SEQ ID Nr.: 91 wiedergegeben wird.

7. Ein Vektor nach Anspruch 5, wobei der Vektor ausgewählt ist aus der Gruppe bestehend aus Plasmid, Phagemid, Phage und Cosmid.

8. Ein Verfahren zur Herstellung eines *CYP52A5B*-Proteins, welches die in SEQ ID Nr.: 101 wiedergegebene Aminosäuresequenz aufweist, umfassend:
a) das Transformieren einer geeigneten Wirtszelle mit einer DNA-Sequenz, welche für ein Protein kodiert, welches die in SEQ ID Nr.: 101 wiedergegebene Aminosäuresequenz aufweist; und
b) das Kultivieren der Zelle unter Bedingungen, welche die Expression des Proteins begünstigen.

9. Das Verfahren nach Anspruch 8, wobei der Schritt des Kultivierens der Zelle das Zusetzen eines organischen Substrates zu dem die Zelle beinhaltenden Medium umfasst.

10. Ein Verfahren zur Steigerung der Produktion eine Dicarbonsäure, umfassend:
a) das Bereitstellen einer Wirtszelle, welche eine natürlich vorkommende Anzahl an *CYP52A5B*-Genen aufweist;
b) das Erhöhen der Anzahl an *CYP52A5B*-Genen, welche ein *CYP52A5B*-Protein mit der in SEQ ID Nr.: 101 wiedergegebenen Aminosäuresequenz kodieren, in der Wirtszelle;
c) das Kultivieren der Wirtszelle in einem Medium beinhaltend ein organisches Substrat, welches das *CYP52A5B*-Gen hochreguliert, um die gesteigerte Produktion von Dicarbonsäure zu bewirken.

11. Ein Verfahren zur Steigerung der Produktion eines *CYP52A5B*-Proteins, welches die in SEQ ID Nr.: 101 wiedergegebene Aminosäuresequenz aufweist, umfassend:
a) das Transformieren einer Wirtszelle, welche eine natürlich vorkommende Menge an *CYP52A5B*-Protein aufweist, mit einer erhöhten Kopienanzahl eines CYP52A5B-Gens, welches für ein *CYP52A5B-*Protein mit der in (SEQ ID Nr.: 101 wiedergegebenen Aminosäuresequenz kodiert; und
b) das Kultivieren der Zelle und dadurch Erhöhen der Expression des Proteins im Vergleich mit der einer Wirtszelle, welche eine natürlich vorkommende Kopienanzahl des *CYP52A5B*-Gens aufweist.

## Revendications

1. Acide nucléique isolé codant pour une protéine *CYP52A5B* ayant la séquence d'acides aminés telle que représentée par SEQ ID NO : 101. 1.

2. Acide nucléique isolé comprenant une région codante définie par les nucléotides 1142 à 2695 telle que représentée par SEQ ID NO : 91.

3. Acide nucléique isolé selon la revendication 2, comprenant la séquence nucléotidique telle que représentée par SEQ ID NO : 91.

4. Protéine isolée comprenant une séquence d'acides aminés telle que représentée par SEQ ID NO : 101.

5. Vecteur comprenant une séquence nucléotidique codant pour une protéine *CYP52A5B* ayant la séquence d'acides aminés telle que représentée par SEQ ID NO : 101.

6. Vecteur selon la revendication 5, dans lequel la séquence nucléotidique est représentée par les nucléotides 1142 à 2695 de SEQ ID NO : 91.

7. Vecteur selon la revendication 5, où le vecteur est choisi dans le groupe constitué de plasmides, phagemides, phages et cosmides.

8. Procédé de production d'une protéine *CYP52A5B* ayant une séquence d'acides aminés telle que représentée par SEQ ID NO : 101 comprenant :
a) la transformation d'une cellule hôte appropriée avec une séquence d'ADN qui code pour la protéine ayant la séquence d'acides aminés telle que représentée par SEQ ID NO : 101 ; et
b) la culture de la cellule dans des conditions favorisant l'expression de la protéine.

9. Procédé selon la revendication 8, dans lequel l'étape de culture de la cellule comprend l'addition d'un substrat organique au milieu contenant la cellule.

10. Procédé permettant d'augmenter la production d'un acide dicarboxylique comprenant :
a) la fourniture d'une cellule hôte ayant un nombre existant à l'état naturel de gènes *CYP52A5B ;*
b) l'augmentation, dans la cellule hôte, du nombre de gènes *CYP52A5B* qui codent pour une protéine *CYP52A5B* ayant la séquence d'acides aminés telle que représentée par SEQ ID NO: 101 ;
c) la culture de la cellule hôte dans un milieu contenant un substrat organique qui régule à la hausse le gène *CYP52A5B,* pour effectuer une production accrue d'acide dicarboxylique.

11. Procédé permettant d'augmenter la production d'une protéine *CYP52A5B* ayant une séquence d'acides aminés telle que représentée par SEQ ID NO : 101 comprenant :
a) la transformation d'une cellule hôte ayant une quantité existant à l'état naturel de protéine *CYP52A5B* avec un nombre accru de copies d'un gène *CYP52A5B* qui code pour la protéine *CYP52A5B* ayant la séquence d'acides aminés telle que représentée par SEQ ID NO : 101 ; et
b) la culture de la cellule et de cette manière l'augmentation de l'expression de la protéine comparativement à celle d'une cellule hôte contenant un nombre existant à l'état naturel de copies du gène *CYP52A5B.*
